(19) Europäisches Patentamt
European Patent Office
Office européen des brevets

(11) EP 2 085 905 A1

(12) # EUROPEAN PATENT APPLICATION

published in accordance with Art. 153(4) EPC

(43) Date of publication:
**05.08.2009 Bulletin 2009/32**

(21) Application number: **07832720.2**

(22) Date of filing: **21.11.2007**

(51) Int Cl.:
***G06F 19/00*** *(2006.01)* ***C07K 14/00*** *(2006.01)*

(86) International application number:
**PCT/JP2007/073005**

(87) International publication number:
**WO 2008/062906 (29.05.2008 Gazette 2008/22)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **22.11.2006 JP 2006316356**

(71) Applicant: **In-Silico Sciences, Inc.**
**Ota-ku,**
**Tokyo 145-0065 (JP)**

(72) Inventors:
- **UMEYAMA, Hideaki**
  **Urayasu-shi**
  **Chiba 279-0011 (JP)**
- **SHITAKA, Mayuko**
  **Higashimurayama-shi**
  **Tokyo 189-0001 (JP)**
- **TERASHI, Genki**
  **Tokyo 152-0034 (JP)**
- **KANOU, Kazuhiko**
  **Tokyo 120-0003 (JP)**
- **KOMATSU, Katsuichiro**
  **Tokyo 145-0065 (JP)**
- **IWADATE, Mitsuo**
  **Tokyo 150-0013 (JP)**

(74) Representative: **HOFFMANN EITLE**
**Patent- und Rechtsanwälte**
**Arabellastraße 4**
**81925 München (DE)**

(54) **PROTEIN THREE-DIMENSIONAL STRUCTURE PROCESSING SYSTEM AND METHOD AND PROGRAM OF PROCESSING PROTEIN THREE-DIMENSIONAL STRUCTURE**

(57) An apparatus for processing protein stereostructure includes a control unit and a storage unit, wherein the storage unit includes protein stereostructure information, and the control unit includes, an unit that predicts protein stereostructure information after an arbitrary amino acid residue A in the protein stereostructure information stored in the storage unit is mutated into another amino acid residue a, and an unit that from the protein stereostructure information after mutation predicted by the mutated stereostructure-predicting unit, collects the amino acid residue A, the amino acid residue a, environment information P around the amino acid residue A before mutation, and environmental data p around the amino acid residue a after mutation upon changing from the environmental data P into the environmental data p related to one another as environment change information, thereby storing the environment change information in the storage unit.

FIG.3

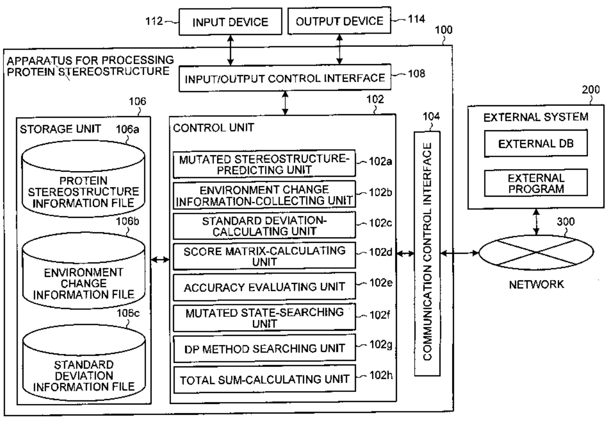


EP 2 085 905 A1

SEQUENCE LISTING

<110> In-Silico Sciences, Inc.

<120> APPARATUS FOR PROCESSING PROTEIN STEREOSTRUCTURE, METHOD OF PROCESSING PROTEIN STEREOSTRUCTURE, AND PROGRAM

<130> PCFA-08240-PCT

<160> 2

<170> PatentIn version 3.4

<210> 1
<211> 341
<212> PRT
<213> Vibrio cholerae

<400> 1

```
Gly Leu Val Gly Trp Arg Gly Met Val Gly Ser Val Leu Met Gln Arg
1               5                   10                  15

Met Val Glu Glu Arg Asp Phe Asp Leu Ile Glu Pro Val Phe Phe Ser
            20                  25                  30

Thr Ser Gln Ile Gly Val Pro Ala Pro Asn Phe Gly Lys Asp Ala Gly
        35                  40                  45

Met Leu His Asp Ala Phe Asp Ile Glu Ser Leu Lys Gln Leu Asp Ala
    50                  55                  60

Val Ile Thr Cys Gln Gly Gly Ser Tyr Thr Glu Lys Val Tyr Pro Ala
65                  70                  75                  80

Leu Arg Gln Ala Gly Trp Lys Gly Tyr Trp Ile Asp Ala Ala Ser Thr
                85                  90                  95

Leu Arg Met Asp Lys Glu Ala Ile Ile Thr Leu Asp Pro Val Asn Leu
            100                 105                 110

Lys Gln Ile Leu His Gly Ile His His Gly Thr Lys Thr Phe Val Gly
        115                 120                 125

Gly Asn Cys Thr Val Ser Leu Met Leu Met Ala Leu Gly Gly Leu Tyr
    130                 135                 140

Glu Arg Gly Leu Val Glu Trp Met Ser Ala Met Thr Tyr Gln Ala Ala
145                 150                 155                 160

Ser Gly Ala Gly Ala Gln Asn Met Arg Glu Leu Ile Ser Gln Met Gly
                165                 170                 175

Val Ile Asn Asp Ala Val Ser Ser Glu Leu Ala Asn Pro Ala Ser Ser
            180                 185                 190

Ile Leu Asp Ile Asp Lys Lys Val Ala Glu Thr Met Arg Ser Gly Ser
        195                 200                 205

Phe Pro Thr Asp Asn Phe Gly Val Pro Leu Ala Gly Ser Leu Ile Pro
    210                 215                 220
```

Trp Ile Asp Val Lys Arg Asp Asn Gly Gln Ser Lys Glu Glu Trp Lys
225 230 235 240

Ala Gly Val Glu Ala Asn Lys Ile Leu Gly Leu Gln Asp Ser Pro Val
245 250 255

Pro Ile Asp Gly Thr Cys Val Arg Ile Gly Ala Met Arg Cys His Ser
260 265 270

Gln Ala Leu Thr Ile Lys Leu Lys Gln Asn Ile Pro Leu Asp Glu Ile
275 280 285

Glu Glu Met Ile Ala Thr His Asn Asp Trp Val Lys Val Ile Pro Asn
290 295 300

Glu Arg Asp Ile Thr Ala Arg Glu Leu Thr Pro Ala Lys Val Thr Gly
305 310 315 320

Thr Leu Ser Val Pro Val Gly Arg Leu Arg Lys Met Ala Met Gly Asp
325 330 335

Asp Phe Leu Asn Ala
340

<210> 2
<211> 343
<212> PRT
<213> Haemophilus influenzae

<400> 2

Gly Phe Ile Gly Trp Arg Gly Met Val Gly Ser Val Leu Met Asp Arg
1 5 10 15

Met Ser Gln Glu Asn Asp Phe Glu Asn Leu Asn Pro Val Phe Phe Thr
20 25 30

Thr Ser Gln Ala Gly Gln Lys Ala Pro Val Phe Gly Gly Lys Asp Ala
35 40 45

Gly Asp Leu Lys Ser Ala Phe Asp Ile Glu Glu Leu Lys Lys Leu Asp
50 55 60

Ile Ile Val Thr Cys Gln Gly Gly Asp Tyr Thr Asn Glu Val Tyr Pro
65 70 75 80

Lys Leu Lys Ala Thr Gly Trp Asp Gly Tyr Trp Val Asp Ala Ala Ser
85 90 95

Ala Leu Arg Met Lys Asp Asp Ala Ile Ile Val Leu Asp Pro Val Asn
100 105 110

Gln His Val Ile Ser Glu Gly Leu Lys Lys Gly Ile Lys Thr Phe Val
115 120 125

Gly Gly Asn Cys Thr Val Ser Leu Met Leu Met Ala Ile Gly Gly Leu
130 135 140

Phe Glu Lys Asp Leu Val Glu Trp Ile Ser Val Ala Thr Tyr Gln Ala

145 150 155 160

Ala Ser Gly Ala Gly Ala Lys Asn Met Arg Glu Leu Leu Ser Gln Met
165 170 175

Gly Leu Leu Glu Gln Ala Val Ser Ser Glu Leu Lys Asp Pro Ala Ser
180 185 190

Ser Ile Leu Asp Ile Glu Arg Lys Val Thr Ala Lys Met Arg Ala Asp
195 200 205

Asn Phe Pro Thr Asp Asn Phe Gly Ala Ala Leu Gly Gly Ser Leu Ile
210 215 220

Pro Trp Ile Asp Lys Leu Leu Pro Glu Thr Gly Gln Thr Lys Glu Glu
225 230 235 240

Trp Lys Gly Tyr Ala Glu Thr Asn Lys Ile Leu Gly Leu Ser Asp Asn
245 250 255

Pro Ile Pro Val Asp Gly Leu Cys Val Arg Ile Gly Ala Leu Arg Cys
260 265 270

Asn Ser Gln Ala Phe Thr Ile Lys Leu Lys Lys Asp Leu Pro Leu Glu
275 280 285

Glu Ile Glu Gln Ile Ile Ala Ser His Asn Glu Trp Val Lys Val Ile
290 295 300

Pro Asn Asp Lys Glu Ile Thr Leu Arg Glu Leu Thr Pro Ala Lys Val
305 310 315 320

Thr Gly Thr Leu Ser Val Pro Val Gly Arg Leu Arg Lys Leu Ala Met
325 330 335

Gly Pro Glu Tyr Leu Ala Ala
340

## Description

TECHNICAL FIELD

The present invention relates to an apparatus for processing protein stereostructure, a method of processing protein stereostructure and a program. The invention relates in particular to the apparatus for processing protein stereostructure, the method of processing protein stereostructure and the program, which are capable of calculating a score matrix that is physical quantity indicating whether amino acid residues in a protein stereostructure are energetically stable in a specific environment.

BACKGROUND ART

A large number of methods have already been known for evaluating predicted stereostructures of proteins. Among them, methods for evaluation by a 3D1D method represented by Verify 3D (see Nonpatent Literatures 1 and 2) are known to enable highly accurate evaluation by a simple computation method.
For example, score function (3D1D SCOREij) of Verify 3D where amino acid residue i appears in environment j is defined by Eqs. (1) and (2) below:
[Eq. 1]

$$3D1D\,score\ ij = \ln\left(\frac{P(i \mid j)}{Pi}\right) \quad \cdots (1)$$

$$P(i \mid j) = \frac{N(i \mid j)}{\sum_{a=1}^{20} N(a \mid j)} \qquad Pi = \frac{N(i)}{\sum_{a=1}^{20} N(a)} \quad \cdots (2)$$

Eq. (1) is used widely as an evaluation function by the 3D1D method. Classification of environment j in Verify 3D is shown in FIG. 1 (excerpt from Nonpatent Literature 1). Fig. 1 is a view showing environment categories of Verify3D. In FIG. 1, the area buried is shown on the abscissa and the fraction of area occupied by polar atoms (fraction polar) on the ordinate.
In FIG. 1, it is meant that the exposed environment is classified into E and the buried environment into B. Depending on the degree to which polar atoms are occupied around a side chain, the environments P and B are subdivided. P is classified into P2 and P1 in order of decreasing polarity in the environment. The environment B is also classified into B3, B2 and B1 in the same manner. This environment categorization is performed every 3 types of secondary structures, and thus there are 18 environments j in total.
In Eq. (1), P(i|j) is the ratio at which amino acid residues i (i = 20 amino acids) appear in the environment j (B1 to B3, P1 to P2, and E in FIG. 1). Pi means the ratio at which the amino acid residues i are present regardless of the environment.
In Eq. (2), N(i|j) means the number at which amino acid residues i is observed in the environment j. N(i|j) is the number at which amino acid residues i is observed.
Fig. 2 is a distribution map of amino acid residue Valine applied to Fig. 1. In FIG. 2, the area buried is shown on the abscissa, and the fraction of area occupied by polar atoms (fraction polar) on the ordinate. The red dots show $\alpha$-helix and the green and blue dots show $\beta$-sheet and another region, respectively.
Nonpatent Literatures 1: Bowie JU, Luthy R, Eisenberg D. A method to identify protein sequences that fold into a known three-dimensional structure. Science. 1991 Jul 12;253(5016):164-70.
Nonpatent Literatures 2: Eisenberg D, Luthy R, Bowie JU. VERIFY3D: assessment of protein models with three-dimensional profiles. Methods Enzymol. 1997;277:396-404.

## DISCLOSURE OF INVENTION

## PROBLEM TO BE SOLVED BY THE INVENTION

However, as is evident from FIG. 1, the method in which the environment of the amino acids (i) is divided only 18 types, that is, (6 types shown in FIG. 1)×(3 types of secondary structures (helix, sheet, coil)) as shown in the conventional art can be said to be very rough in category. Accordingly, the 3D1D score shown in Eq. (1) in the conventional art appears as a score function lacking in continuity. However, the number of protein stereostructures that have been analyzed by experiments up to now is limited, so it is known that there is a problem that as the category is further subdivided, the number of statistical data (N(i|j)) for obtaining the value of P(i|j) in Eq. (1) by statistical methods is decreased. This leads to a problem that evaluation of amino acid residues i in the environment j short of statistical data cannot be accurately evaluated.

Accordingly, the present inventors predicted, by a homology modeling method, protein stereostructures derived from experimentally analyzed protein stereostructures by mutating 1 amino acid residue, thereby deducing the distribution of N(i|j) to examine the correction of statistical data. The inventors further examined whether there is a correlation of the amino acid residues with the environment j. As a result, the inventors found that the score function in Eq. (1) can be modified by which extremely highly accurate structural evaluation is made feasible, and the present invention was thereby arrived at.

That is, one object of the present invention is to provide an apparatus for processing protein stereostructure, a method of processing protein stereostructure and a program wherein how the environment j of amino acid residue i, shown in Eq. (1), is distributed by another amino acid residue is examined by constructing structures derived from an experimental structure by mutating one residue and statistically examining them, thereby solving the problem of decrease in statistical data attributable to subdivision of the environment.

Another object of the present invention is to provide an apparatus for processing protein stereostructure, a method of processing protein stereostructure and a program, by which the accuracy of a protein stereostructure predicted by the homology modeling method is evaluated.

Still another object of the present invention is to provide an apparatus for processing protein stereostructure, a method of processing protein stereostructure and a program, wherein predicted protein stereostructure information is used to evaluate the reliability of the structure highly accurately.

Still another object of the present invention is to provide an apparatus for processing protein stereostructure, a method of processing protein stereostructure and a program, which prepare a score matrix used in predicting the accuracy of protein stereostructures predicted by the homology modeling method. This score matrix is used to determine protein stereostructures in order of decreasing stability to provide a method of highly accurately predicting protein stereostructures. Further this score matrix is used in a means of obtaining predicted stability of interaction among proteins upon amino acid substitution, thereby improving protein functions for utilization in industry. Further, this score matrix is also used and extended to a score matrix also including ligand molecules, thereby improving functions in enzyme reaction or in binding of a protein to a ligand. Further, this score matrix is used to acquire alignment of primary structures of a plurality of proteins similar to one another in stereostructure, thereby enabling homology modeling and increasing the industrial usefulness of modeled proteins. Further, this score matrix is used to acquire an improved score matrix which is specially applicable to proteins caused to undergo structural change, thereby predicting the stability of folding in the proteins to provide an apparatus for processing protein stereostructure, a method of processing protein stereostructure and a program that are industrially utilizable.

## MEANS FOR SOLVING PROBLEM

First, the inventors statistically examined, from information on experimentally analyzed protein stereostructures, how the environment varies upon amino acid residue mutation without changing their surrounding environment. One amino acid residue of protein is thereby substituted and its information is used, thereby replacing only the environment category by the environment category after substitution without modeling a new protein stereostructure, to enable approximate calculation.

Eq. (5) below is a score function determined by using information after amino acid residue substitution.

[Eq. 2]

$$w(polar, buried, p, b) = \exp\left(\frac{-p^2}{2 \times \sigma_{polar}^2}\right) \times \exp\left(\frac{-b^2}{2 \times \sigma_{buried}^2}\right) \quad \cdots (3)$$

$$P(AA \mid ss, polar, buried) = \frac{\sum_{m,n} w(polar, buried, m, n) N(AA \mid ss, polar + m, buried + n)}{\sum_{aa} \sum_{m,n} w(polar, buried, m, n) N(aa \mid ss, polar + m, buried + n)} \quad \cdots (4)$$

$$SCORE(AA \mid ss, polar, buried) = \log\left(\frac{P(AA \mid ss, polar, buried)}{P(AA)}\right) \quad \cdots (5)$$

w (polar, buried, p, b) in Eq. (3) means the weighting parameter wherein the fraction polar (polar) is shifted by p and the fraction buried (buried) is shifted by b in the environment information defined by 3 parameters consisting of fraction polar (polar), fraction buried (buried), and secondary structure information (ss). $\sigma_{polar}$ represents the standard deviation in the fraction polar obtained from information on the amino acid residues after mutation. $\sigma_{buried}$ represents a standard deviation in fraction buried obtained from information after amino acid substitution.

P (AA|ss, polar, buried) in Eq. (4) means the ratio of amino acid residue AA present in the environment information consisting of the secondary structure information (ss), the fraction polar (polar), and the fraction buried (buried). In the present invention, Eq. (4) determined by Eq. (3) is handled as term P(i|j) in Eq. (1). P(i|j) means the ratio at which amino acid residues i appear in the environment j. Eq. (1) is thereby expressed in Eq. (5) above.

Accordingly, the present invention is achieved by an apparatus for processing protein stereostructure, a method of processing protein stereostructure and a program, which evaluate the accuracy of protein stereostructures by the apparatus for processing protein stereostructure, the method of processing protein stereostructure and the program which calculate and prepare a score matrix defined by Eq. (5) and by the score matrix thus prepared.

That is, in the present invention, protein stereostructures are constructed by a homology modeling method with one residue mutated into another amino acid residue in experimentally determined protein stereostructure information (PDB data, and the like). A large number of pieces of the information are produced, whereby the distribution of changes in the environment around the amino acid residue upon mutation into another amino acid residue is obtained. Assuming that the distribution of changes in the environment follows the Gaussian distribution, the variance and standard deviation indicative of the distribution are calculated. Using the standard deviation thus obtained, the number of data belonging to the same environment, on amino acid residues present in a certain environment, is acquired in consideration of the Gaussian distribution. From the obtained data, a score matrix in consideration of expectation is prepared. The prepared score matrix and protein stereostructure are input to evaluate the accuracy of the protein stereostructure.

By a means of obtaining, by this score matrix, the predictive value of stability of protein interaction upon amino acid substitution, protein functions are improved and utilized in industry.

When one amino acid residue is substituted by other 19 amino acid residues, a parameter that determines a score matrix determining the environment of the amino acid residues of the original amino acid residue sequence is stored while numerical values in score matrixes that determine the environments of other 19 amino acid residues are obtained, and the proteins after amino acid substitution are compared with the protein before substitution to predict their stability.

Further, this score matrix is used and extended to a score matrix also including ligand molecules, thereby improving functions in enzyme reaction or in binding of a protein to a ligand. In this case, all solution procedures are the same as in the method used in conversion into amino acid residue except that the scored matrix is one including ligand molecules.

Further, this score matrix is utilized to acquire alignment of primary structures of a plurality of proteins similar to one another in stereostructures, thereby enabling homology modeling and increasing the industrial usefulness of modeled proteins. At this time, the method of preparing the profile is the same as in the method used in conversion into amino acid residues.

Utilizing this score matrix, an improved score matrix applicable specially to proteins caused to undergo structural change is obtained to predict the stability of folding in the proteins and utilized in industry. At this time, not only the stabilization free energy of proteins but also the importance of amino acid residues considered helpful in keeping a shape important for functioning is reevaluated to provide methodologies and programs usable specifically in structural stability.

For solving the problems and for achieving the object, the present invention relates to an apparatus for processing protein stereostructure, including a control unit and a storage unit, which calculates a score matrix that is physical quantity indicating whether amino acid residues in a protein stereostructure are energetically stable in a specific environment, and a method of processing protein stereostructure or a program which is executed by the apparatus, wherein the storage unit includes protein stereostructure information that defines stereostructure coordinates of a protein composed of a plurality of the amino acid residues, and the control unit includes a mutated stereostructure-predicting unit (or step) that predicts protein stereostructure information after an arbitrary amino acid residue A in the protein stereostructure information stored in the storage unit is mutated into another amino acid residue a, an environment change information-collecting unit (or step) that from the protein stereostructure information after mutation predicted by the mutated stereostructure-predicting unit, collects the amino acid residue A, the amino acid residue a, environment information P around the amino acid residue A before mutation, and environmental data p around the amino acid residue a after mutation upon changing from the environmental data P into the environmental data p related to one another as environment change information, thereby storing the environment change information in the storage unit, a standard deviation-calculating unit (or step) that while assuming that a plurality of pieces of the environment information p after mutation relative to the environment information P in the environment change information collected by the environment information-collecting unit follow normal distribution, calculates standard deviation σ (P) for the environment information P and relates the environment information P to the standard deviation σ (P) to store the standard deviation σ (P)in the storage unit, and a score matrix-calculating unit (or step) that when the number (N(i|j)) of amino acid residues i present in a specific environment information j upon calculation of the score matrix, corrects the N(i|j) to calculate the score matrix by using a weighting parameter considering standard deviation σ (J) for environment information J after mutation relative to the environment information j stored by the standard deviation-calculating unit, and considering the standard deviation and an absolute value in difference between the environment information other than the environment information j and the environment information j.

The present invention is **characterized in that** the environment information is defined by the ratio of area occupied by polar atoms (fraction polar) that is the ratio of polar atoms occupying the surface of the amino acid residues, the fraction of buried area of the surface of the amino acid residues (fraction buried), and secondary structure information.

The present invention is **characterized in that** the score matrix-calculating unit (or step) uses the following equation as the weighting parameter:

[Eq. 3]

$$w(polar, buried, p, b) = \exp\left(\frac{-p^2}{2 \times \sigma_{polar}{}^2}\right) \times \exp\left(\frac{-b^2}{2 \times \sigma_{buried}{}^2}\right)$$

wherein w (polar, buried, p, b) is the weighting parameter wherein the fraction polar (polar) is shifted by p and the fraction buried (buried) is shifted by b in the environment information defined by 3 parameters consisting of fraction polar (polar), fraction buried (buried), and secondary structure information (ss), p is the absolute value in difference between the fraction polar before and after mutation, b is the absolute value in difference between the fraction buried before and after mutation, $\sigma_{polar}$ is the standard deviation in the fraction polar obtained from information on the amino acid residues after mutation, and $\sigma_{buried}$ is the standard deviation in fraction buried obtained from information the amino acid residues after mutation.

The present invention is **characterized in that** the score matrix-calculating unit (or step) calculates, as the score matrix, SCORE (AA|ss, polar, buried) by using the following equation:

[Eq. 4]

$$P(AA \mid ss, polar, buried) = \frac{\sum_{m,n} w(polar, buried, m, n) N(AA \mid ss, polar+m, buried+n)}{\sum_{aa} \sum_{m,n} w(polar, buried, m, n) N(aa \mid ss, polar+m, buried+n)}$$

$$SCORE(AA \mid ss, polar, buried) = \log\left(\frac{P(AA \mid ss, polar, buried)}{P(AA)}\right)$$

wherein P (AA|ss, polar, buried) is the ratio of amino acid residue AA present in the environment information consisting of the secondary structure information (ss), the fraction polar (polar), and the fraction buried (buried).

The present invention is characterized by further including an accuracy evaluating unit (or step) that calculates the score matrix for each of amino acid residues, and thereof obtains the sum total, thereby evaluating the accuracy of the protein stereostructure information.

The present invention is **characterized in that** the protein stereostructure information includes stereostructure information on a protein complex of a protein-interacting ligand and the protein or a protein complex into which a ligand has been inserted with a docking program, and amino acid residues around the ligand are changed thereby calculating the score matrix, to search for the state of amino acid residue mutation wherein the highest score matrix is calculated.

The present invention is **characterized in that** the environment change information on the amino acid residues is used, and the score matrix is used, to search for protein stereostructures similar to one another in folding state by dynamic programming (DP).

The present invention is **characterized in that** 3D1Dscore ij shown in the following equation using, as correction term k(i), the strength of influence of the amino acid residues i on protein folding structure is calculated as the score matrix:

[Eq. 5]

$$3D1D\ score\ ij = k(i) \cdot \ln\left(\frac{P(i \mid j)}{Pi}\right)$$

$$P(i \mid j) = \frac{N(i \mid j)}{\sum_{a=1}^{20} N(a \mid j)} \qquad Pi = \frac{N(i)}{\sum_{a=1}^{20} N(a)}$$

wherein P(i|j) is the frequency of appearance of the amino acid residues i in the environment information j, Pi is the ratio of the amino acid residues i present regardless of the environment, N(i|j) is the number of the amino acid residues i observed in the environment information j, and N(i) is the number of the amino acid residues i observed.

Also, the present invention is **characterized in that** the total sum of numerical values is calculated by the score matrix for amino acid residues around a specific amino acid residue of a protein for evaluating the modification of functions of the protein and the degree of contribution of the specific amino acid residue to functions of the protein.

EFFECT OF THE INVENTION

According to the present invention, the environment category in the score function by the 3D1D method can be virtually infinitely subdivided, and the evaluation of amino acid residues by the environment can be accurately conducted. That is, there is brought about an effect of providing a protein structure processing apparatus, a method of processing protein stereostructure and a program, wherein even if the environment category is subdivided, the number of N(i|j) can be corrected so as to serve as statistically sufficient data quantity, while the score matrix, that is, the physical quantity indicating whether the amino acid residues i present in the environment j are energetically stable can be prepared.

BRIEF DESCRIPTION OF DRAWINGS

[Fig. 1] Fig. 1 is a view showing environment categories of conventional Verify3D.

[Fig. 2] Fig. 2 is a distribution map of amino acid residue Valine applied to Fig. 1.

[Fig. 3] Fig. 3 is a block diagram showing an example of a configuration of a system to which the present embodiment is applied.

[Fig. 4] Fig. 4 is a scatter plot of change of fraction buried after amino acid residue mutation.

[Fig. 5] Fig. 5 is a scatter plot of change of fraction polar after amino acid residue mutation.

[Fig. 6] Fig. 6 is a number distribution map (frequency) with red line in each fraction buried after mutation and a Gaussian distribution map with green line obtained from calculated standard deviation in a category 0 of fraction buried of amino acid residue divided into 25.

[Fig. 7] Fig. 7 is a number distribution map (frequency) with red line in each fraction buried after mutation and a Gaussian distribution map with green line obtained from calculated standard deviation in a category 1 of the fraction buried of amino acid residue divided into 25.

[Fig. 8] Fig. 8 is a number distribution map (frequency) with red line in each fraction buried after mutation and a Gaussian distribution map with green line obtained from calculated standard deviation in a category 2 of the fraction buried of amino acid residue divided into 25.

[Fig. 9] Fig. 9 is a number distribution map (frequency) with red line in each fraction buried after mutation and a Gaussian distribution map with green line obtained from calculated standard deviation in a category 3 of the fraction buried of amino acid residue divided into 25.

[Fig. 10] Fig. 10 is a number distribution map (frequency) with red line in each fraction buried after mutation and a Gaussian distribution map with green line obtained from calculated standard deviation in a category 4 of the fraction buried of amino acid residue divided into 25.

[Fig. 11] Fig. 11 is a number distribution map (frequency) with red line in each fraction buried after mutation and a Gaussian distribution map with green line obtained from calculated standard deviation in a category 5 of the fraction buried of amino acid residue divided into 25.

[Fig. 12] Fig. 12 is a number distribution map (frequency) with red line in each fraction buried after mutation and a Gaussian distribution map with green line obtained from calculated standard deviation in a category 6 of the fraction buried of amino acid residue divided into 25.

[Fig. 13] Fig. 13 is a number distribution map (frequency) with red line in each fraction buried after mutation and a Gaussian distribution map with green line obtained from calculated standard deviation in a category 7 of the fraction buried of amino acid residue divided into 25.

[Fig. 14] Fig. 14 is a number distribution map (frequency) with red line in each fraction buried after mutation and a Gaussian distribution map with green line obtained from calculated standard deviation in a category 8 of the fraction buried of amino acid residue divided into 25.

[Fig. 15] Fig. 15 is a number distribution map (frequency) with red line in each fraction buried after mutation and a Gaussian distribution map with green line obtained from calculated standard deviation in a category 9 of the fraction buried of amino acid residue divided into 25.

[Fig. 16] Fig. 16 is a number distribution map (frequency) with red line in each fraction buried after mutation and a Gaussian distribution map with green line obtained from calculated standard deviation in a category 10 of the fraction buried of amino acid residue divided into 25.

[Fig. 17] Fig. 17 is a number distribution map (frequency) with red line in each fraction buried after mutation and a Gaussian distribution map with green line obtained from calculated standard deviation in a category 11 of the fraction buried of amino acid residue divided into 25.

[Fig. 18] Fig. 18 is a number distribution map (frequency) with red line in each fraction buried after mutation and a Gaussian distribution map with green line obtained from calculated standard deviation in a category 12 of the fraction buried of amino acid residue divided into 25.

[Fig. 19] Fig. 19 is a number distribution map (frequency) with red line in each fraction buried after mutation and a Gaussian distribution map with green line obtained from calculated standard deviation in a category 13 of the fraction buried of amino acid residue divided into 25.

[Fig. 20] Fig. 20 is a number distribution map (frequency) with red line in each fraction buried after mutation and a Gaussian distribution map with green line obtained from calculated standard deviation in a category 14 of the fraction buried of amino acid residue divided into 25.

[Fig. 21] Fig. 21 is a number distribution map (frequency) with red line in each fraction buried after mutation and a Gaussian distribution map with green line obtained from calculated standard deviation in a category 15 of the fraction buried of amino acid residue divided into 25.

[Fig. 22] Fig. 22 is a number distribution map (frequency) with red line in each fraction buried after mutation and a Gaussian distribution map with green line obtained from calculated standard deviation in a category 16 of the fraction buried of amino acid residue divided into 25.
[Fig. 23] Fig. 23 is a number distribution map (frequency) with red line in each fraction buried after mutation and a Gaussian distribution map with green line obtained from calculated standard deviation in a category 17 of the fraction buried of amino acid residue divided into 25.
[Fig. 24] Fig. 24 is a number distribution map (frequency) with red line in each fraction buried after mutation and a Gaussian distribution map with green line obtained from calculated standard deviation in a category 18 of the fraction buried of amino acid residue divided into 25.
[Fig. 25] Fig. 25 is a number distribution map (frequency) with red line in each fraction buried after mutation and a Gaussian distribution map with green line obtained from calculated standard deviation in a category 19 of the fraction buried of amino acid residue divided into 25.
[Fig. 26] Fig. 26 is a number distribution map (frequency) with red line in each fraction buried after mutation and a Gaussian distribution map with green line obtained from calculated standard deviation in a category 20 of the fraction buried of amino acid residue divided into 25.
[Fig. 27] Fig. 27 is a number distribution map (frequency) with red line in each fraction buried after mutation and a Gaussian distribution map with green line obtained from calculated standard deviation in a category 21 of the fraction buried of amino acid residue divided into 25.
[Fig. 28] Fig. 28 is a number distribution map (frequency) with red line in each fraction buried after mutation and a Gaussian distribution map with green line obtained from calculated standard deviation in a category 22 of the fraction buried of amino acid residue divided into 25.
[Fig. 29] Fig. 29 is a number distribution map (frequency) with red line in each fraction buried after mutation and a Gaussian distribution map with green line obtained from calculated standard deviation in a category 23 of the fraction buried of amino acid residue divided into 25.
[Fig. 30] Fig. 30 is a number distribution map (frequency) with red line in each fraction buried after mutation and a Gaussian distribution map with green line obtained from calculated standard deviation in a category 24 of the fraction buried of amino acid residue divided into 25.
[Fig. 31] Fig. 31 is a number distribution map (frequency) with red line in each fraction polar after mutation and a Gaussian distribution map with green line obtained from calculated standard deviation in a category 0 of fraction polar of amino acid residue divided into 25.
[Fig. 32] Fig. 32 is a number distribution map (frequency) with red line in each fraction polar after mutation and a Gaussian distribution map with green line obtained from calculated standard deviation in a category 1 of the fraction polar of amino acid residue divided into 25.
[Fig. 33] Fig. 33 is a number distribution map (frequency) with red line in each fraction polar after mutation and a Gaussian distribution map with green line obtained from calculated standard deviation in a category 2 of the fraction polar of amino acid residue divided into 25.
[Fig. 34] Fig. 34 is a number distribution map (frequency) with red line in each fraction polar after mutation and a Gaussian distribution map with green line obtained from calculated standard deviation in a category 3 of the fraction polar of amino acid residue divided into 25.
[Fig. 35] Fig. 35 is a number distribution map (frequency) with red line in each fraction polar after mutation and a Gaussian distribution map with green line obtained from calculated standard deviation in a category 4 of the fraction polar of amino acid residue divided into 25.
[Fig. 36] Fig. 36 is a number distribution map (frequency) with red line in each fraction polar after mutation and a Gaussian distribution map with green line obtained from calculated standard deviation in a category 5 of the fraction polar of amino acid residue divided into 25.
[Fig. 37] Fig. 37 is a number distribution map (frequency) with red line in each fraction polar after mutation and a Gaussian distribution map with green line obtained from calculated standard deviation in a category 6 of the fraction polar of amino acid residue divided into 25.
[Fig. 38] Fig. 38 is a number distribution map (frequency) with red line in each fraction polar after mutation and a Gaussian distribution map with green line obtained from calculated standard deviation in a category 7 of the fraction polar of amino acid residue divided into 25.
[Fig. 39] Fig. 39 is a number distribution map (frequency) with red line in each fraction polar after mutation and a Gaussian distribution map with green line obtained from calculated standard deviation in a category 8 of the fraction polar of amino acid residue divided into 25.
[Fig. 40] Fig. 40 is a number distribution map (frequency) with red line in each fraction polar after mutation and a Gaussian distribution map with green line obtained from calculated standard deviation in a category 9 of the fraction polar of amino acid residue divided into 25.
[Fig. 41] Fig. 41 is a number distribution map (frequency) with red line in each fraction polar after mutation and a Gaussian distribution map with green line obtained from calculated standard deviation in a category 10 of the fraction polar of amino acid residue divided into 25.

[Fig. 42] Fig. 42 is a number distribution map (frequency) with red line in each fraction polar after mutation and a Gaussian distribution map with green line obtained from calculated standard deviation in a category 11 of the fraction polar of amino acid residue divided into 25.

[Fig. 43] Fig. 43 is a number distribution map (frequency) with red line in each fraction polar after mutation and a Gaussian distribution map with green line obtained from calculated standard deviation in a category 12 of the fraction polar of amino acid residue divided into 25.

[Fig. 44] Fig. 44 is a number distribution map (frequency) with red line in each fraction polar after mutation and a Gaussian distribution map with green line obtained from calculated standard deviation in a category 13 of the fraction polar of amino acid residue divided into 25.

[Fig. 45] Fig. 45 is a number distribution map (frequency) with red line in each fraction polar after mutation and a Gaussian distribution map with green line obtained from calculated standard deviation in a category 14 of the fraction polar of amino acid residue divided into 25.

[Fig. 46] Fig. 46 is a number distribution map (frequency) with red line in each fraction polar after mutation and a Gaussian distribution map with green line obtained from calculated standard deviation in a category 15 of the fraction polar of amino acid residue divided into 25.

[Fig. 47] Fig. 47 is a number distribution map (frequency) with red line in each fraction polar after mutation and a Gaussian distribution map with green line obtained from calculated standard deviation in a category 16 of the fraction polar of amino acid residue divided into 25.

[Fig. 48] Fig. 48 is a number distribution map (frequency) with red line in each fraction polar after mutation and a Gaussian distribution map with green line obtained from calculated standard deviation in a category 17 of the fraction polar of amino acid residue divided into 25.

[Fig. 49] Fig. 49 is a number distribution map (frequency) with red line in each fraction polar after mutation and a Gaussian distribution map with green line obtained from calculated standard deviation in a category 18 of the fraction polar of amino acid residue divided into 25.

[Fig. 50] Fig. 50 is a number distribution map (frequency) with red line in each fraction polar after mutation and a Gaussian distribution map with green line obtained from calculated standard deviation in a category 19 of the fraction polar of amino acid residue divided into 25.

[Fig. 51] Fig. 51 is a number distribution map (frequency) with red line in each fraction polar after mutation and a Gaussian distribution map with green line obtained from calculated standard deviation in a category 20 of the fraction polar of amino acid residue divided into 25.

[Fig. 52] Fig. 52 is a number distribution map (frequency) with red line in each fraction polar after mutation and a Gaussian distribution map with green line obtained from calculated standard deviation in a category 21 of the fraction polar of amino acid residue divided into 25.

[Fig. 53] Fig. 53 is a number distribution map (frequency) with red line in each fraction polar after mutation and a Gaussian distribution map with green line obtained from calculated standard deviation in a category 22 of the fraction polar of amino acid residue divided into 25.

[Fig. 54] Fig. 54 is a number distribution map (frequency) with red line in each fraction polar after mutation and a Gaussian distribution map with green line obtained from calculated standard deviation in a category 23 of the fraction polar of amino acid residue divided into 25.

[Fig. 55] Fig. 55 is a number distribution map (frequency) with red line in each fraction polar after mutation and a Gaussian distribution map with green line obtained from calculated standard deviation in a category 24 of the fraction polar of amino acid residue divided into 25.

[Fig. 56] Fig. 56 is a view showing Gaussian distribution in the environment with 0 to 4% fraction buried and 0 to 4% fraction polar.

[Fig. 57] Fig. 57 is a view showing Gaussian distribution in the environment with 4.8 to 5.2% fraction buried and 4.8 to 5.2% fraction polar.

[Fig. 58] Fig. 58 is a number (N(i|j) in Eq. (2)) distribution chart obtained from protein stereostructure information analyzed by experiments on hydrophilic amino acid Lysine (LYS) whose secondary structure is 3 consecutive coils as CCC.

[Fig. 59] Fig. 59 is a distribution chart (frequency data) wherein the number distribution in an experimental structure where a hydrophilic amino acid Lysine secondary structure is CCC (3 consecutive coils) has been smoothed by Eq. (3) in which the fraction buried (%) is shown on the abscissa and the fraction polar (%) on the ordinate.

[Fig. 60] Fig. 60 is a score distribution chart of Eq. (5) where a hydrophilic amino acid Lysine secondary structure is CCC (3 consecutive coils).

[Fig. 61] Fig. 61 is a number distribution chart calculated from a data set of experimental structure where a hydrophilic amino acid Leucine (LEU) secondary structure is CCC (3 consecutive coils).

[Fig. 62] Fig. 62 is a distribution chart (frequency data) wherein the number distribution in an experimental structure where a hydrophilic amino acid Leucine secondary structure is CCC (3 consecutive coils) has been smoothed by Eq. (3).
[Fig. 63] Fig. 63 is a score distribution chart of Eq. (5) where a hydrophilic amino acid Leucine secondary structure is CCC (3 consecutive coils).
[Fig. 64] Fig. 64 is a distribution chart of correlation coefficient between GDT_TS score showing the accuracy of the structure by comparison with the experimental structure and Eq. (6).
[Fig. 65A and B] Fig. 65A and B are tables showing effect of the invention in CASP7.
[Fig. 66A and B] Fig. 66A and B are tables showing evaluation in which each domain structure of a protein stereostructure was evaluated by MAXSUB_DOM.
[Fig. 67A and B] Fig. 67A and B are tables showing results of evaluation by TM-Score.
[Fig. 68] Fig. 68 is a table showing results of all targets evaluated with the value of (MaxSub+Tm-score+GDT_TS)/3
[Fig. 69] Fig. 69 is a table showing evaluation in a category in difficulty level of targets being EASY.
[Fig. 70] Fig. 70 is a table showing evaluation in a category in difficulty level of targets being HARD.
[Fig. 71A and B] Fig. 71A and B are tables showing results of Robetta assessment.
[Fig. 72] Fig. 72 is a table showing results of Robetta assessment.
[Fig. 73] Fig. 73 is a view showing protein-protein interaction of a transcriptional regulator SlyA dimer.
[Fig. 74] Fig. 74 is a view showing interaction structure between a protein-protein complex of the transcriptional regulator SlyA dimer and DNA as a large ligand for the transcriptional regulator SlyA dimer.
[Fig. 75] Fig. 75 is a chart of alignment results with similar profile searched by a profile-profile alignment method.
[Fig. 76] Fig. 76 is a chart showing information on alignments plotted in the vicinity of 60% homology (sequence numbers: 1 and 2).
[Fig. 77] Fig. 77 is a diagram showing coefficients optimized in correlation coefficient.

## EXPLANATIONS OF LETTERS OR NUMERALS

100 Apparatus for processing protein stereostructure
102 Control unit
102a Mutated stereostructure-predicting unit
102b Environment change information-collecting unit
102c Standard deviation-calculating unit
102d Score matrix-calculating unit
102e Accuracy evaluating unit
102f Mutated state-searching unit
102g DP method searching unit
102h Total sum-calculating unit
104 Communication control interface
106 Storage unit
106a protein stereostructure information file
106b Environment change information file
106c Standard deviation information file
108 Input/output interface
110 User program executing unit
112 Input device
114 Output device
200 External system
300 Network

## BEST MODE(S) FOR CARRYING OUT THE INVENTION

The following describes an embodiment of an apparatus for processing protein stereostructure, a method of processing protein stereostructure and a program according to the present invention in detail with reference to the drawings. The embodiment is illustrative only, and is not intended to limit the present invention in any way.

[Overview of the System]

The following outlines the present invention, and then, a configuration and processing of the present invention are explained in detail.

In general, the invention has schematically following basic features. That is to say, the invention relates in particular to the apparatus for processing protein stereostructure, the method of processing protein stereostructure and the program, which are capable of calculating a score matrix that is physical quantity (for example, the Eq. (1) etc.) indicating whether amino acid residues in a protein stereostructure are energetically stable in a specific environment.

The storage unit in the apparatus for processing protein stereostructure stores protein stereostructure information that defines stereostructure coordinates of a protein composed of a plurality of the amino acid residues.

The control unit in the apparatus for processing protein stereostructure predicts protein stereostructure information after an arbitrary amino acid residue A in the protein stereostructure information stored in the storage unit is mutated into another amino acid residue a.

The control unit in the apparatus for processing protein stereostructure, from the protein stereostructure information after mutation predicted, collects the amino acid residue A, the amino acid residue a, environment information P around the amino acid residue A before mutation, and environmental data p around the amino acid residue a after mutation upon changing from the environmental data P into the environmental data p related to one another as environment change information, thereby storing the environment change information in the storage unit.

Herein, the environment information may be defined by the ratio of area occupied by polar atoms (fraction polar) that is the ratio of polar atoms occupying the surface of the amino acid residues, the fraction of buried area of the surface of the amino acid residues (fraction buried), and secondary structure information (ss).

The control unit in the apparatus for processing protein stereostructure, while assuming that a plurality of pieces of the environment information p after mutation relative to the environment information P in the environment change information collected follow normal distribution, calculates standard deviation σ (P) for the environment information P and relates the environment information P to the standard deviation σ (P) to store the standard deviation σ (P)in the storage unit.

The control unit in the apparatus for processing protein stereostructure, when the number (N(i|j)) of amino acid residues i present in a specific environment information j upon calculation of the score matrix, corrects the N(i|j) to calculate the score matrix by using a weighting parameter considering standard deviation σ (J) for environment information J after mutation relative to the environment information j stored, and considering the standard deviation and an absolute value in difference between the environment information other than the environment information j and the environment information j.

Herein, Eq. (3) below may be used as the weighting parameter:

[Eq. 6]

$$w(polar, buried, p, b) = \exp\left(\frac{-p^2}{2 \times \sigma_{polar}^{\;2}}\right) \times \exp\left(\frac{-b^2}{2 \times \sigma_{buried}^{\;2}}\right) \quad \cdots (3)$$

In Eq. (3), w (polar, buried, p, b) is the weighting parameter wherein the fraction polar (polar) is shifted by p and the fraction buried (buried) is shifted by b in the environment information defined by 3 parameters consisting of fraction polar (polar), fraction buried (buried), and secondary structure information (ss), p is the absolute value in difference between the fraction polar before and after mutation, b is the absolute value in difference between the fraction buried before and after mutation, $\sigma_{polar}$ is the standard deviation in the fraction polar obtained from information on the amino acid residues after mutation, and $\sigma_{buried}$ is the standard deviation in fraction buried obtained from information the amino acid residues after mutation.

SCORE (AA|ss, polar, buried) may be calculated, as the score matrix, by using Eqs. (4) and (5).

[Eq. 7]

$$P(AA \mid ss, polar, buried) = \frac{\sum_{m,n} w(polar, buried, m, n) N(AA \mid ss, polar + m, buried + n)}{\sum_{aa} \sum_{m,n} w(polar, buried, m, n) N(aa \mid ss, polar + m, buried + n)} \quad \cdots (4)$$

$$SCORE(AA \mid ss, polar, buried) = \log\left(\frac{P(AA \mid ss, polar, buried)}{P(AA)}\right) \quad \cdots (5)$$

In Eqs. (4) and (5), P (AA|ss, polar, buried) is the ratio of amino acid residue AA present in the environment information consisting of the secondary structure information (ss), the fraction polar (polar), and the fraction buried (buried).

The control unit in the apparatus for processing protein stereostructure may calculate the score matrix for each of amino acid residues, and thereof obtain the sum total, thereby evaluating the accuracy of the protein stereostructure information.

The protein stereostructure information may include stereostructure information on a protein complex of a protein-interacting ligand and the protein or a protein complex into which a ligand has been inserted with a docking program, and in the control unit in the apparatus for processing protein stereostructure, amino acid residues around the ligand may be changed thereby calculating the score matrix, to search for the state of amino acid residue mutation wherein the highest score matrix is calculated.

In the control unit in the apparatus for processing protein stereostructure, the environment change information on the amino acid residues may be used, and the score matrix may be used, to search for protein stereostructures similar to one another in folding state by dynamic programming (DP).

In the control unit in the apparatus for processing protein stereostructure, Eq. (8) below using, as correction term k(i), the strength of influence of the amino acid residues i on protein folding structure may be calculated as the score matrix.

[Eq. 8]

$$3D1D\ score\ ij = k(i) \cdot \ln\left(\frac{P(i \mid j)}{Pi}\right) \quad \cdots (8)$$

In Eq. 8, P(i|j) is the frequency of appearance of the amino acid residues i in the environment information j and Pi is the ratio of the amino acid residues i present regardless of the environment.

In the control unit in the apparatus for processing protein stereostructure, the total sum of numerical values may be calculated by the score matrix for amino acid residues around a specific amino acid residue of a protein for evaluating the modification of functions of the protein and the degree of contribution of the specific amino acid residue to functions of the protein.

[Configuration of the System]

The following describes a configuration of the system. Fig. 3 is a block diagram showing an example of a configuration of a system to which the present embodiment is applied, and conceptually shows only parts related to the present invention. In general, the system is provided with a apparatus for processing protein stereostructure 100 communicating thorough a network 300, external system 200 providing external database (DB) concerning amino acid sequence and stereostructure of protein, and external program of motif search, homology search, and the like.

In Fig. 3, the network 300 has function of connecting the apparatus for processing protein stereostructure 100 with the external system 200, and is internet , and the like.

In Fig. 3, the external system 200 is connected to the apparatus for processing protein stereostructure 100 thorough a network 300, and has function of providing to users external DB concerning amino acid sequence and stereostructure of protein (PDB, and the like), and websites executing external program of motif search, homology search, and the like.

The external system 200 can be configured as WEB server, ASP server, and the like, and the hardware can be configured by a information processing device and the accessory devices such as existing personal computer, and workstation. Each function of the external system 200 can be realized by a central processing unit (CPU) in the external system 200, a disk device, a input device, a output device, a communication control interface, and the like, and a computer program etc. controlling these devices.

In Fig. 3, the apparatus for processing protein stereostructure 100 is conceptually provided with the control unit 102 such as CPU which controls the apparatus for processing protein stereostructure 100 totally, or the like, the communication control interface 104 which connects to a communication device (not shown) such as a router connected to a communication channel or the like, the input/output interface 108 connected to the input device 112 and the output device 114, and the storage unit 106 which stores various databases and tables, and the units and devices are communicatably connected through an optional communication channel. Further, the apparatus for processing protein stereostructure 100 are communicatably connected to a network 300 via a communication device such as a router, and a wired or wireless communication line such as a dedicated line.

The various databases and tables (such as an protein stereostructure information file 106a, environment change information file 106b, and standard deviation information file 106c) stored in the storage unit 106 are storage units such as fixed disk devices. As shown in Fig. 3, the storage units store various programs, various tables, various databases, various file for web page and the like used in various processes.

As shown in Fig. 3, the protein stereostructure information file 106a in the storage unit 106 is a protein stereostructure information storage unit that stores protein stereostructure information that defines stereostructure coordinates of a protein composed of a plurality of the amino acid residues. The protein stereostructure information may include stereostructure information on a protein complex of a protein-interacting ligand and the protein or a protein complex into which a ligand was inserted with a docking program.

The environment change information file 106b is environment change information storage unit that collects the amino acid residue A, the amino acid residue a, environment information P around the amino acid residue A before mutation, and environmental data p around the amino acid residue a after mutation upon changing from the environmental data P into the environmental data p related to one another as environment change information, thereby storing the environment change information.

The standard deviation information file 106c is standard deviation storage unit that while assuming that a plurality of pieces of the environment information p after mutation relative to the environment information P in the environment change information follow normal distribution, calculates standard deviation σ (P) for the environment information P and relates the environment information P to the standard deviation σ (P) to store the standard deviation σ (P).

In Fig. 3, the communication control interface 104 controls communication between the apparatus for processing protein stereostructure 100 and the network 300 (or a communication device such as a router). That is to say, the communication control interface 104 has a function to communicate data to another terminal through a communication line.

In Fig. 3, the input/output control interface 108 controls the input device 112 and the output device 114. The output device 114 corresponds to a display (monitor), a speaker, a external memory device, and the like, and the input device corresponds to a keyboard, a mouse, microphone, and the like.

In Fig. 3, The control unit 102 has an internal memory that stores a control program such as an operating system (OS), a program defining various procedures, and required data. The control unit 102 performs information processing for executing various processing by the programs or the like. The control unit 102 functionally conceptually has a mutated stereostructure-predicting unit 102a, an environment change information-collecting unit 102b, a standard deviation-calculating unit 102c, a score matrix-calculating unit 102d, an accuracy evaluating unit 102e, a mutated state-searching unit 102f, a DP method searching unit 102g, and a total sum-calculating unit 102h.

The mutated stereostructure-predicting unit 102a is a mutated stereostructure-predicting unit that predicts protein stereostructure information after an arbitrary amino acid residue A in the protein stereostructure information stored in the protein stereostructure information file 106a is mutated into another amino acid residue a.

The environment change information-collecting unit 102b is an environment change information-collecting unit that from the protein stereostructure information after mutation predicted by the mutated stereostructure-predicting unit, collects the amino acid residue A, the amino acid residue a, environment information P around the amino acid residue A before mutation, and environmental data p around the amino acid residue a after mutation upon changing from the environmental data P into the environmental data p related to one another as environment change information, thereby storing the environment change information in the environment change information file 106b.

The standard deviation-calculating unit 102c is a standard deviation-calculating unit that while assuming that a plurality of pieces of the environment information p after mutation relative to the environment information P in the environment change information collected by the environment information-collecting unit follow normal distribution, calculates standard deviation σ (P) for the environment information P and relates the environment information P to the standard deviation σ (P) to store the standard deviation σ (P)in the standard deviation information file 106c.

The score matrix-calculating unit 102d is a score matrix-calculating unit that when the number (N(i|j)) of amino acid residues i present in a specific environment information j upon calculation of the score matrix, corrects the N(i|j) to calculate the score matrix by using a weighting parameter considering standard deviation σ (J) for environment information J after mutation relative to the environment information j stored by the standard deviation-calculating unit, and considering the standard deviation and an absolute value in difference between the environment information other than the environment information j and·the environment information j.

The accuracy evaluating unit 102e is an accuracy evaluating unit that calculates the score matrix for each of amino acid residues, and thereof obtains the sum total, thereby evaluating the accuracy of the protein stereostructure information.

The mutated state-searching unit 102f is a mutated state-searching unit that using the protein stereostructure information includes stereostructure information on a protein complex of a protein-interacting ligand and the protein or a protein complex into which a ligand has been inserted with a docking program, changes amino acid residues around the ligand, thereby calculating the score matrix, to search for the state of amino acid residue mutation wherein the highest score matrix is calculated.

The DP method searching unit 102g is DP method searching unit that uses the environment change information on the amino acid residues, and uses the score matrix, to search for protein stereostructures similar to one another in folding state by dynamic programming (DP).

The total sum-calculating unit 102h is a total sum-calculating unit that calculates the total sum of numerical values by the score matrix for amino acid residues around a specific amino acid residue of a protein for evaluating the modification of functions of the protein and the degree of contribution of the specific amino acid residue to functions of the protein.

Details on the processes in the units hereinafter are described.

[Processing in the system]

The following describes in detail one example of the processing in the system with reference to Fig. 4-63.

The environment in which the amino acid residue i is present is defined by 3 parameters, that is, the ratio of area occupied by polar atoms (fraction polar) that is the ratio of polar atoms occupying the surface of the amino acid residues, the fraction of buried area of the surface of the amino acid residues (fraction buried), and the secondary structure information (ss) formed by the amino acid residues i. Without paying attention to only the amino acid residue in question, a secondary structure formed by an amino acid residue just before or after the amino acid residue may also be taken into consideration as the secondary structure information. The fraction polar and fraction buried were those considered in 100 divisions every 1%.

A PDB file on experimentally analyzed protein stereostructures was used as protein stereostructure information file 106a.

In the apparatus for processing protein stereostructure 100, a part of amino acid residues in the protein stereostructure information file 106a were substituted by other amino acid residues by processing with the mutated stereostructure-predicting unit 102a, and the structure was optimized with homology modeling software FAMS developed by the present inventors.

Originally, data should be collected by experimentally mutating an amino acid sequence and examining its stereostructure. However, data necessary for calculating the value corresponding statistically to free energy are hundreds of thousand of mutation data. Not all of these data can be experimentally obtained. Accordingly, FAMS (Ogata, K. and Umeyama, H. (2000). An automatic homology modeling method consisting of database searches and simulated annealing. J. Mol. Graphics Mod. 18, 258-272) which has been reliably used in construction of side chain etc. was used to rapidly predict mutation data.

Specifically, 997 amino acid sequences having 150 or less amino acid residues at a resolution of 2.0 or less (value for PDB to be excellent in coordinate accuracy), out of PDB from which amino acid sequences having 50% or more homology to one another had been removed, were subjected 100 times to random 1-residue mutation calculation. However, due to the high speed calculation, when mutated sites are apart by 15 angstroms or more in one protein stereostructure information (PDB), mutations in a plurality of sites are simultaneously conducted in once calculation. As a result, mutations at 504,716 sites were conducted to construct stereostructures by the homology modeling method using FAMS.

Then, from the calculated stereostructure information, how the two environments (fraction buried and fraction polar) were changed respectively before and after mutation was examined by processing with the environment change information-collecting unit 102b. As a result, it was found that there were distributions in FIGS. 4 and 5.

Fig. 4 is a scatter plot of change of fraction buried after amino acid residue mutation. In FIG. 4, the state before mutation is shown on the abscissa and the state after mutation on the ordinate.

Fig. 5 is a scatter plot of change of fraction polar after amino acid residue mutation. In FIG. 5, the state before mutation is shown on the abscissa and the state after mutation on the ordinate.

As to this data, the fraction buried was divided into 25 sections by processing with the standard deviation-calculating unit 102c, and the standard deviation of frequency of appearance in each section of fraction buried (%) was calculated.

Figs. 6-30 are number distribution maps (frequency) with red line in each fraction buried after mutation and Gaussian distribution maps with green line obtained from calculated standard deviation in each fraction buried of amino acid residue divided into 25. Because the fraction buried was divided into 25 sections, the Gaussian distribution consists of 25 graphs from category 0 to category 24 (corresponding to FIG. 6 to FIG. 30 respectively). The fraction buried (%) is shown on the abscissa and the frequency on the ordinate.

For example, the graph (FIG. 6) for category: 0 indicates the distribution showing how data (fraction buried) on amino acid side chain in 0 to 4% section before amino acid residue mutation were changed after amino acid mutation. Their standard deviation was determined for the 25-divided data, and the Gaussian distribution was plotted with green lines such that the extremal value becomes a mean value of fraction buried before mutation. It can be seen that the Gaussian distribution well indicates the distribution of actual mutation data.

Similarly to plotting of the fraction buried, the fraction polar was also plotted. The frequency of appearance in each section of fraction polar (%) was plotted as mutation data in a histogram with red lines in each of FIGS. 31 to 55.

Figs. 31-55 are number distribution maps (frequency) with red line in each fraction polar after mutation and Gaussian distribution maps with green line obtained from calculated standard deviation in each fraction polar of amino acid residue divided into 25. The fraction polar was divided into 25 sections, the Gaussian distribution consists of 25 graphs from category 0 to category 24 (corresponding to FIG. 31 to FIG. 55 respectively). The fraction polar (%) is shown on the abscissa and the frequency on the ordinate. Their standard deviation was determined for the 25-divided data, and the Gaussian distribution was plotted with green lines such that the extremal value becomes a mean value of fraction polar before mutation. It can be said that similarly to the graph of fraction buried, the Gaussian distribution can indicate the frequency of appearance of fraction polar.

Then, the weighing shown in Eq. (3) is graphed by processing with the score matrix-calculating unit 102d and shown in FIGS. 56 and 57. Fig. 56 is a view showing Gaussian distribution in the environment with 0 to 4% fraction buried and 0 to 4% fraction polar. That is, the weighed graph (category fb:0 fp:0) of Eq. (3) used in calculating P(i|j) corresponding to a shift in fraction polar and fraction buried in the environment with 0 to 4% fraction buried and 0 to 4% fraction polar. In FIG. 56, the fraction buried is shown on the X-axis, the fraction polar on the Y-axis, and the weight on the Z-axis.

Fig. 57 is a view showing Gaussian distribution in the environment with 4.8 to 5.2% fraction buried and 4.8 to 5.2% fraction polar. That is, the weighed graph (category fb:12 fp:12) of Eq. (3) used in calculating P(i|j) corresponding to a shift in fraction polar and fraction buried in the environment with 48 to 52% fraction buried and 48 to 52% fraction polar. The fraction buried is shown on the X-axis, the fraction polar on the Y-axis, and the weight on the Z-axis.

Then, by processing with the score matrix-calculating unit 102d, the obtained standard deviation in each section was used to calculate Eqs. (3) to (5). As a result, the score matrix of amino acid residue i for the environment (secondary structure information (ss), fraction polar and fraction buried) was calculated.

For example, Fig. 58 is a number (N(i|j) in Eq. (2)) distribution chart obtained from protein stereostructure information analyzed by experiments on hydrophilic amino acid Lysine (LYS) whose secondary structure is 3 consecutive coils as CCC. That is, the number distribution (frequency data) calculated from a data set of an experimental structure in which a hydrophilic amino acid Lysine secondary structure is CCC (3 consecutive coils) is shown. The fraction buried (%) is shown on the abscissa and the fraction polar (%) on the ordinate.

The data after correction by weighing with the function in Eq. (3) by processing with the score matrix-calculating unit 102d are shown in FIG. 59. That is, a distribution chart (frequency data) wherein the number distribution in an experimental structure where a hydrophilic amino acid Lysine secondary structure is CCC (3 consecutive coils) has been smoothed by Eq. (3) is shown. The fraction buried (%) is shown on the abscissa and the fraction polar (%) on the ordinate.

The score matrix calculated in Eq. (5) by processing with the score matrix-calculating unit 102d is shown in FIG. 60. Fig. 60 is a score distribution chart of Eq. (5) where a hydrophilic amino acid Lysine secondary structure is CCC (3 consecutive coils). The fraction buried (%) is shown on the abscissa and the fraction polar (%) on the ordinate. Thus, it can be seen that experimental data on a hydrophilic residue Lysine are concentrated in the upper left of the graph. It can also be seen from FIG. 60 showing score distribution that the score of Lysine (LYS) is higher in the environment in the upper left of the graph. From this result, it can be seen that Lysine (LYS) occurs more readily in the environment where the fraction buried is low and the fraction polar (fraction of area occupied by polar atoms) is high.

It can also be seen that an amino acid Leucine (LEU) that is hydrophobic opposite to Lysine tends to occur readily in the lower right environment of the graph, as shown in FIGS. 61, 62 and 63. Fig. 61 is a number distribution chart calculated from a data set of experimental structure where a hydrophilic amino acid Leucine (LEU) secondary structure is CCC (3 consecutive coils). Fig. 62 is a distribution chart (frequency data) wherein the number distribution in an experimental structure where a hydrophilic amino acid Leucine secondary structure is CCC (3 consecutive coils) has been smoothed by Eq. (3). Fig. 63 is a score distribution chart of Eq. (5) where a hydrophilic amino acid Leucine secondary structure is CCC (3 consecutive coils). The fraction buried (%) is shown on the abscissa and the fraction polar (%) on the ordinate.

By processing with the score matrix-calculating unit 102d, the results of the score matrix are output to a file.

By processing with the accuracy evaluating unit 102e, the score matrix calculated with the score matrix-calculating unit 102d, and the protein stereostructure information file 106a such as stereostructure information file (PDB) as input file, are used to evaluate the accuracy of the protein stereostructure by Eq. (6) below. It is meant that as the resulting score increases, the protein stereostructure reflects statistical data of the experimental structure. According to the present invention, the score of each amino acid residue in the protein stereostructure is also simultaneously output.

[Eq. 9]

$$TOTALSCORE = \sum_{n=0}^{L} SCORE(AA_n \mid ss_n, polar_n, buried_n) \quad \cdots (6)$$

EXAMPLE 1

By molecular dynamics (MD) calculation, the experimental structure of protein stereostructure was intentionally destroyed to calculate the score in Eq. (6). The distribution of the correlation coefficient between GDT_TS score showing the accuracy of the structure by comparison with the experimental structure and Eq. (6) is shown in FIG. 64. A higher GDT_TS score is indicative of higher similarity to the experimental structure and is used as an indicator of the accuracy of structures in an international protein stereostructure prediction contest CASP (Critical Assessment of Techniques for Protein Structure Prediction:

http://predictioncenter.gc.ucdavis.edu/).

To obtain the correlation coefficient, 50 destroyed structure were generated from one PDB structure by MD. This verification was conducted by using 3140 PDB structures. In FIG. 64, individual verification examples were shown on the abscissa, and their correlation coefficients were plotted on the ordinate. In 2859 verification examples, the correlation coefficients were equal to or higher than 0.8. This means that when predicted protein stereostructures of unknown experimental structures are evaluated with numerical values of Eq. (6), the accuracy of the structures can be predicted in many cases.

FIGS. 65A to 72 show the effect of the present invention in the protein stereostructure prediction contest, CASP7 (Critical Assessment of Techniques for Protein Structure Prediction, http://predictioncenter.org/casp7/). The CASP is a contest of competition for accuracy of a stereostructure, predicted from its amino acid sequence, of a protein of unknown stereostructure.

In CASP7, those methods which we carried out by utilizing the present invention are as follows:

(1) For amino acid sequences on the test, alignments were obtained from a plurality of alignment programs (BLAST, PSI-BLAST, PRS-BLAST, HMMER etc.). Information on alignments and predicted structures was obtained from alignment servers and modeling servers (ROBETTA, SP3, SPARKS2 and GenTHREADER) open to the public.

(2) Homology modeling software FAMS (Ogata, K. and Umeyama, H. (2000). An automatic homology modeling method consisting of data base searches and simulated annealing. J. Mol. Graphics Mod. 18, 258-272) was used to construct predicted structures of protein stereostructures from the alignments obtained in (1). The predicted structures in other servers obtained in (1) were optimized with the homology modeling software FAMS.

(3) A large number of predicted structures calculated in (2) were evaluated in Eq. (7) below.

[Eq. 10]

$$TotalScore = \begin{cases} 0.35 \times SSscore + 3D1Dscore_{CM} & CM \\ 0.75 \times SSscore + 3D1Dscore_{FRNF} & FR\ or\ NF \end{cases} \quad \cdots (7)$$

Depending on the difficulty level of targets on the test, 2 types of score functions were used. CM refers to targets with such a difficulty level that alignments predominately showing homology can be obtained with alignment program PSI-BLAST. It is in the CM category that the technique of homology modeling can be effectively used. FR or NF refers to targets that are more difficult than in CM. SSscore shows scored coincidence between secondary structures predicted with secondary structure prediction program PSIPRED and predicted secondary structures of stereostructures. 3D1Dscore CM is a score shown in Eq. (6). 3D1Dscore FRNF means a score calculated from Eqs. (4) and (6) with fixed average values of σ polar and σ buried in Eq. (3).

(4) From the results of evaluation of predicted structures in (3), 5 structures out of those having the highest score in Eq. (7) were submitted to the bureau of CASP7. The server name is CIRCLE.

These results are those where models submitted to CASP 7 as predicted structures by about 60 prediction servers (all procedures are executed automatically with a computer program) participating in CASP 7 were evaluated separately by various groups. Although not officially announced by the CASP organizer, the ranking of the prediction server teams can be known.

The results of MAXSUB assessment in which the whole lengths of target structures were evaluated by an evaluation method MAXSUB are shown in FIG. 65A and B. The average point of all targets in this result was 45.516, and the perfect score was 51.2. On the ordinate, those participating in CAFASP5 (servers participating in CASP7) are listed in order of decreasing score. The results of each target are listed on the abscissa.
(http://fischerlab.cse.buffalo.edu/~mtgattie/CASPResults/CA SPSummaryTable.php?sort=Rank&reverse=0)

The evaluation in which each domain structure of a protein stereostructure was evaluated by MAXSUB_DOM is shown in FIG. 66A and B. The average point of all targets in this result was 46.626, and the perfect score was 52.577. On the ordinate, those participating in CAFASP5 are listed in order of decreasing score. The results of each target are listed on the abscissa.
(http://fischerlab.cse.buffalo.edu/~mtgattie/CASPResults/Mu ltipleDomainTable.php?sort=Rank&reverse=0)

FIG. 67A and B show the results of evaluation by TM-Score. (http://zhang.bioinformatics.ku.edu/casp7/). On the ordinate, the modeling server names (Predictors) in CASP7 are listed in order of decreasing TM-score from the score of a model submitted in the highest level. Items on the abscissa are as follows: N is the number of target models submitted, Rank is the ranking; and TM_1 is the TM score of the model submitted in the highest level. TM-score<0.17 is prediction near to random structure. TM-score=0 means a target not submitted, or the absence of overlapping between a right structure and its predicted structure.

Z score is Z score obtained from the corresponding score of each target. MS_1 is the MAXSub score of the model submitted in the highest level. MAXSub-score is calculated based on TM-score search engine. GDT_1 is the GDT score of the model submitted in the highest level. GDT-score is calculated based on TM-score search engine. RM_ 1 is RMSD between the model submitted in the highest level and the corresponding right structure. cov is the proportion (%) of amino acid residues in the prediction model based on the right structure. DGyr is a difference in radius gyration between the predicted model and the right structure. That is, DGyr= | Gyr_model-Gyr_native |, where Gyr_model and Gyr_native are radius gyrations of the predicted model and the right structure, respectively. When the radius gyration is calculated, the same set of amino acid residues is used in the predicted model and the right structure. NC is the number of models clashed (with atoms completely clashed against one another). The clashed model was defined by a Valencia's rule (Proteins, Suppl 7:27-45, 2005). That is, the clashed model includes a model having 4 or more strict clashes (Cα-Cα distance<1.9 angstroms) or a model having 50 bumps (with atom clashed against one another) (Cα-Cα distance<3.6 angstroms). All TM_B, MS_B, GDT_B, and RM_B show not only the results in the model submitted in the highest level but also the best results in the first to firth models.

The results of all targets evaluated with the value of (MaxSub+Tm-score+GDT_TS)/3 are shown in FIG. 68 (http://www.pdc.kth.se/-bjornw/casp7/targets/results/index_ all.html). In this evaluation site, the category in difficulty level of targets is divided into EASY (FIG. 69) and HARD (FIG. 70) for evaluation. In these tables, the modeling server names are shown on the ordinate, and N (number of targets on the test) and results by each of 4 evaluation methods (Max-Sub+TM-score+GDT_TS)/3, MaxSub, TM-score, and GDT_TS) are listed on the abscissa. In the evaluation methods on the abscissa, the following 4 results are contained: Top 1 shows the sum total of scores in the model submitted in the first position, and Top 5 shows the total sum of scores in 5 models in the first to fifth positions. Top 1 rank shows the average ranking based on the model submitted in the first position, and Top 5 rank shows the average ranking based on 5 models in the first to fifth positions.

FIGS. 71A and B, and 72 are tables showing the results of Robetta assessment (http://robetta.bakerlab.org/CASP7_eval/index.html). Evaluation is conducted with the total value of GDT_MM (GDT using MAMMOTH MaxSub at 1, 2, 3, 4 and 7 angstroms) and the standardized Z-score (Z score derived from GDT_MM in each target) by 2 methods. The results were divided by the following 4 categories: CM_easy (homology search at the BLAST level can be utilized), CM_hard (homology search at the PSI-BLAST level can be utilized), FR_H (slight homology with high reliability by 3D-Jury can be detected), and FR_A-NF (homology with high reliability by 3D-Jury cannot be detected), and these categories are in accordance with those used previously in CASP6. Domain division is judged with human eyes, and in each domain, evaluation is conducted. By so doing, it is possible to prevent the evaluated value from significantly changing with changing relative arrangement of domain. Accordingly, there is a possibility for more accurate evaluation to be carried out than in other evaluation sites with automatic evaluation. FIG. 71A and B show the results of First-GDT_MM (only the model submitted in the first position) of CM_easy target. The modeling server names are shown in the left, and the total and the score for each target number are shown on the abscissa. FIG. 72 shows the results of First-GDT_MM of CM_hard target.

The fact that the method of the present invention is novel is evident from the lack of similar literatures, and the higher position of our team than other teams participating in CASP7 indicates that protein stereostructures are accurately evaluated, and our study methods and computer programs contribute significantly to evaluation of protein structures. The fact that our method of evaluating protein stereostructures is good enables selection of best stereostructures from among those formed based on alignments obtained by various methods, to realize industrial utility with which protein stereostructures are accurately constructed.

EXAMPLE 2

The three inventors (Mayuko Takeda-Shitaka, Kazuhiko Kanou, Hideaki Umeyama) published a research paper (Nobuhiko Okada, Yorie Oi, Mayuko Takeda-Shitaka, Kazuhiko Kanou, Hideaki Umeyama, Takeshi Haneda, Tsuyoshi Miki, Sachiko Hosoya and Hirofumi Danbara, Identification of amino acid residues of Salmonella SlyA that are critical for transcriptional regulation. Microbiology (England), 2007, 153, 548-560.). The following outlines the research paper. The inventors demonstrated modeling a *Salmonella* SlyA dimer-DNA complex in the research paper.

It is estimated that *Salmonella* SlyA gene is a transcription factor essential for *Salmonella* to survive with resistance to chemical substances, organic solvents, oxidative stress, and virulent foreign factors. By homology modeling of a stereostructure of this gene, a dimer of two proteins and a complex having this dimer protein bound to a DNA were modeled. The protein elucidated in an experiment used in homology modeling is a protein whose PDB code is 1S3J. The homology in sequence between this reference protein and the objective modeled protein was 19% estimated to be a usually difficult level. Using the dimer and the DNA complex, amino acid residues which were estimated by visual check to influence transcriptional functions were subjected to Alanine residue substitution, and as a result, experimental results in which the dimer structure and the dimer/DNA complex structure are valid were obtained.

The fact that the dimer structure is valid is shown in Figure 5. (b) in the literature. The fact that the dimer/DNA complex structure is valid is shown in Figure 3. (b).

For amino acid residue substitution determined by visually checking a stereostructure obtained by homology modeling as described above, it was attempted at previously predicting a result of amino acid residue substitution by applying the score matrix of amino acid residues determined by Eq. (5). That is, for a specific amino acid residue, a score before amino acid residue substitution is calculated from Eq. (5). A score after amino acid substitution is calculated by changing the term indicative of the type of amino acid residue in Eq. (5). By comparing the resulting score before amino acid substitution with the score after amino acid substitution, whether the amino acid substitution occurs is predicted.

The prediction and explanation of a result of amino acid residue substitution in the *Salmonella* transcriptional regulator SlyA dimer by applying the score matrix of amino acid residues shown in claim 1 etc. are claim 6 etc. The prediction and explanation of a result of SlyA amino acid residue substitution in a *Salmonella* transcriptional regulator SlyA dimer/DNA complex by applying the score matrix of amino acid residues shown in claim 1 etc. are claim 6 etc. The prediction and explanation are valid, and as a result, the present invention can be used with novelty, inventive step and industrial applicability for the event in which the structural stability of the protein is involved. Claim 6 etc. can be used in converting the interaction among proteins into industrially useful interaction and to change the efficiency of an enzyme reaction to be useful in industry.

FIG. 73 shows a representation of claim 6 etc. Fig. 73 is a view showing protein-protein interaction of a transcriptional regulator SlyA dimer. The proteins in the SlyA dimer are shown by a white stick model and a gray ball-and-stick. These proteins are called molecules A and B respectively.

Examples of claim 6 etc. are shown below.

Table 1 below is a table in which an increase or decrease (plus, stability increase; minus, instability increase) in a score matrix of amino acid residues in claim 1 etc., is compared to a change (minus, instability indicator; plus, stability indicator) in experimental values by Alanine residue substitution in amino acid residues in a protein within the 6-angstrom border between protein molecules A and B for claim 3 etc.

In L1/A A, L1 indicates a Leucine residue at position 1 from the N-terminal amino acid of the protein, /A indicates protein molecule A, and the latter A indicates that a Leucine residue in molecule A is substituted by an Alanine residue.

The second column shows an increase or decrease (plus, stability increase; minus, instability increase) in the score matrix of amino acid residues in claim 1; the third column shows the proportion (plus, good storability of the residue in a specific place from the N-terminal residue; minus, poor storability) at which the residue is stored in a protein similar in sequence to SlyA; and the fourth column shows a change in an experimental value by Alanine residue substitution in amino acid residues of the protein (minus is indicative of a decrease in transcriptional activity as biological activity; plus is indicative of an increase in the biological activity;is indicative of few experimental data).

For example, in the example of

L126/A A -0.358 2 -84,

when L126 in the protein molecule A is replaced by A (Alanine residue), the score matrix of amino acid residues in claim 1 etc. shows instability increase in the protein; the third column shows that a protein similar in sequence can naturally occur; and the fourth column shows a decrease in transcriptional activity as biological activity. The score matrix of amino acid residues in claim 1 etc. explains experimental values.

In the example of

I114/A A +0.100 1 16,

the score matrix of amino acid residues in claim 1 etc. shows stability increase in the protein; the third column shows that a protein similar in sequence can naturally occur; and the fourth column shows an increase in transcriptional activity as biological activity. The score matrix of amino acid residues in claim 1 etc. explains experimental values.

Similar examples are also shown in:

| | | | | | |
|---|---|---|---|---|---|
| L129/A | A | -0.183 | 0 | -3, | |
| I130/A | A | -0.019 | -4 | -4, | |
| L133/A | A | -0.194 | -3 | -5, | |
| L9/B | A | -0.134 | -3 | -24, | |
| L126/B | A | -0.325 | 2 | -84, | and |
| L129/B | A | -0.126 | 0 | -3. | |

On the other hand, there are some examples wherein although the transcriptional activity is increased, the score matrix of amino acid residues in claim 1 etc. shows instability increase in the protein. The score matrix of amino acid residues in claim 1 etc. does not explain experimental values. It is highly possible that this case is correlated with phenomena such as structural change in the protein.

Examples are also shown in:

| | | | |
|---|---|---|---|
| L9/A A | +0.048 | -3 | -24, |
| L19/A A | +0.838 | 1 | -12, |
| I114/B A | -0.060 | 1 | 16, |
| I130/B A | +0.029 | -4 | -4, and |
| L133/B A | +0.003 | -3 | -5. |

In the 8 examples described above, the score matrix of amino acid residues in claim 1 etc. explains experimental values. In 5 examples, however, the score matrix of amino acid residues in claim 1 etc. does not explain experimental values. This is to predict transcriptional activity as biological activity with 62% accuracy upon substitution by A (Alanine residue). When it is considered that there has been no method of showing experimental guidelines by such an easy method, one step is forwarded, and it is also industrially worth utilizing the method. The examples where the fourth column is ---- are those giving experimental guidelines. The substitution explained this time is limited to substitution by A (Alanine residue), which however can naturally serve as an experimental guideline for substitution by other amino acid residues.

[Table 1]

Table in which an increase or decrease (plus, stability increase; minus, instability increase) in the score of a specific amino acid residue calculated by Eq. (5) in claim 4 etc., is compared to a change (minus, instability indicator; plus, stability indicator) in experimental values by Alanine residue substitution in amino acid residues in the protein within the 6-angstrom border between protein molecules A and B for claim 6 etc.

[Table 1-1]

| ALANINE RESIDUE SUBSTITUTION IN A OR B AMINO ACID CHAIN | INCREASE OR DECREASE IN SCORE MATRIX | RATIO AT WHICH AMINO ACID RESIDUES ARE PRESERVED | CHANGE IN EXPERIMENTAL VALUE BY ALANINE RESIDUE SUBSTITUTION IN AMINO ACID |
|---|---|---|---|
| L1/A A | 0.000 | -3 | - |
| E2/A A | 0.078 | -3 | - |
| P4/A A | 0.964 | 0 | - |
| L5/A A | -0.209 | -2 | - |
| G6/A A | 0.438 | 2 | - |
| S7/A A | 0.184 | 0 | - |
| D8/A A | 1.457 | -1 | - |
| L9/A A | 0.048 | -3 | -24 |
| R11/A A | -0.518 | -1 | - |
| V13/A A | 0.488 | 4 | - |
| R14/A A | 0.091 | -3 | - |
| I15/A A | 0.823 | -1 | - |
| W16/A A | -0.212 | -4 | - |
| R17/A A | -0.019 | -3 | - |
| L19/A A | 0.838 | 1 | -12 |
| D21/A A | -0.63 | -3 | - |
| Q31/A A | -0.588 | -1 | - |
| W34/A A | -0.224 | 2 | - |
| V35/A A | 0.471 | 0 | - |
| H38/A A | 0.065 | 0 | - |
| N39/A A | 0.569 | 0 | - |
| Q42/A A | -0.793 | -3 | - |
| E104/A A | -0.581 | -1 | - |
| I107/A A | 0.034 | 2 | - |
| H108/A A | -0.196 | 1 | - |
| T110/A A | 0.845 | -1 | - |
| R111/A A | 2.445 | -2 | - |
| G112/A A | 1.4 | 3 | - |
| E113/A A | -1.164 | 0 | - |

(continued)

| ALANINE RESIDUE SUBSTITUTION IN A OR B AMINO ACID CHAIN | INCREASE OR DECREASE IN SCORE MATRIX | RATIO AT WHICH AMINO ACID RESIDUES ARE PRESERVED | CHANGE IN EXPERIMENTAL VALUE BY ALANINE RESIDUE SUBSTITUTION IN AMINO ACID |
|---|---|---|---|
| 1114/A A | 0.1 | 1 | 16 |
| L115/A A | -0.224 | -4 | - |
| G117/A A | -0.581 | -1 | - |
| I118/A A | -0.651 | -3 | - |
| S119/A A | -2.084 | 0 | - |
| S120/A A | 0.114 | 1 | - |
| E121/A A | -0.558 | 2 | - |
| E122/A A | 0.834 | -2 | - |
| I123/A A | 0.148 | -2 | - |
| E124/A A | -0.875 | 1 | - |
| L125/A A | -0.138 | 2 | - |
| L126/A A | -0.358 | 2 | -84 |
| I127/A A | 0.007 | 0 | - |
| K128/A A | -0.662 | -1 | - |
| L129/A A | -0.183 | 0 | -3 |
| I130/A A | -0.019 | -4 | -4 |
| K132/A A | -0.823 | 1 | - |
| L133/A A | -0.194 | -3 | -5 |
| E134/AA | 0.197 | 1 | - |
| H135/A A | 0.218 | 2 | - |
| N136/A A | -0.112 | 1 | - |
| 1137/A A | -0.046 | 1 | - |
| M138/A A | 0.192 | 1 | - |

[Table 1-2]

| | | | |
|---|---|---|---|
| L1/B A | -0.465 | -3 | - |
| E2B A | -1.153 | -3 | - |
| S3/B A | 0.314 | 0 | - |
| P4/B A | 0.934 | 0 | - |
| L5/B A | -0.315 | -2 | - |
| G6/B A | 1.537 | 2 | - |
| S7/B A | 0.207 | 0 | - |
| D8/B A | 1.016 | -1 | - |
| L9/B A | -0.134 | -3 | - |
| R11/B A | -0.856 | -1 | - |

(continued)

| | | | |
|---|---|---|---|
| V13/B A | 0.38 | 4 | - |
| R14/B A | -0.891 | -3 | - |
| I15/B A | 0.583 | -1 | - |
| W16/B A | -0.312 | -4 | - |
| R17/B A | -1.029 | -3 | - |
| V35/B A | 0.517 | 0 | - |
| H108/B A | -0.142 | 1 | - |
| R111/B A | -0.163 | -2 | - |
| G112/B A | 1.435 | 3 | - |
| E113/B A | -0.944 | 0 | - |
| I114/B A | -0.06 | 1 | 16 |
| L115/B A | -0.099 | -4 | - |
| G117/B A | 1.689 | -1 | - |
| I118/B A | -0.651 | -3 | - |
| S119/B A | -2.005 | 0 | - |
| S120/B A | 0.029 | 1 | - |
| E121/B A | -0.34 | 2 | - |
| E122/B A | 1.481 | -2 | - |
| I123/B A | -0.013 | -2 | - |
| E124/B A | -0.791 | 1 | - |
| L125/B A | -0.282 | 2 | - |
| L126/B A | -0.325 | 2 | -84 |
| I127/B A | -0.173 | 0 | - |
| K128/B A | -0.706 | -1 | - |
| L129/B A | -0.126 | 0 | -3 |
| I130/B A | 0.029 | -4 | -4 |
| K132/B A | -0.825 | 1 | - |
| L133/BA | 0.003 | -3 | -5 |
| E134/B A | -0.463 | 1 | - |
| H135/B A | 0.739 | 2 | - |
| N136/B A | -0.523 | 1 | - |
| I137B A | 0 | 1 | - |

FIG. 74 shows a representation of claim 6 etc. Fig. 74 is a view showing interaction structure between a protein-protein complex of the transcriptional regulator SlyA dimer and DNA as a large ligand for the transcriptional regulator SlyA dimer. The proteins in the SlyA dimer are shown by a white stick model and a gray ball-and-stick. The DNA is illustrated in a gray CPK model.

Examples of claim 6 etc. are shown below. Table 2 below is a table in which an increase or decrease (plus, stability increase; minus, instability increase) in a score of a specific amino acid residue calculated from Eq. (5), is compared to a change (minus, instability indicator; plus, stability indicator) in experimental values by Alanine residue substitution in amino acid residues in the protein within the 6-angstrom border between the protein molecule (protein molecules A and B) and a DNA as a large ligand molecule for claim 6.

As shown below, a Proline residue in the protein molecule A is regarded important for structural maintenance, and by substitution of the amino acid residue by an Alanine residue, the score matrix of a specific amino acid residue calculated in Eq. (5) is plus to stabilize stability, but the transcriptional activity seems to decrease.

P61/A A +1.088 2 -43

By substituting a Serine residue in the protein molecule B below by an Alanine residue in the amino acid residues, the score matrix of amino acid residues in claim 1 is plus to increase stability, but the transcriptional activity seems to decrease. A structural change may be important.

S62/B A +0.147 -1 -99

The following shows that when D68 in the protein molecule A is substituted by A (Alanine residue), the score of the specific amino acid residue calculated from Eq. (5) shows instability increase in the protein, the third column shows that a protein similar in sequence does not naturally occur, and the fourth column shows a decrease in transcriptional activity as biological activity. The score of a specific amino acid residue calculated from Eq. (5) explains experimental values.

D68/A A -0.364 -3 -51

The following shows that when Q69 in the protein molecule B is substituted by A (Alanine residue), the score matrix of amino acid residues shows instability increase in the protein, the third column shows that a protein similar in sequence does not naturally occur, and the fourth column shows a decrease in transcriptional activity as biological activity. The score matrix of amino acid residues in claim 1 explains experimental values.

Q69/B A -0.568 -3 -7

The following shows that when R85 in the protein molecule B is substituted by A (Alanine residue), the score matrix of amino acid residues shows instability increase in the protein, the third column shows that a protein similar in sequence does not naturally occur, and the fourth column shows a decrease in transcriptional activity as biological activity. The score matrix of amino acid residues in claim 1 explains experimental values.

R85/B A -0.405 -3 -13

The following shows that when R85 in the protein molecule A is substituted by A (Alanine residue), the score matrix of amino acid residues shows instability increase in the protein, the third column shows that a protein similar in sequence does not naturally occur, and the fourth column shows a decrease in transcriptional activity as biological activity. The score matrix of amino acid residues in claim 1 etc. explains experimental values.

R85/A A -0.463 -3 -13

In the forgoing description, the score matrix of amino acid residues cannot explain experimental values in the former 2 examples but can explain those in the latter 4 examples. The score matrix of amino acid residues in claim 1 etc. can explain experimental values by 67%. This means that upon substitution by A (Alanine residues), the biological activity of transcriptional activity is predicted with 67% accuracy. When it is considered that there has been no method of showing experimental guidelines by such an easy method, one step is forwarded, and it is also industrially worth utilizing the method. The examples where the fourth column is ---- are those giving experimental guidelines. The substitution explained this time is limited to substitution by A (Alanine residue), which however can naturally serve as an experimental guideline for substitution by other amino acid residues.

Table 2: Table in which an increase or decrease (plus, stability increase; minus, instability increase) in the score matrix of amino acid residues in claim 4 etc., is compared to a change (minus, instability indicator; plus, stability indicator) in experimental values by Alanine residue substitution in amino acid residues in the protein within the 6-angstrom border between the protein molecule (protein molecules A and B) and a DNA as a large ligand molecule for claim 7 etc.

[Table 2]

| ALANINE RESIDUE SUBSTITUTION IN A OR B AMINO ACID CHAIN | INCREASE OR DECREASE IN SCORE MATRIX | RATIO AT WHICH AMINO ACID RESIDUES ARE PRESERVED | CHANGE IN EXPERIMENTAL VALUE BY ALANINE RESIDUE SUBSTITUTION IN AMINO ACID |
|---|---|---|---|
| L12/A A | -0.04 | 1 | - |
| V13/A A | 0.488 | 4 | - |
| R14/A A | 0.091 | -3 | - |
| I15/A A | 0.823 | -1 | - |
| W16/A A | -0.212 | -4 | - |
| R17/A A | -0.019 | -3 | - |
| A18/A A | 80.838 | 3 | - |
| L19/A A | 0.838 | 1 | - |
| 120/A A | 0.038 | 2 | - |
| D21/B A | -0.509 | -3 | - |
| K25/B A | -1.009 | 3 | - |
| L29/B A | -0.557 | -3 | - |
| T30/B A | -2.188 | -1 | - |
| Q31/B A | -0.04 | -1 | - |
| T32/B A | 0.046 | 5 | - |
| H33/B A | 0.105 | -1 | - |
| I58/B A | -0.239 | -3 | - |
| E59/B A | -1.211 | -3 | - |
| Q60/A A | -0.645 | 1 | - |
| P61/B A | 1.276 | 2 | -43 |
| P61/A A | 1.088 | 2 | -43 |
| S62/B A | 0.147 | -1 | -99 |
| D68/A A | -0.364 | -3 | -51 |
| Q69/B A | -0.568 | -3 | -7 |
| R78/A A | -1.716 | -3 | - |
| C81/B A | -0.789 | -3 | - |
| C81/A A | -1.012 | -3 | - |
| S83/B A | -0.491 | 1 | - |
| D84/A A | -1.201 | -4 | - |
| D84/B A | -1.208 | -4 | - |
| R85/B A | -0.405 | -3 | -13 |
| R85/A A | -0.463 | -3 | -13 |
| R89/A A | -1.952 | -1 | -7 |
| I90/A A | -1.016 | -3 | - |

EXAMPLE 3

Examples for claim 7 etc. are shown below. A profile showing the tendency for an experimental structure of a protein whose PDB ID is 1MB4 to be mutated by substitution with another amino acid residue was prepared in the method described in claim 1 etc. Using this profile, profiles similar to those calculated from previously calculated 4621 protein stereostructures were searched by a profile-profile alignment method. The results are shown in FIG. 75. Points shown in red indicate alignments obtained by the profile-profile alignment method. Homology (%) of amino acid residues in the resulting alignments is shown on the abscissa in the graph, and scores obtained by the profile-profile alignment method are shown on the ordinate.

Information on alignments plotted in the vicinity of 60% homology (sequence numbers: 1 and 2) are shown in FIG. 76. Among the similar amino acid sequences, those also similar in structural property could be searched by using the profile information in this manner. Further accurate searching can be effected by searching in consideration of the profile considering the genetic mutation tendency of amino acids formed from amino acid sequences.

EXAMPLE 4

Hereinafter, an example in claim 8 is shown. Using a molecular dynamics calculation (MD) program, an experimentally analyzed protein stereostructure (PDB) was gradually physically destroyed to generate 50 structures which were then subjected to energy minimization calculation. Using these data in one system, this MD calculation was conducted for 2859 PDB with 50% or less homology in amino acid sequence at a resolution of 2.0 or less, which are not single-stranded proteins or membrane proteins, to form 142950 protein stereostructures. This data set was subjected to the metropolis Monte Carlo method to optimize the coefficient of each amino acid described in Eq. 8 in claim 8. The maximum value of each coefficient was set at 2.0 and the minimum value at 0.0. The objective function in the metropolis Monte Carlo method is a coefficient of correlation between GDT_TS score and 3D1D score in individual systems.

This optimization was carried out 10 times, and as a result, the average coefficient for each amino acid was obtained. The optimized coefficient is as shown in FIG. 77. The coefficients for amino acids PRO, SER, and GLY are small. This means that the environment around these amino acids tends to be strongly present from PDB database, but do not exert a significant influence on protein stereostructure. Particularly, PRO forms a ring consisting of its side chain and main chain and is different in properties from other amino acid residues. GLY is characterized by being composed of only a main-chain structure. These amino acid residues have such significantly different properties from other amino acid residues, and thus their coefficient may be lowered.

Using the coefficient optimized in correlation coefficient in FIG. 77, targets on the test in the protein stereostructure prediction contest CASP7 (Critical Assessment of Techniques for Protein Structure Prediction, http://predictioncenter.org/casp7/) were verified. All structures submitted by the server team in CASP7 were optimized for side-chain arrangement by homology modeling software FAMS. For the structures, the sum total of GDT_TS scores of protein stereostructures by 2 methods from:

(1) those having the highest scores in Eqs. in claim 8 etc. where the coefficient of each amino acid is 1.0, and

(2) those having the highest scores in Eqs. in claim 8 etc. where the coefficient of each amino acid was the value in FIG. 75 above. As a result, the result in (1) was 5018.67, and the result in (2) was 5080.7, and the accuracy of structural evaluation was improved. That is, it is meant that by optimizing the coefficient for each amino acid residue, the accuracy of structural prediction can be further improved.

[Other Embodiments]

Although the invention has been described with respect to a specific embodiment for a complete and clear disclosure, the appended claims are not to be thus limited but are to be construed as embodying all modifications and alternative constructions that may occur to one skilled in the art which fairly fall within the basic teaching herein set forth.

For example, the apparatus for processing protein stereostructure 100 can be configured to perform processes in response to request from a client terminal, which is a separate unit, and return the process results to the client terminal.

All the automatic processes explained in the present embodiment can be, entirely or partially, carried out manually. Similarly, all the manual processes explained in the present embodiment can be, entirely or partially, carried out automatically by a known method.

The process procedures, the control procedures, specific names, information including registration data for each process and various parameters such as search conditions, display example, and database construction, mentioned in the description and drawings can be changed as required unless otherwise specified.

The constituent elements of the apparatus for processing protein stereostructure 100 are merely conceptual and may not necessarily physically resemble the structures shown in the drawings. For instance, the apparatus need not necessarily have the structure that is illustrated.

For example, the process functions performed by each device of the apparatus for processing protein stereostructure 100, especially the each process function performed by the control unit 102, can be entirely or partially realized by CPU and a computer program executed by the CPU or by a hardware using wired logic. The computer program, recorded on a recording medium to be described later, can be mechanically read by the apparatus for processing protein stereostructure 100 as the situation demands.

In other words, the storage unit 106 such as read-only memory (ROM) or hard disk (HD) stores the computer program that can work in coordination with OS to issue commands to the CPU and cause the CPU to perform various processes. The computer program is first loaded to the random access memory (RAM), and forms a control unit 102 in collaboration with the CPU. Alternatively, the computer program can be stored in any application program server connected to the apparatus for processing protein stereostructure 100 via the network 300, and can be fully or partially loaded as the situation demands.

The computer-readable recording medium on which the computer program can be stored may be a portable type such as flexible disk, magneto optic (MO) disk, ROM, erasable programmable ROM (EPROM), electrically erasable programmable ROM (EEPROM), compact disk-read-only memory (CD-ROM), digital versatile disk (DVD), or a communication medium that stores the computer program for a short term such as communication channels or carrier waves that transmit the computer program over the network 300 such as local area network (LAN), wide area network (WAN), and the Internet.

Computer program refers to a data processing method written in any computer language and written method, and can have software codes and binary codes in any format. The computer program can be a dispersed form in the form of a plurality of modules or libraries, or can perform various functions in collaboration with a different program such as the OS. Any known configuration in the each device according to the embodiment can be used for reading the recording medium. Similarly, any known process procedure for reading or installing the computer program can be used.

The storage unit 106 is a fixed disk device such as RAM, ROM, and HD or flexible disk, optical disk, and stores therein various programs, tables, databases (such as the protein stereostructure information file 106a, the environment change information file 106b, and the standard deviation information file 106c), and files required for various processes.

The apparatus for processing protein stereostructure 100 can also be connected to any existing personal computer, workstation, etc. and can be operated by executing software (that includes computer program, data, etc.) that implements the method according to the present invention in the personal computer or workstation.

The distribution and integration of the apparatus for processing protein stereostructure 100 are not limited to those illustrated in the figures. The device as a whole or in parts can be functionally or physically distributed or integrated in an arbitrary unit according to various attachments or how the device is to be used.

The network 300 has function of connecting the apparatus for processing protein stereostructure 100 with the external device 200, and may at least include internet, intranet, LAN (wired/wireless), VAN, personal computer communication network, public telephone network (analog/digital), leased circuit (analog/digital), cable TV network, mobile phone switching network/ packet-switching data network by IMT2000, GSM, PDC/PDC-P, or the like, radio paging network, local wireless network such as Bluetooth (R), PHS network, satellite communication network such as CS, BS, and ISDB, and the like. The system can transmit and receive various data through any wired or wireless network.

## INDUSTRIAL APPLICABILITY

As described above, the apparatus for processing protein stereostructure, the method of processing protein stereostructure and the program according to the present invention can be used in various fields in industry, particularly in the fields of pharmaceutical, medical and life science industries and are extremely useful.

## Claims

**1.** An apparatus for processing protein stereostructure, comprising:

a control unit and a storage unit, which calculates a score matrix that is physical quantity indicating whether amino acid residues in a protein stereostructure are energetically stable in a specific environment, wherein the storage unit includes:
protein stereostructure information that defines stereostructure coordinates of a protein composed of a plurality of the amino acid residues, and
the control unit includes:
a mutated stereostructure-predicting unit that predicts protein stereostructure information after an arbitrary amino acid residue A in the protein stereostructure information stored in the storage unit is mutated into another amino acid residue a,

an environment change information-collecting unit that from the protein stereostructure information after mutation predicted by the mutated stereostructure-predicting unit, collects the amino acid residue A, the amino acid residue a, environment information P around the amino acid residue A before mutation, and environmental data p around the amino acid residue a after mutation upon changing from the environmental data P into the environmental data p related to one another as environment change information, thereby storing the environment change information in the storage unit,
a standard deviation-calculating unit that while assuming that a plurality of pieces of the environment information p after mutation relative to the environment information P in the environment change information collected by the environment information-collecting unit follow normal distribution, calculates standard deviation $\sigma$ (P) for the environment information P and relates the environment information P to the standard deviation $\sigma$ (P) to store the standard deviation $\sigma$ (P)in the storage unit, and
a score matrix-calculating unit that when the number (N(i|j)) of amino acid residues i present in a specific environment information j upon calculation of the score matrix, corrects the N(i|j) to calculate the score matrix by using a weighting parameter considering standard deviation $\sigma$ (J) for environment information J after mutation relative to the environment information j stored by the standard deviation-calculating unit, and considering the standard deviation and an absolute value in difference between the environment information other than the environment information j and the environment information j.

**2.** The apparatus for processing protein stereostructure according to claim 1, wherein
the environment information is defined by the ratio of area occupied by polar atoms (fraction polar) that is the ratio of polar atoms occupying the surface of the amino acid residues, the fraction of buried area of the surface of the amino acid residues (fraction buried), and secondary structure information.

**3.** The apparatus for processing protein stereostructure according to claim 2,
wherein the score matrix-calculating unit uses the following equation as the weighting parameter:

[Eq. 1]

$$w(polar, buried, p, b) = \exp\left(\frac{-p^2}{2 \times \sigma_{polar}^2}\right) \times \exp\left(\frac{-b^2}{2 \times \sigma_{buried}^2}\right)$$

wherein w (polar, buried, p, b) is the weighting parameter wherein the fraction polar (polar) is shifted by p and the fraction buried (buried) is shifted by b in the environment information defined by 3 parameters consisting of fraction polar (polar), fraction buried (buried), and secondary structure information (ss); p is the absolute value in difference between the fraction polar before and after mutation; b is the absolute value in difference between the fraction buried before and after mutation; $\sigma_{polar}$ is the standard deviation in the fraction polar obtained from information on the amino acid residues after mutation; and $\sigma_{buried}$ is the standard deviation in fraction buried obtained from information the amino acid residues after mutation.

**4.** The apparatus for processing protein stereostructure according to claim 3, wherein
the score matrix-calculating unit calculates, as the score matrix, SCORE (AA|ss, polar, buried) by using the following equation:

[Eq. 2]

$$P(AA \mid ss, polar, buried) = \frac{\sum_{m,n} w(polar, buried, m, n) N(AA \mid ss, polar + m, buried + n)}{\sum_{aa} \sum_{m,n} w(polar, buried, m, n) N(aa \mid ss, polar + m, buried + n)}$$

$$SCORE(AA \mid ss, polar, buried) = \log\left(\frac{P(AA \mid ss, polar, buried)}{P(AA)}\right)$$

wherein P (AA|ss, polar, buried) is the ratio of amino acid residue AA present in the environment information consisting of the secondary structure information (ss), the fraction polar (polar), and the fraction buried (buried).

**5.** The apparatus for processing protein stereostructure according to claim 1, further comprising:

an accuracy evaluating unit that calculates the score matrix for each of amino acid residues, and thereof obtains the sum total, thereby evaluating the accuracy of the protein stereostructure information.

**6.** The apparatus for processing protein stereostructure according to claim 1, wherein
the protein stereostructure information includes stereostructure information on a protein complex of a protein-interacting ligand and the protein or a protein complex into which a ligand has been inserted with a docking program, and amino acid residues around the ligand are changed thereby calculating the score matrix, to search for the state of.amino acid residue mutation wherein the highest score matrix is calculated.

**7.** The apparatus for processing protein stereostructure according to claim 1, wherein
the environment change information on the amino acid residues is used, and the score matrix is used, to search for protein stereostructures similar to one another in folding state by dynamic programming (DP).

**8.** The apparatus for processing protein stereostructure according to claim 1, wherein
3D1Dscore ij shown in the following equation using, as correction term k(i), the strength of influence of the amino acid residues i on protein folding structure is calculated as the score matrix:

[Eq. 3]

$$3D1D\,score\;ij = k(i)\cdot \ln\left(\frac{P(i\mid j)}{Pi}\right)$$

$$P(i\mid j) = \frac{N(i\mid j)}{\sum_{a=1}^{20} N(a\mid j)} \qquad Pi = \frac{N(i)}{\sum_{a=1}^{20} N(a)}$$

wherein P(i|j) is the frequency of appearance of the amino acid residues i in the environment information j; Pi is the ratio of the amino acid residues i present regardless of the environment; N(i|j) is the number of the amino acid residues i observed in the environment information j; and N(i) is the number of the amino acid residues i observed.

**9.** The apparatus for processing protein stereostructure according to claim 1, wherein
the total sum of numerical values is calculated by the score matrix for amino acid residues around a specific amino acid residue of a protein for evaluating the modification of functions of the protein and the degree of contribution of the specific amino acid residue to functions of the protein.

**10.** A method for processing a protein stereostructure executed by an apparatus for processing protein stereostructure including:

a control unit and a storage unit, which calculates a score matrix that is physical quantity indicating whether amino acid residues in a protein stereostructure are energetically stable in a specific environment, wherein the storage unit includes:
protein stereostructure information that defines stereostructure coordinates of a protein composed of a plurality of the amino acid residues, and
the method comprises:
a mutated stereostructure-predicting step of predicting protein stereostructure information after an arbitrary amino acid residue A in the protein stereostructure information stored in the storage unit is mutated into another amino acid residue a;
an environment change information-collecting step of collecting the amino acid residue A, the amino acid residue a, environment information P around the amino acid residue A before mutation, and environmental data p around the amino acid residue a after mutation upon changing from the environmental data P into the environmental data p related to one another as environment change information, thereby storing the environment change information in the storage unit from the protein stereostructure information after mutation predicted at the mutated stereostructure-predicting step;
a standard deviation-calculating step of calculating standard deviation σ (P) for the environment information P and relating the environment information P to the standard deviation σ (P) to store the standard deviation σ (P) in the storage unit while assuming that a plurality of pieces of the environment information p after mutation relative to the environment information P in the environment change information collected at the environment information-collecting step follow normal distribution; and
a score matrix-calculating step of correcting the N(i|j) to calculate the score matrix by using a weighting parameter considering standard deviation σ (J) for environment information J after mutation relative to the environment information j stored at the standard deviation-calculating step, and considering the standard deviation and an absolute value in difference between the environment information other than the environment information j and the environment information j when the number (N(i|j)) of amino acid residues i present in a specific environment information j upon calculation of the score matrix,
wherein the steps are executed by the control unit.

**11.** The method for processing a protein stereostructure according to claim 10, wherein
the environment information is defined by the ratio of area occupied by polar atoms (fraction polar) that is the ratio of polar atoms occupying the surface of the amino acid residues, the fraction of buried area of the surface of the amino acid residues (fraction buried), and secondary structure information.

**12.** The method for processing a protein stereostructure according to claim 11, wherein
the score matrix-calculating step uses the following equation as the weighting parameter:

[Eq. 4]

$$w(polar, buried, p, b) = \exp\left(\frac{-p^2}{2\times\sigma_{polar}^{\ 2}}\right)\times\exp\left(\frac{-b^2}{2\times\sigma_{buried}^{\ 2}}\right)$$

wherein w (polar, buried, p, b) is the weighting parameter wherein the fraction polar (polar) is shifted by p and the fraction buried (buried) is shifted by b in the environment information defined by 3 parameters consisting of fraction polar (polar), fraction buried (buried), and secondary structure information (ss); p is the absolute value in difference between the fraction polar before and after mutation; b is the absolute value in difference between the fraction buried before and after mutation; $\sigma_{polar}$ is the standard deviation in the fraction polar obtained from information on the amino acid residues after mutation; and $\sigma_{buried}$ is the standard deviation in fraction buried obtained from information the amino acid residues after mutation.

**13.** The method for processing a protein stereostructure according to claim 12, wherein
the score matrix-calculating step includes calculating, as the score matrix, SCORE (AA|ss, polar, buried) by using the following equation:

[Eq. 5]

$$P(AA \mid ss, polar, buried) = \frac{\sum_{m,n} w(polar, buried, m, n) N(AA \mid ss, polar+m, buried+n)}{\sum_{aa}\sum_{m,n} w(polar, buried, m, n) N(aa \mid ss, polar+m, buried+n)}$$

$$SCORE(AA \mid ss, polar, buried) = \log\left(\frac{P(AA \mid ss, polar, buried)}{P(AA)}\right)$$

wherein P (AA|ss, polar, buried) is the ratio of amino acid residue AA present in the environment information consisting of the secondary structure information (ss), the fraction polar (polar), and the fraction buried (buried).

**14.** The method for processing a protein stereostructure according to claim 10, further comprising:

an accuracy evaluating step of calculating the score matrix for each of amino acid residues, and thereof obtains the sum total, thereby evaluating the accuracy of the protein stereostructure information.

**15.** The method for processing a protein stereostructure according to claim 10, wherein
the protein stereostructure information includes stereostructure information on a protein complex of a protein-interacting ligand and the protein or a protein complex into which a ligand has been inserted with a docking program, and amino acid residues around the ligand are changed thereby calculating the score matrix, to search for the state of amino acid residue mutation wherein the highest score matrix is calculated.

**16.** The method for processing a protein stereostructure according to claim 10, wherein
the environment change information on the amino acid residues is used, and the score matrix is used, to search for protein stereostructures similar to one another in folding state by dynamic programming (DP).

**17.** The method for processing a protein stereostructure according to claim 10, wherein
3D1Dscore ij shown in the following equation using, as correction term k(i), the strength of influence of the amino acid residues i on protein folding structure is calculated as the score matrix:

[Eq. 6]

$$3D1D\,score\ ij = k(i)\cdot \ln\left(\frac{P(i\mid j)}{Pi}\right)$$

$$P(i\mid j) = \frac{N(i\mid j)}{\sum_{a=1}^{20} N(a\mid j)} \qquad Pi = \frac{N(i)}{\sum_{a=1}^{20} N(a)}$$

wherein P(i|j) is the frequency of appearance of the amino acid residues i in the environment information j; Pi is the ratio of the amino acid residues i present regardless of the environment; N(i|j) is the number of the amino acid residues i observed in the environment information j; and N(i) is the number of the amino acid residues i observed.

**18.** The method for processing a protein stereostructure according to claim 10, wherein
the total sum of numerical values is calculated by the score matrix for amino acid residues around a specific amino acid residue of a protein for evaluating the modification of functions of the protein and the degree of contribution of the specific amino acid residue to functions of the protein.

**19.** A computer program executed by an apparatus for processing protein stereostructure including:

a control unit and a storage unit, which calculates a score matrix that is physical quantity indicating whether amino acid residues in a protein stereostructure are energetically stable in a specific environment, wherein the storage unit includes:
protein stereostructure information that defines stereostructure coordinates of a protein composed of a plurality of the amino acid residues and,
the computer program comprises:
a mutated stereostructure-predicting step of predicting protein stereostructure information after an arbitrary amino acid residue A in the protein stereostructure information stored in the storage unit is mutated into another amino acid residue a;
an environment change information-collecting step of, collecting the amino acid residue A, the amino acid residue a, environment information P around the amino acid residue A before mutation, and environmental data p around the amino acid residue a after mutation upon changing from the environmental data P into the environmental data p related to one another as environment change information, thereby storing the environment change information in the storage unit from the protein stereostructure information after mutation predicted at the mutated stereostructure-predicting step;
a standard deviation-calculating step of calculating standard deviation σ (P) for the environment information P and relates the environment information P to the standard deviation σ (P) to store the standard deviation σ (P)in the storage unit while assuming that a plurality of pieces of the environment information p after mutation relative to the environment information P in the environment change information collected at the environment information-collecting step follow normal distribution; and
a score matrix-calculating step of correcting the N(i|j) to calculate the score matrix by using a weighting parameter considering standard deviation σ (J) for environment information J after mutation relative to the environment information j stored at the standard deviation-calculating step, and considering the standard deviation and an absolute value in difference between the environment information other than the environment information j and the environment information j when the number (N(i|j)) of amino acid residues i present in a specific environment information j upon calculation of the score matrix,
wherein the steps are executed by the control unit.

**20.** The computer program according to claim 19, wherein
the environment information is defined by the ratio of area occupied by polar atoms (fraction polar) that is the ratio of polar atoms occupying the surface of the amino acid residues, the fraction of buried area of the surface of the amino acid residues (fraction buried), and secondary structure information.

**21.** The computer program according to claim 20,
wherein the score matrix-calculating step uses the following equation as the weighting parameter:

[Eq. 7]

$$w(polar,buried,p,b) = \exp\left(\frac{-p^2}{2\times\sigma_{polar}{}^2}\right)\times\exp\left(\frac{-b^2}{2\times\sigma_{buried}{}^2}\right)$$

wherein w (polar, buried, p, b) is the weighting parameter wherein the fraction polar (polar) is shifted by p and the fraction buried (buried) is shifted by b in the environment information defined by 3 parameters consisting of fraction polar (polar), fraction buried (buried), and secondary structure information (ss); p is the absolute value in difference between the fraction polar before and after mutation; b is the absolute value in difference between the fraction buried before and after mutation; $\sigma_{polar}$ is the standard deviation in the fraction polar obtained from information on the amino acid residues after mutation; and $\sigma_{buried}$ is the standard deviation in fraction buried obtained from information the amino acid residues after mutation.

**22.** The computer program according to claim 21, wherein
the score matrix-calculating step includes calculating, as the score matrix, SCORE (AA|ss, polar, buried) by using the following equation:

[Eq. 8]

$$P(AA \mid ss,polar,buried) = \frac{\sum_{m,n} w(polar,buried,m,n)N(AA \mid ss,polar+m,buried+n)}{\sum_{aa}\sum_{m,n} w(polar,buried,m,n)N(aa \mid ss,polar+m,buried+n)}$$

$$SCORE(AA \mid ss,polar,buried) = \log\left(\frac{P(AA \mid ss,polar,buried)}{P(AA)}\right)$$

wherein P (AA|ss, polar, buried) is the ratio of amino acid residue AA present in the environment information consisting of the secondary structure information (ss), the fraction polar (polar), and the fraction buried (buried).

**23.** The computer program according to claim 19, further comprising:

an accuracy evaluating step of calculating the score matrix for each of amino acid residues, and thereof obtains the sum total, thereby evaluating the accuracy of the protein stereostructure information.

**24.** The computer program according to claim 19, wherein
the protein stereostructure information includes stereostructure information on a protein complex of a protein-interacting ligand and the protein or a protein complex into which a ligand has been inserted with a docking program, and amino acid residues around the ligand are changed thereby calculating the score matrix, to search for the state of amino acid residue mutation wherein the highest score matrix is calculated.

**25.** The computer program according to claim 19, wherein
the environment change information on the amino acid residues is used, and the score matrix is used, to search for protein stereostructures similar to one another in folding state by dynamic programming (DP).

**26.** The computer program according to claim 19, wherein
3D1Dscore ij shown in the following equation using, as correction term k(i), the strength of influence of the amino acid residues i on protein folding structure is calculated as the score matrix:

[Eq. 9]

$$3D1D\,score\ ij = k(i) \cdot \ln\left(\frac{P(i \mid j)}{Pi}\right)$$

$$P(i \mid j) = \frac{N(i \mid j)}{\sum_{a=1}^{20} N(a \mid j)} \qquad Pi = \frac{N(i)}{\sum_{a=1}^{20} N(a)}$$

wherein P(i|j) is the frequency of appearance of the amino acid residues i in the environment information j; Pi is the ratio of the amino acid residues i present regardless of the environment; N(i|j) is the number of the amino acid residues i observed in the environment information j; and N(i) is the number of the amino acid residues i observed.

**27.** The computer program according to claim 19, wherein
the total sum of numerical values is calculated by the score matrix for amino acid residues around a specific amino acid residue of a protein for evaluating the modification of functions of the protein and the degree of contribution of the specific amino acid residue to functions of the protein.

FIG.1

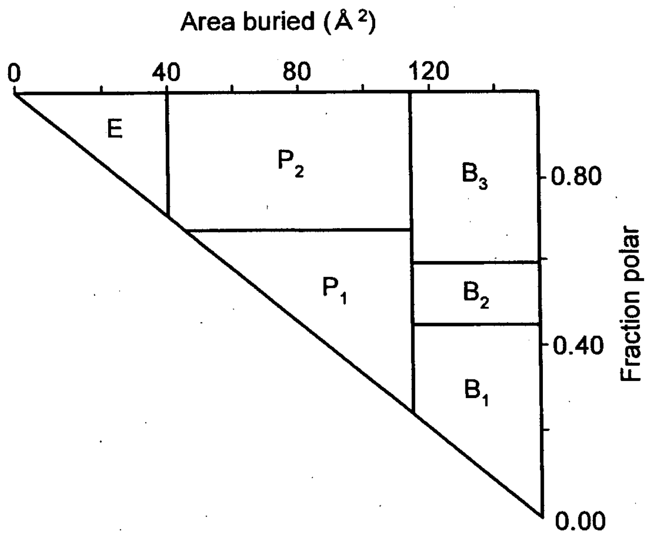
Area buried (Å 2)
0
40
80
120
E
P2
B3
0.80
P1
B2
0.40
B1
0.00
Fraction polar

FIG.2

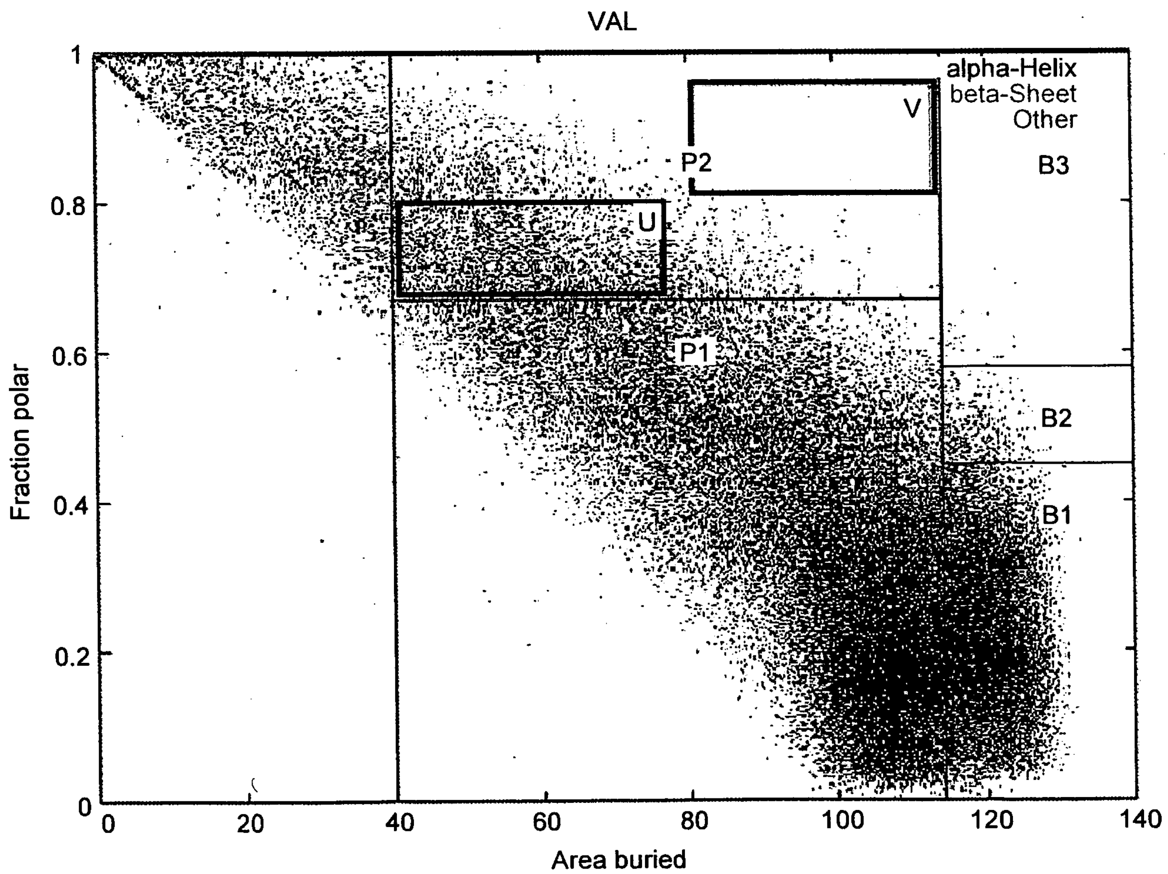
VAL
1
0.8
0.6
0.4
0.2
0
alpha-Helix
beta-Sheet
Other
V
P2
B3
U
P1
B2
B1
Fraction polar
0
20
40
60
80
100
120
140
Area buried

FIG.3

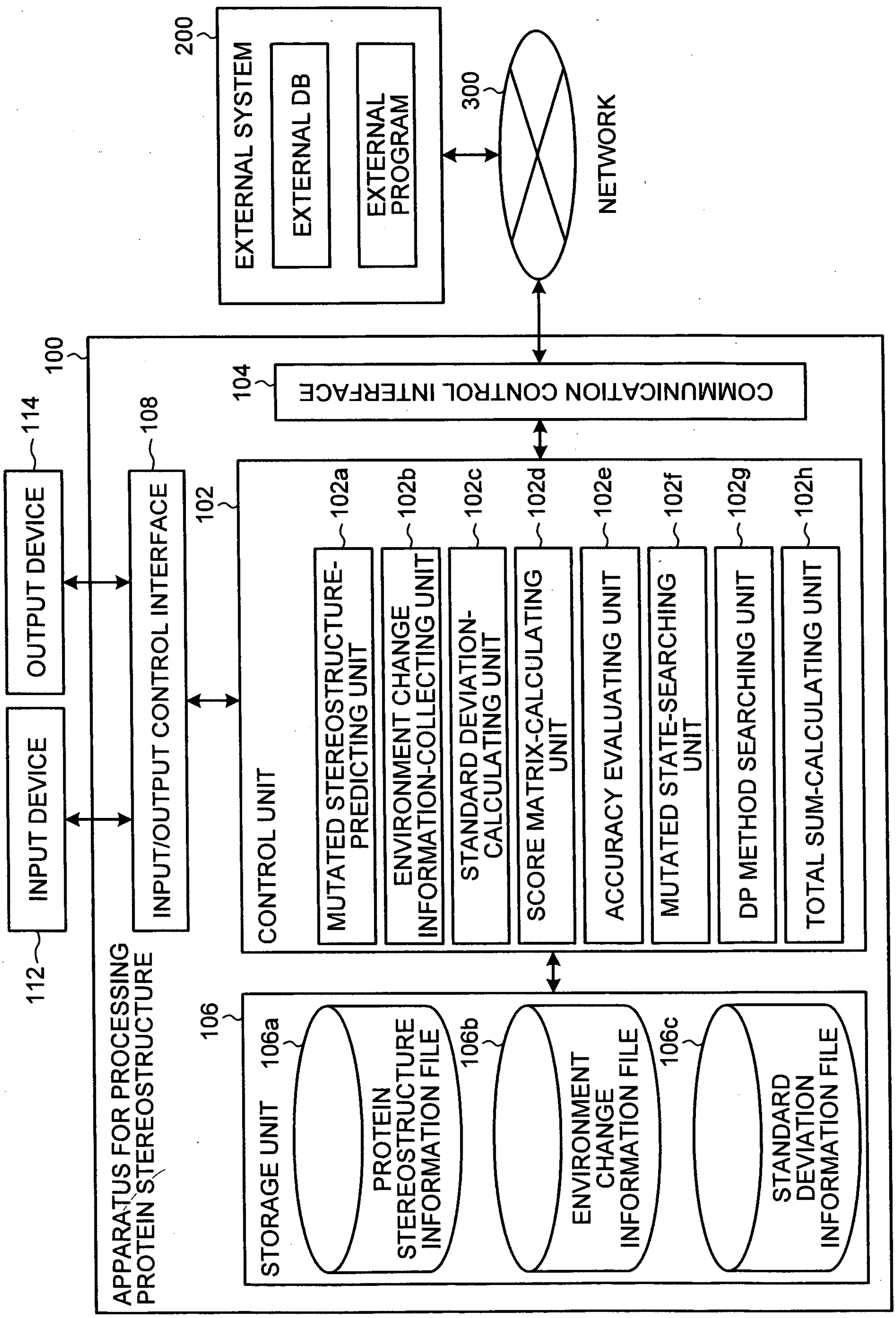
200
EXTERNAL SYSTEM
EXTERNAL DB
EXTERNAL PROGRAM
300
NETWORK
100
APPARATUS FOR PROCESSING PROTEIN STEREOSTRUCTURE
112
INPUT DEVICE
114
OUTPUT DEVICE
108
INPUT/OUTPUT CONTROL INTERFACE
104
COMMUNICATION CONTROL INTERFACE
102
CONTROL UNIT
102a
MUTATED STEREOSTRUCTURE-PREDICTING UNIT
102b
ENVIRONMENT CHANGE INFORMATION-COLLECTING UNIT
102c
STANDARD DEVIATION-CALCULATING UNIT
102d
SCORE MATRIX-CALCULATING UNIT
102e
ACCURACY EVALUATING UNIT
102f
MUTATED STATE-SEARCHING UNIT
102g
DP METHOD SEARCHING UNIT
102h
TOTAL SUM-CALCULATING UNIT
106
STORAGE UNIT
106a
PROTEIN STEREOSTRUCTURE INFORMATION FILE
106b
ENVIRONMENT CHANGE INFORMATION FILE
106c
STANDARD DEVIATION INFORMATION FILE

FIG.4

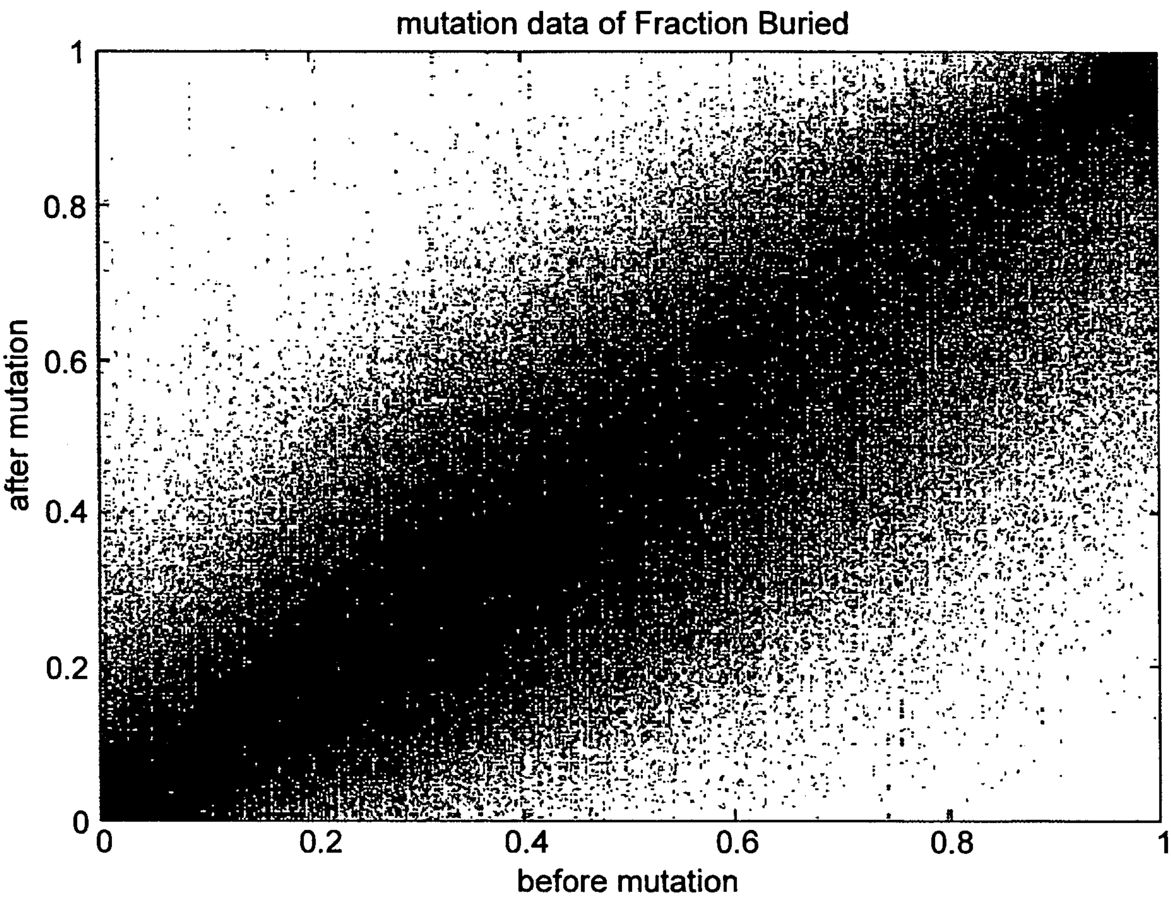
mutation data of Fraction Buried
after mutation
before mutation
0
0.2
0.4
0.6
0.8
1

FIG.5

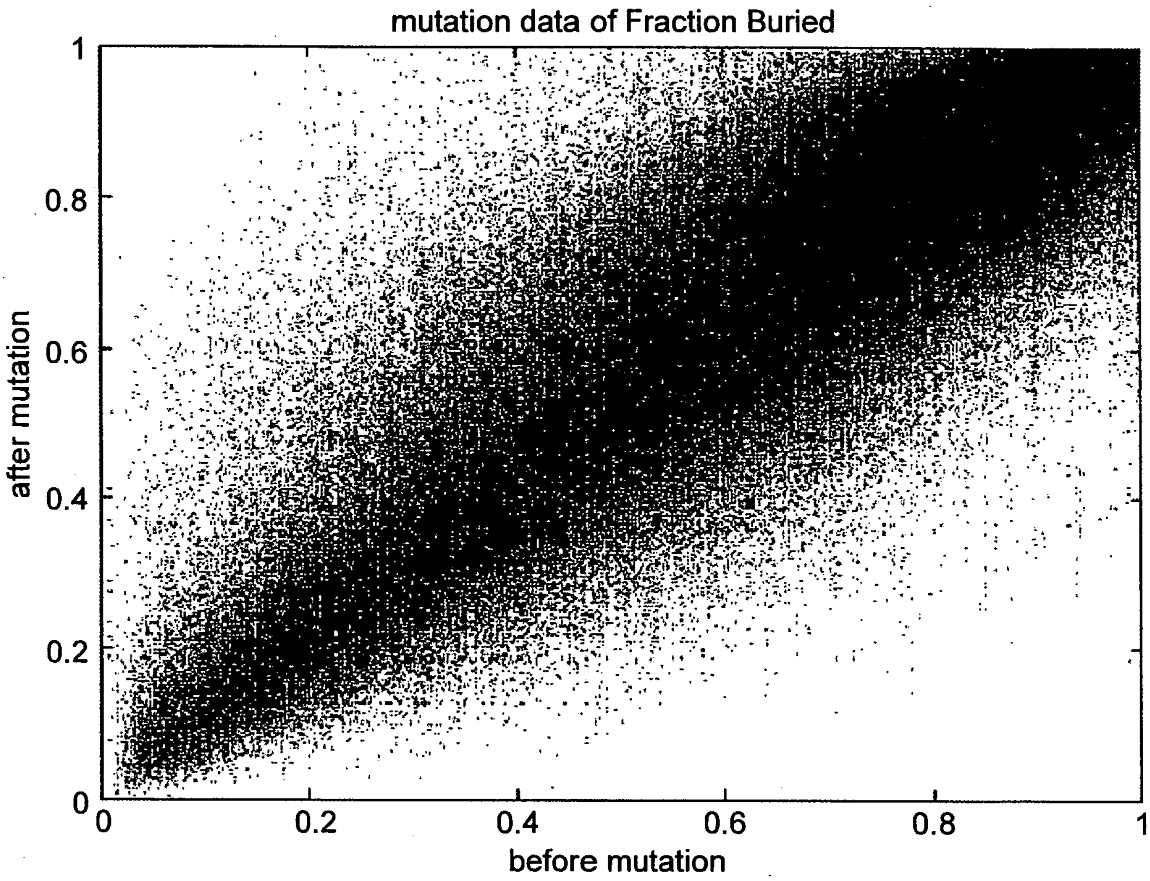
mutation data of Fraction Buried
after mutation
before mutation
0
0.2
0.4
0.6
0.8
1
0
0.2
0.4
0.6
0.8
1

FIG.6

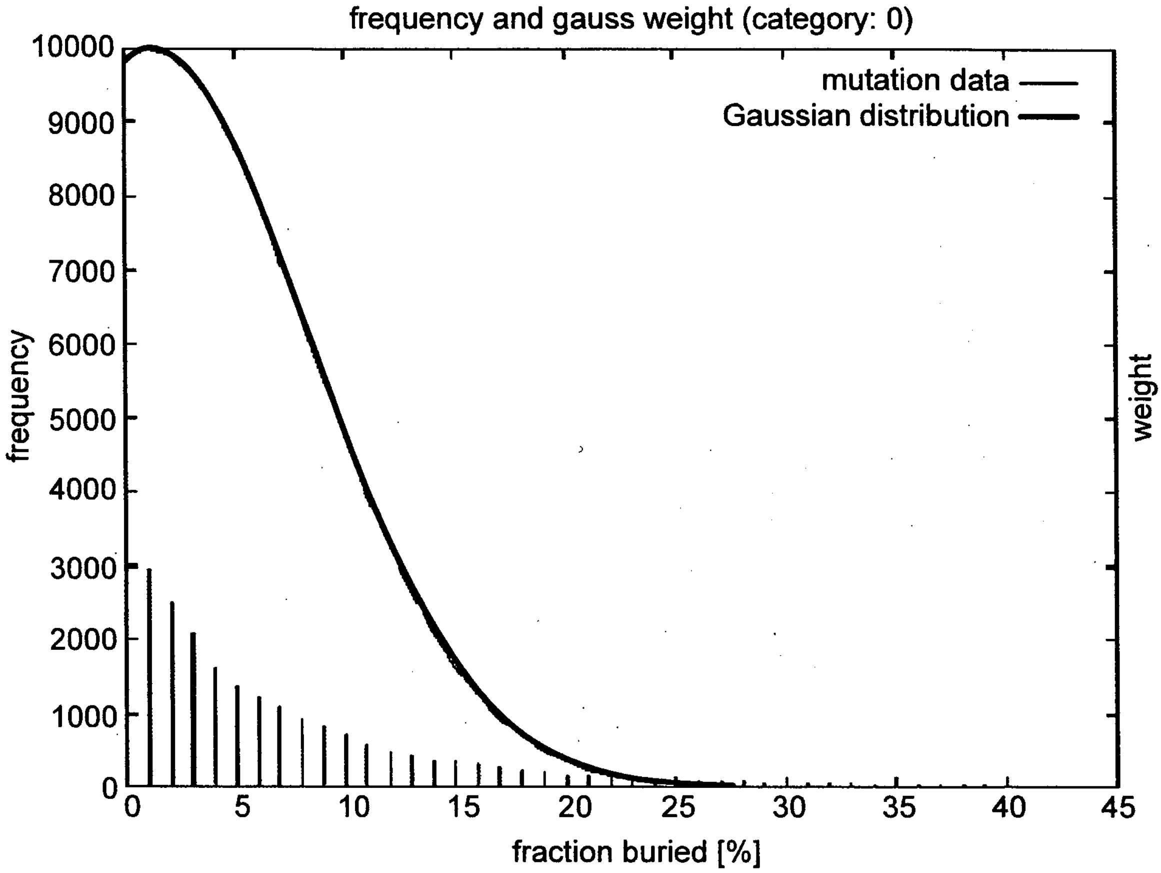
frequency and gauss weight (category: 0)
mutation data
Gaussian distribution
frequency
weight
fraction buried [%]
10000
9000
8000
7000
6000
5000
4000
3000
2000
1000
0
0
5
10
15
20
25
30
35
40
45

FIG.7

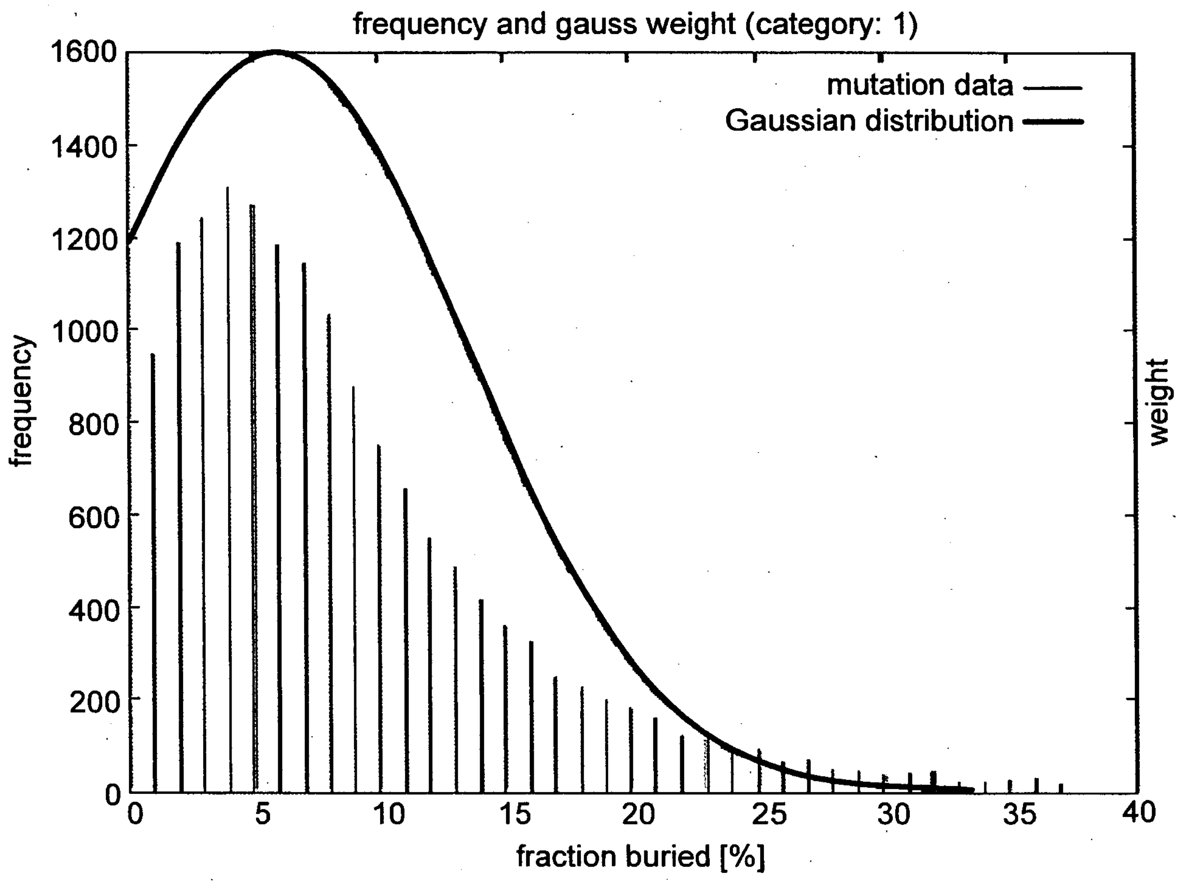
frequency and gauss weight (category: 1)
mutation data
Gaussian distribution
frequency
weight
fraction buried [%]
0
200
400
600
800
1000
1200
1400
1600
0
5
10
15
20
25
30
35
40

FIG.8

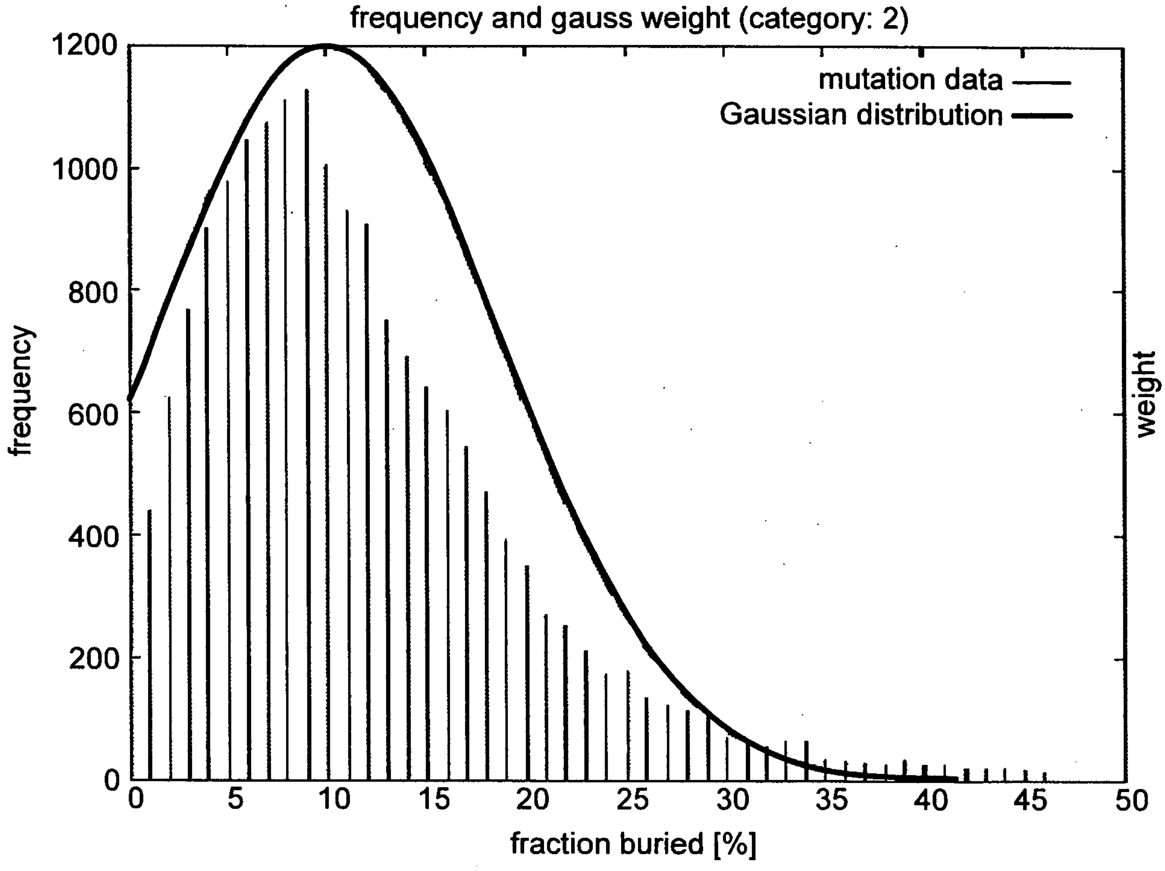
frequency and gauss weight (category: 2)
mutation data
Gaussian distribution
frequency
weight
fraction buried [%]
0
200
400
600
800
1000
1200
0
5
10
15
20
25
30
35
40
45
50

FIG.9

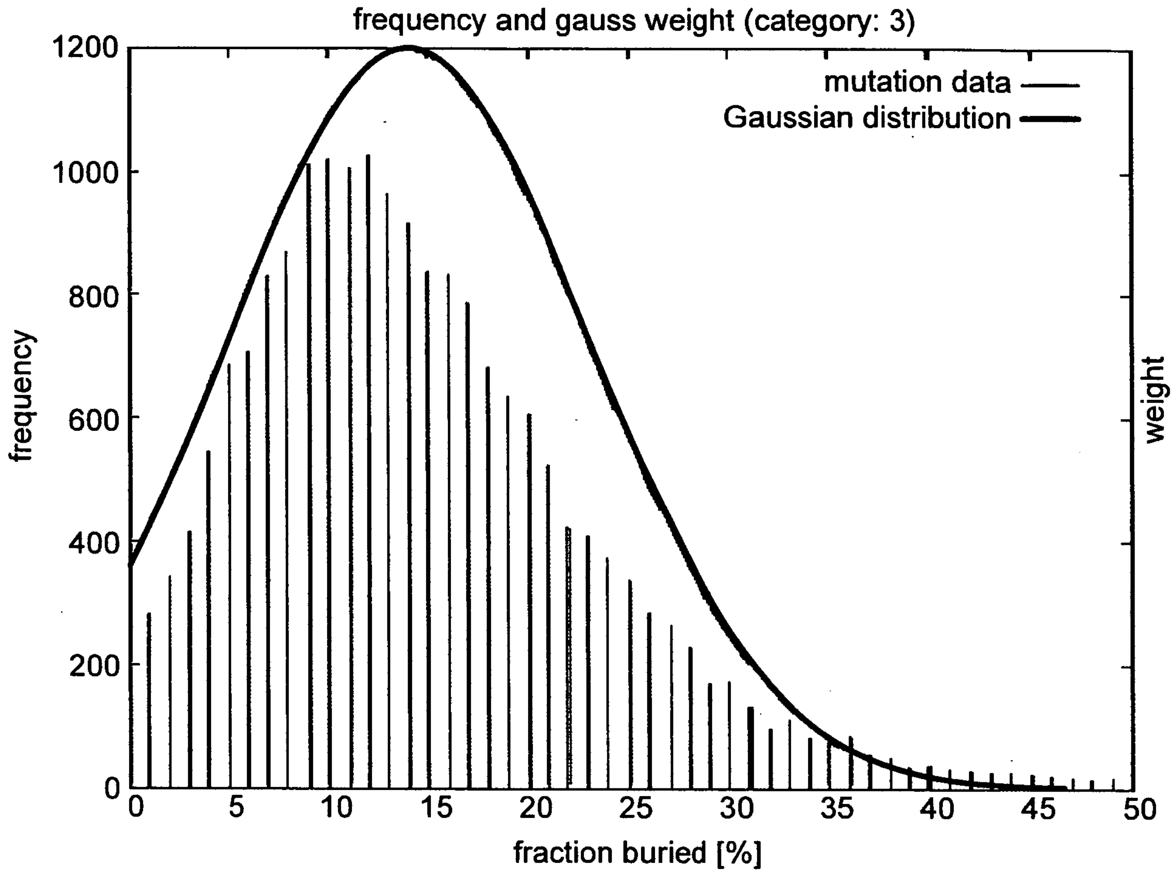
frequency and gauss weight (category: 3)
mutation data
Gaussian distribution
frequency
weight
fraction buried [%]
1200
1000
800
600
400
200
0
0
5
10
15
20
25
30
35
40
45
50

FIG.10

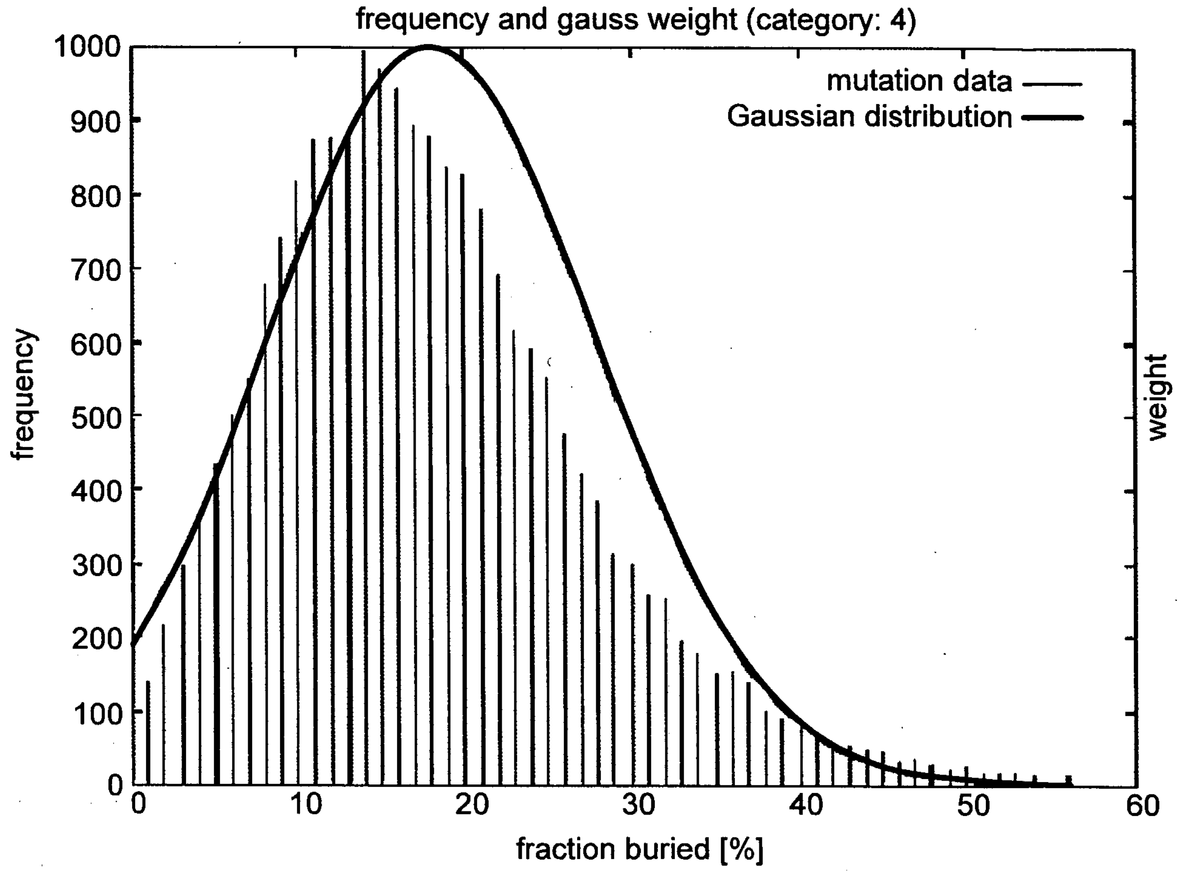
frequency and gauss weight (category: 4)
mutation data
Gaussian distribution
frequency
weight
fraction buried [%]
0
100
200
300
400
500
600
700
800
900
1000
0
10
20
30
40
50
60

FIG.11

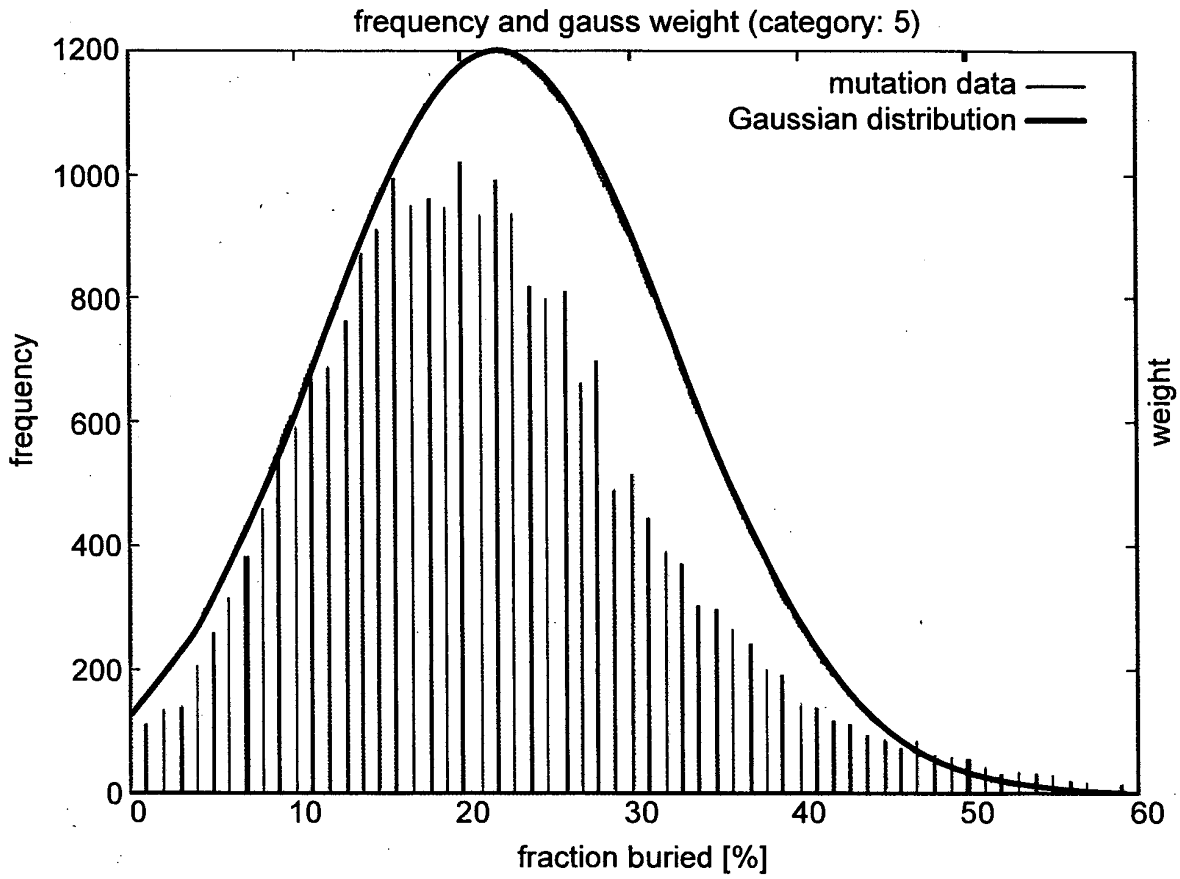
frequency and gauss weight (category: 5)
mutation data
Gaussian distribution
1200
1000
800
600
400
200
0
frequency
weight
0
10
20
30
40
50
60
fraction buried [%]

FIG.12

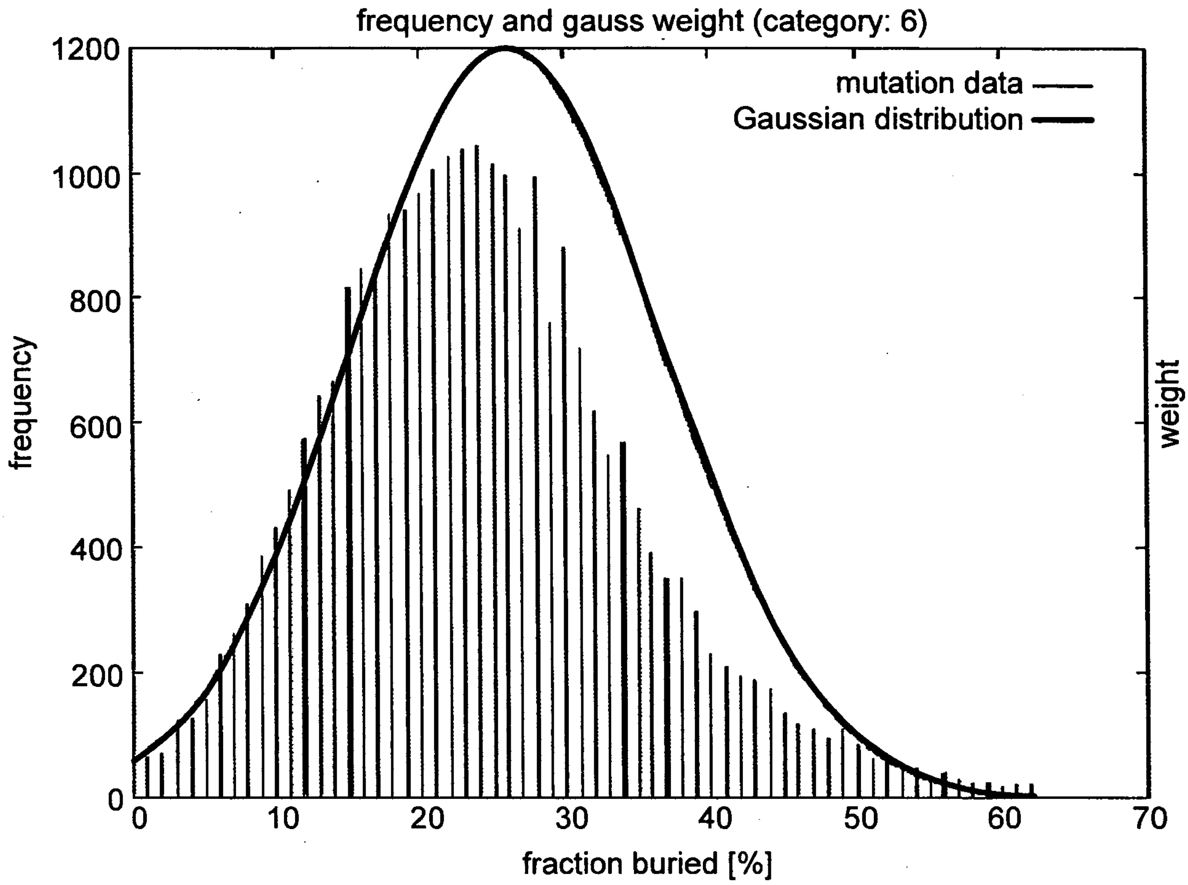
frequency and gauss weight (category: 6)
mutation data
Gaussian distribution
frequency
weight
fraction buried [%]
0
200
400
600
800
1000
1200
0
10
20
30
40
50
60
70

FIG.13

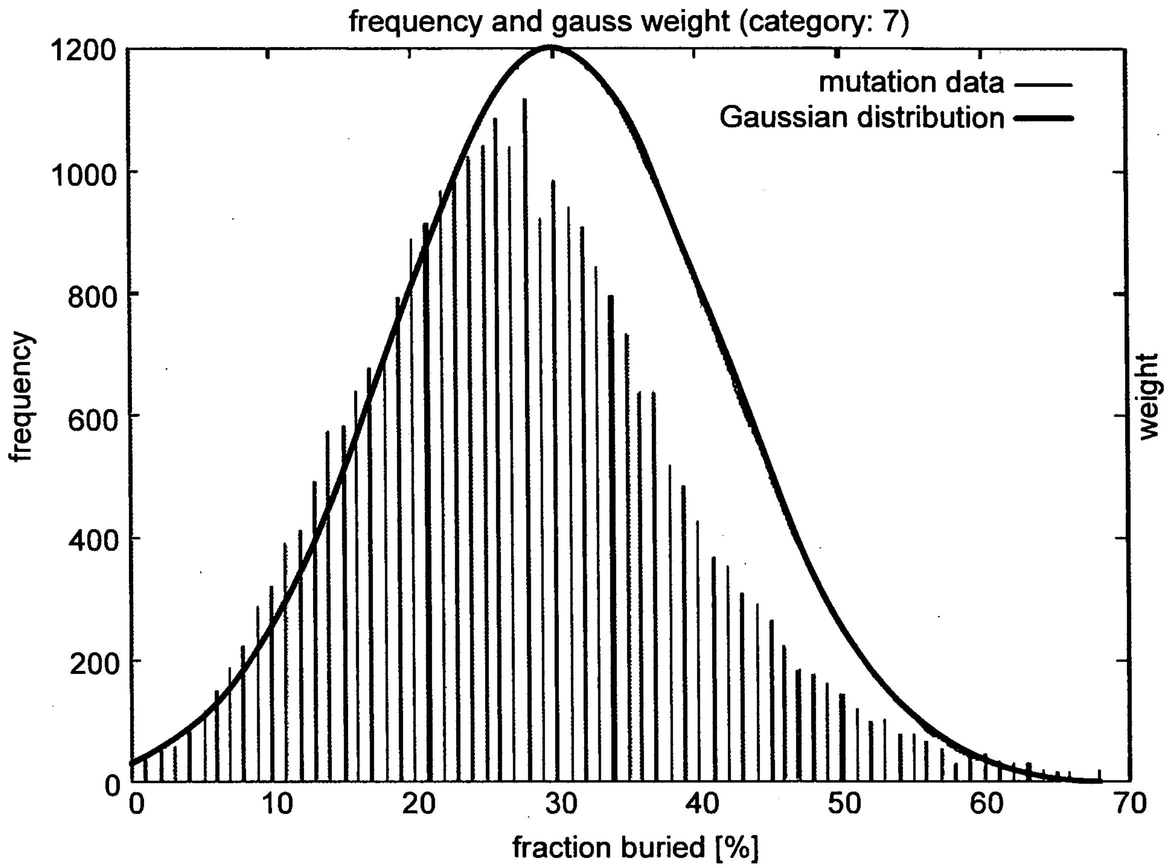
frequency and gauss weight (category: 7)
mutation data
Gaussian distribution
frequency
weight
fraction buried [%]
0
200
400
600
800
1000
1200
0
10
20
30
40
50
60
70

FIG.14

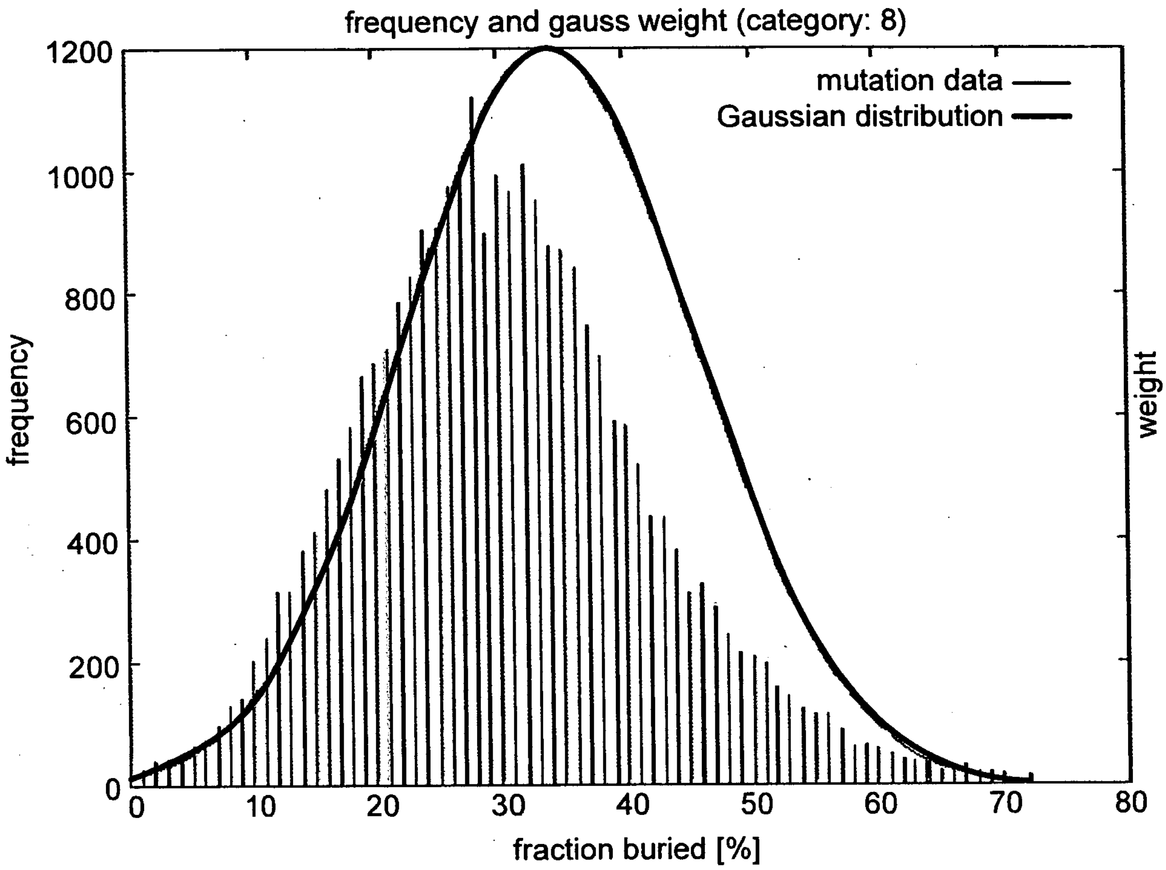
frequency and gauss weight (category: 8)
mutation data
Gaussian distribution
frequency
weight
fraction buried [%]
0
200
400
600
800
1000
1200
0
10
20
30
40
50
60
70
80

FIG.15

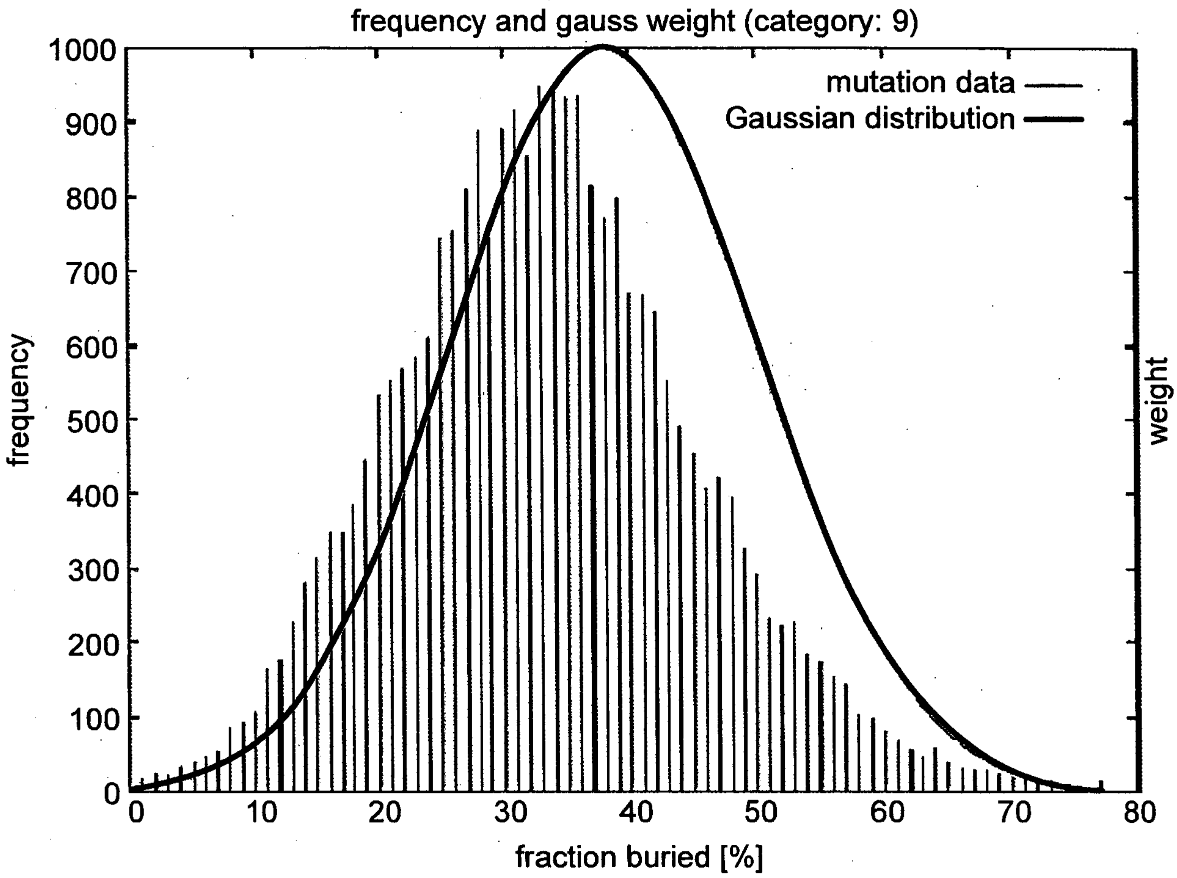
frequency and gauss weight (category: 9)
mutation data
Gaussian distribution
frequency
weight
fraction buried [%]
1000
900
800
700
600
500
400
300
200
100
0
0
10
20
30
40
50
60
70
80

FIG.16

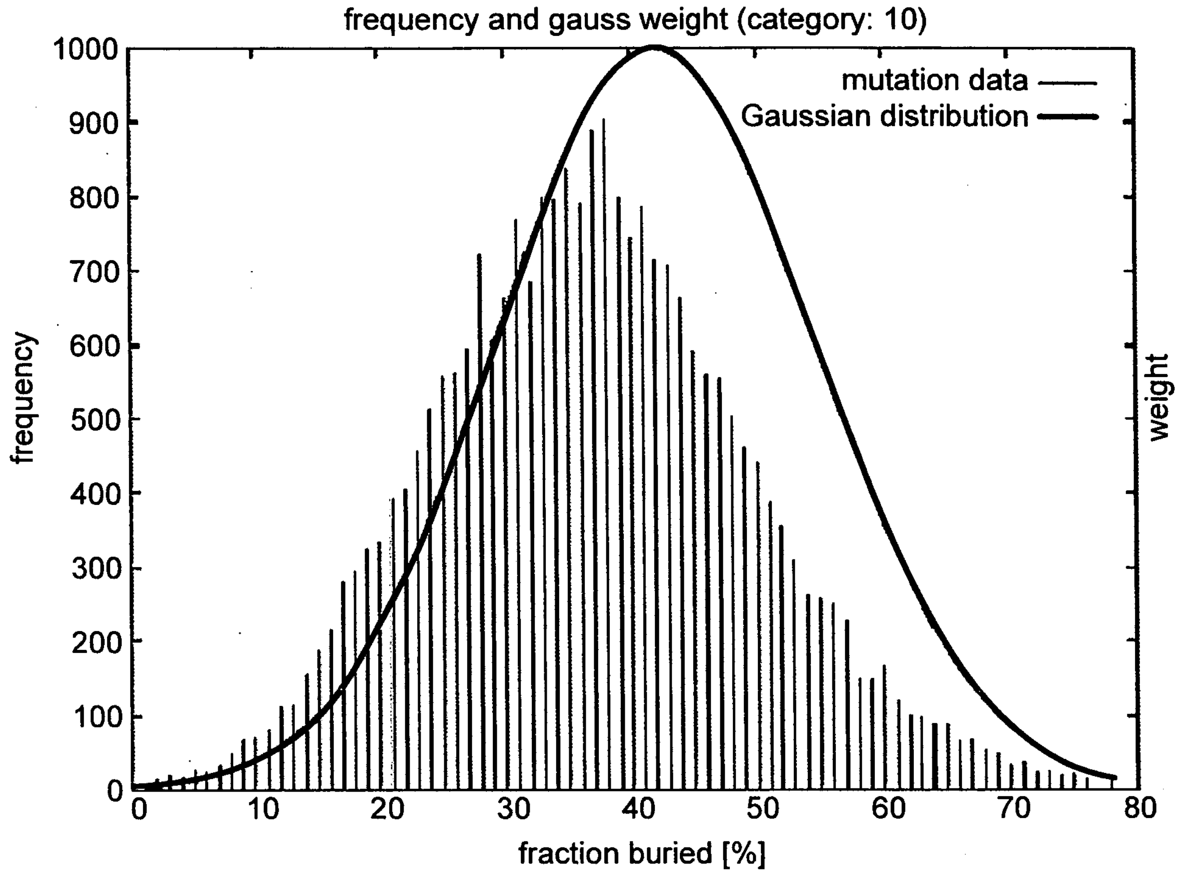
frequency and gauss weight (category: 10)
mutation data
Gaussian distribution
frequency
weight
fraction buried [%]
0
100
200
300
400
500
600
700
800
900
1000
0
10
20
30
40
50
60
70
80

FIG.17

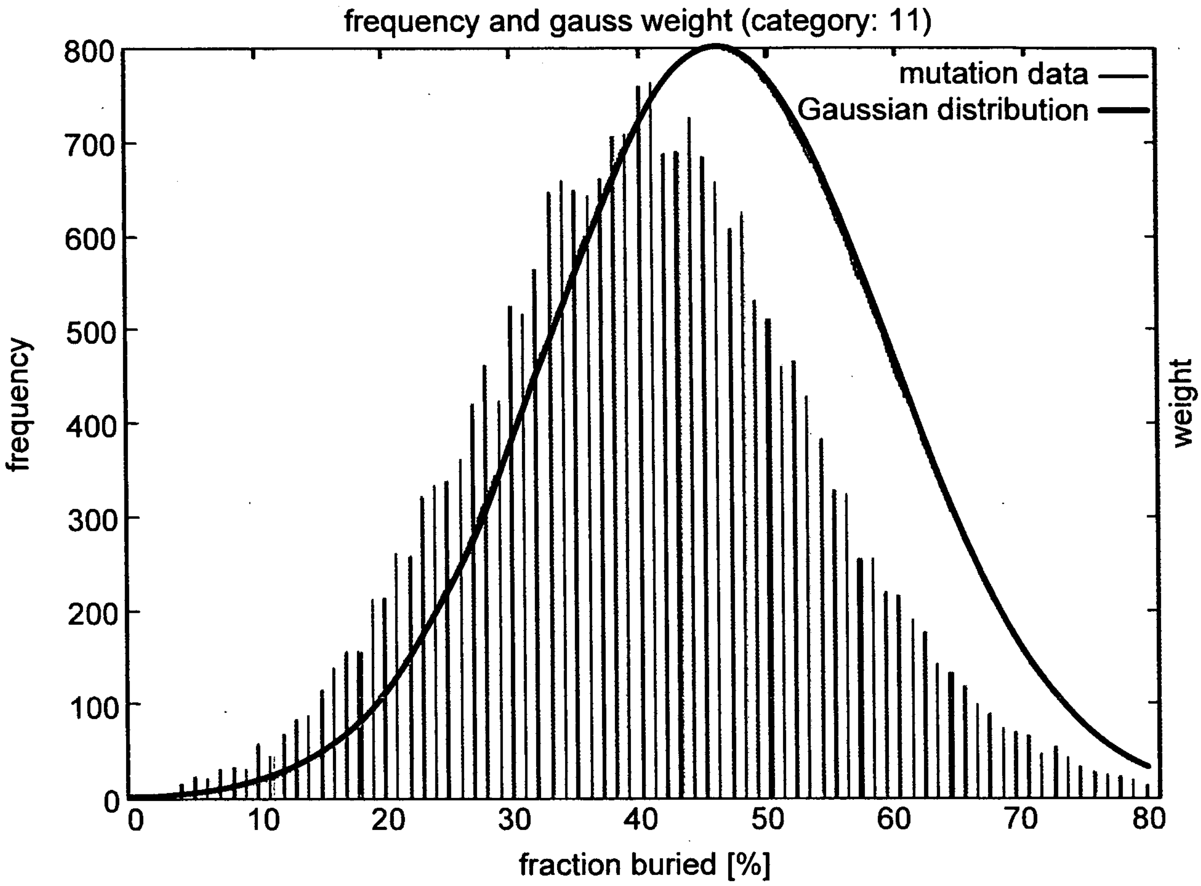
frequency and gauss weight (category: 11)
frequency
weight
fraction buried [%]
mutation data
Gaussian distribution
0
100
200
300
400
500
600
700
800
0
10
20
30
40
50
60
70
80

FIG.18

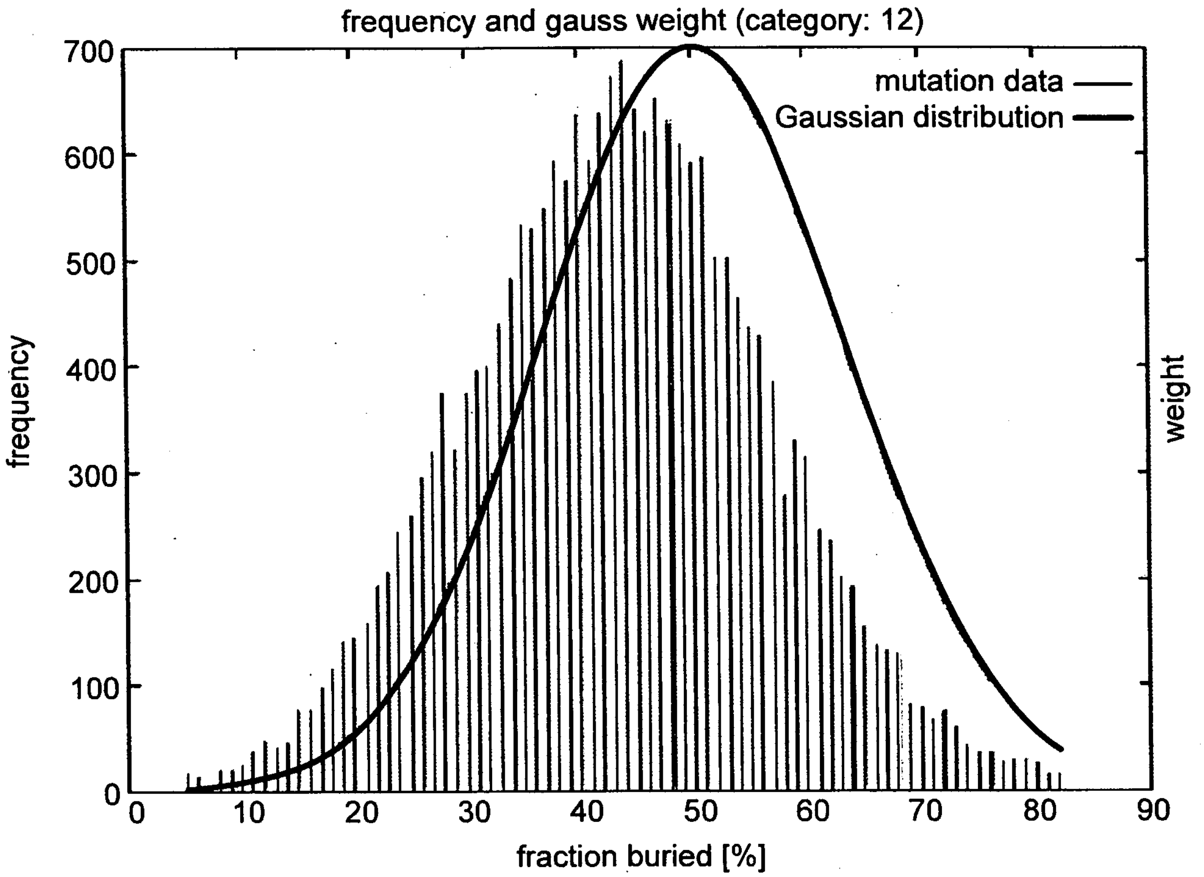
frequency and gauss weight (category: 12)
mutation data
Gaussian distribution
frequency
weight
fraction buried [%]
0
100
200
300
400
500
600
700
0
10
20
30
40
50
60
70
80
90

FIG.19

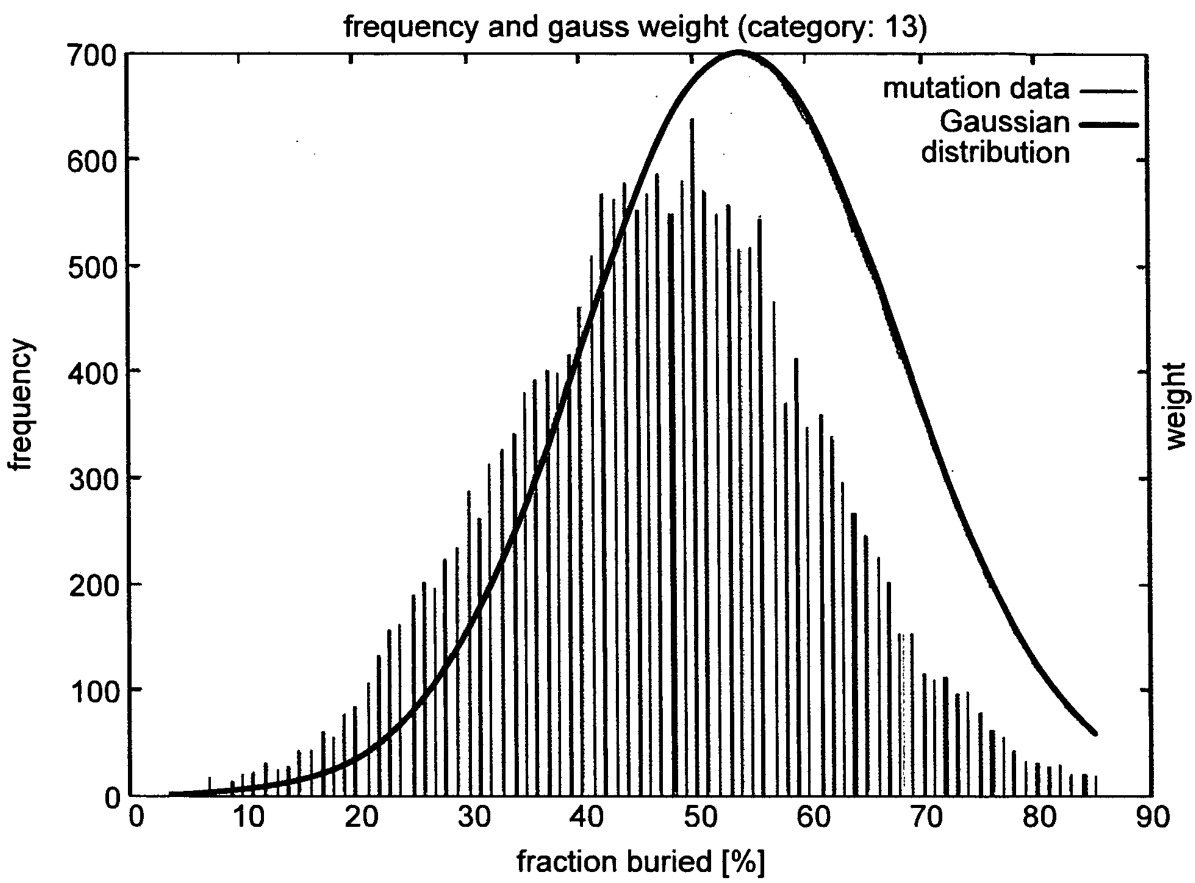
frequency and gauss weight (category: 13)
700
600
500
400
300
200
100
0
frequency
0
10
20
30
40
50
60
70
80
90
fraction buried [%]
weight
mutation data
Gaussian distribution

# FIG.20

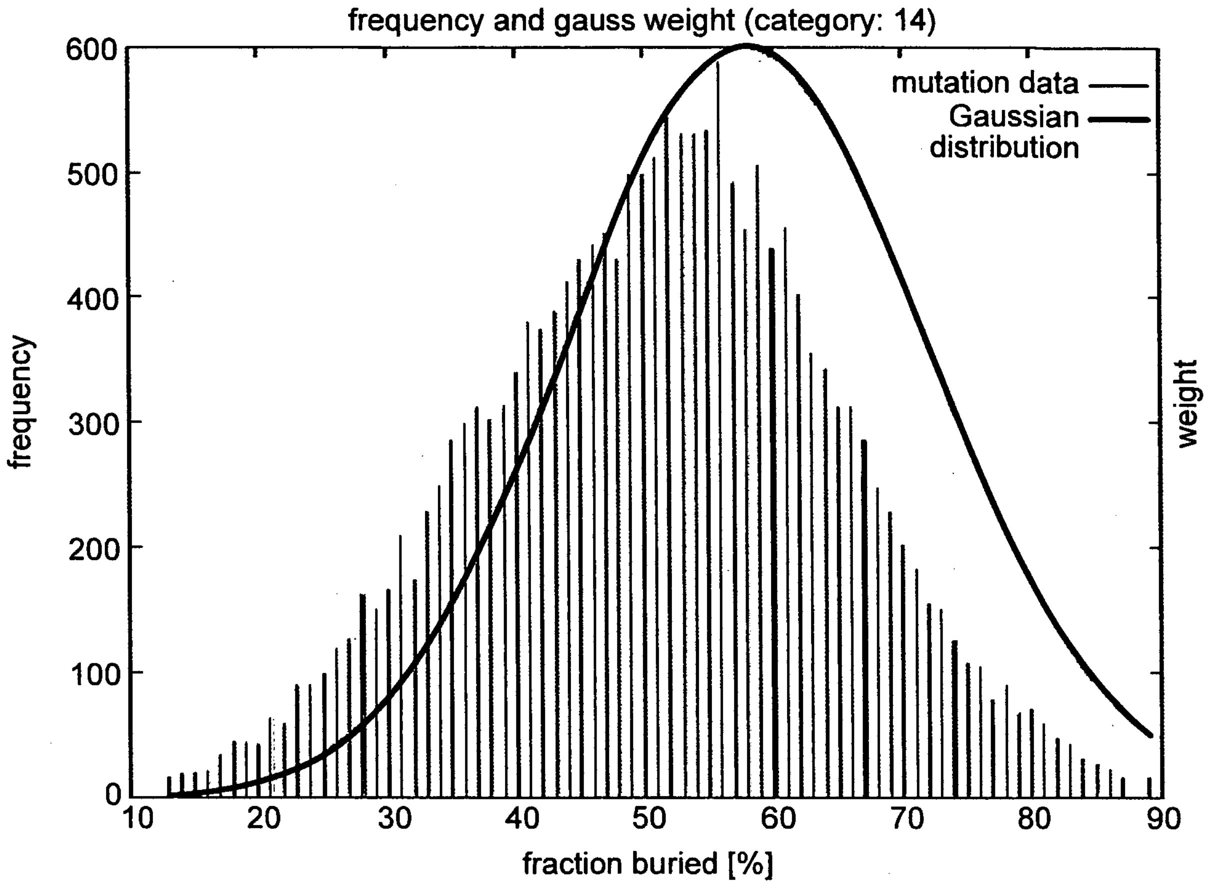
frequency and gauss weight (category: 14)
mutation data
Gaussian
distribution
frequency
weight
600
500
400
300
200
100
0
10
20
30
40
50
60
70
80
90
fraction buried [%]

FIG.21

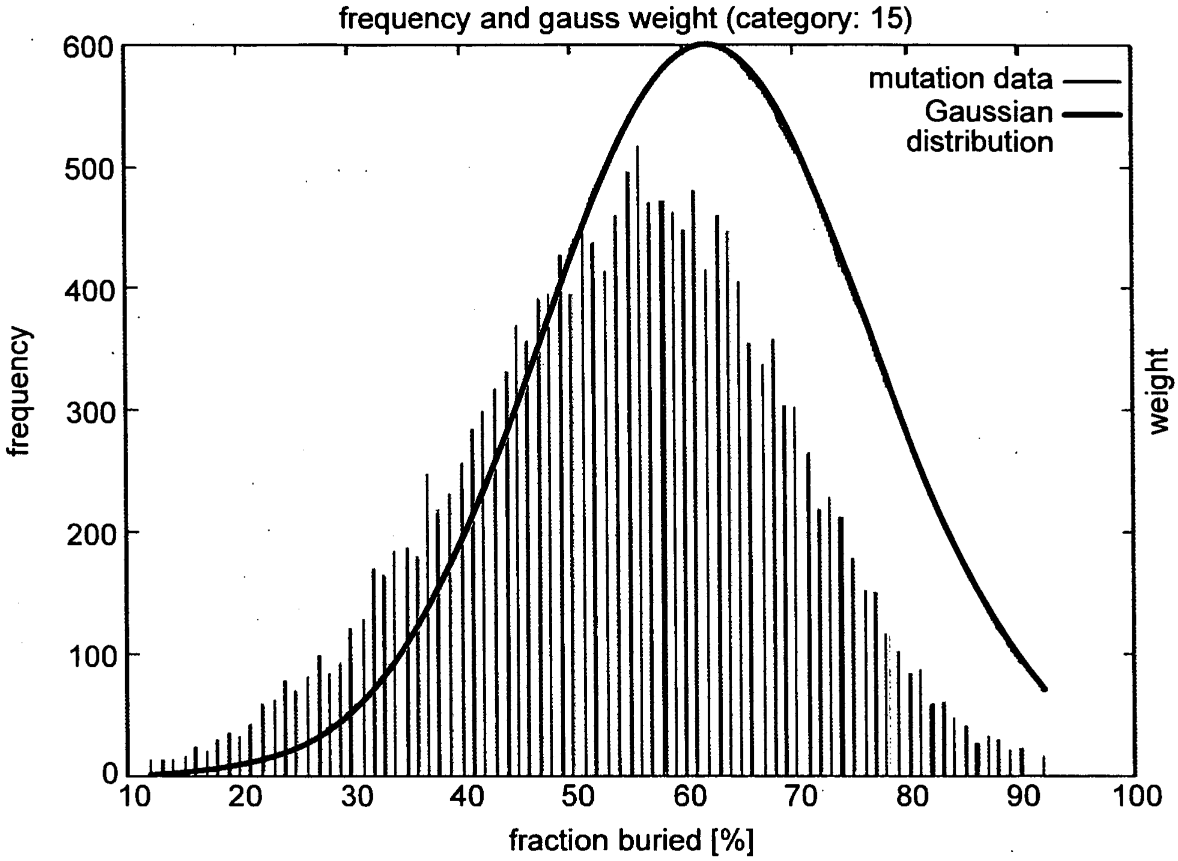
frequency and gauss weight (category: 15)
600
500
400
300
200
100
0
10
20
30
40
50
60
70
80
90
100
frequency
weight
fraction buried [%]
mutation data
Gaussian distribution

FIG.22

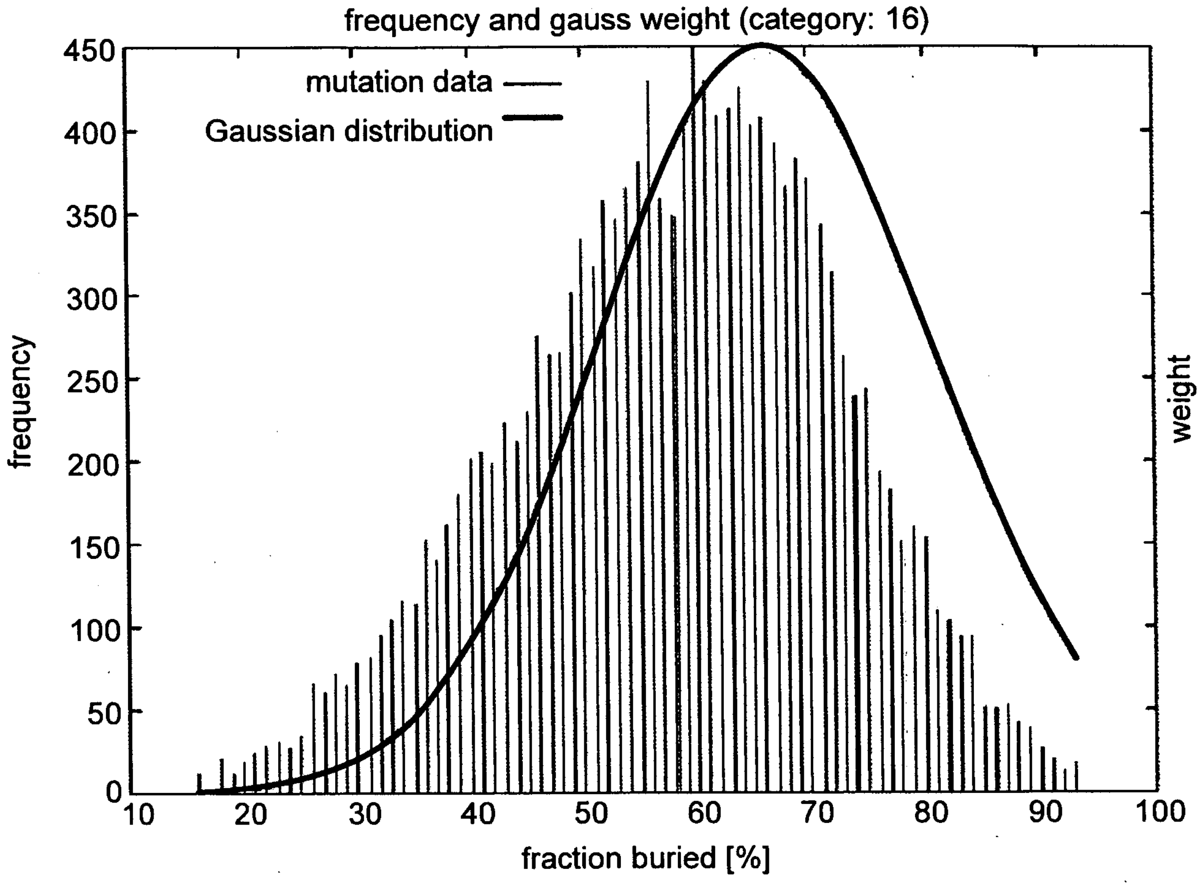
frequency and gauss weight (category: 16)
mutation data
Gaussian distribution
frequency
weight
fraction buried [%]
450
400
350
300
250
200
150
100
50
0
10
20
30
40
50
60
70
80
90
100

FIG.23

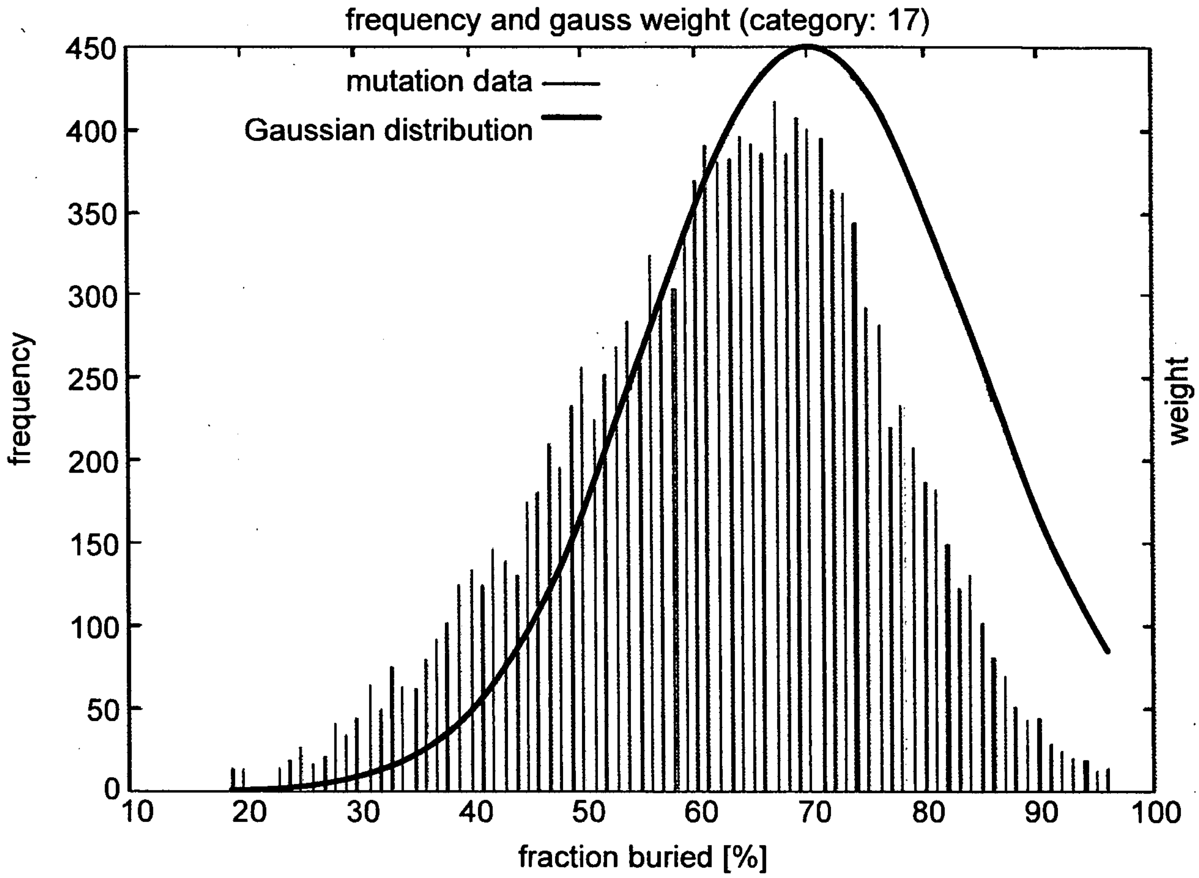
frequency and gauss weight (category: 17)
mutation data
Gaussian distribution
frequency
weight
fraction buried [%]
450
400
350
300
250
200
150
100
50
0
10
20
30
40
50
60
70
80
90
100

FIG.24

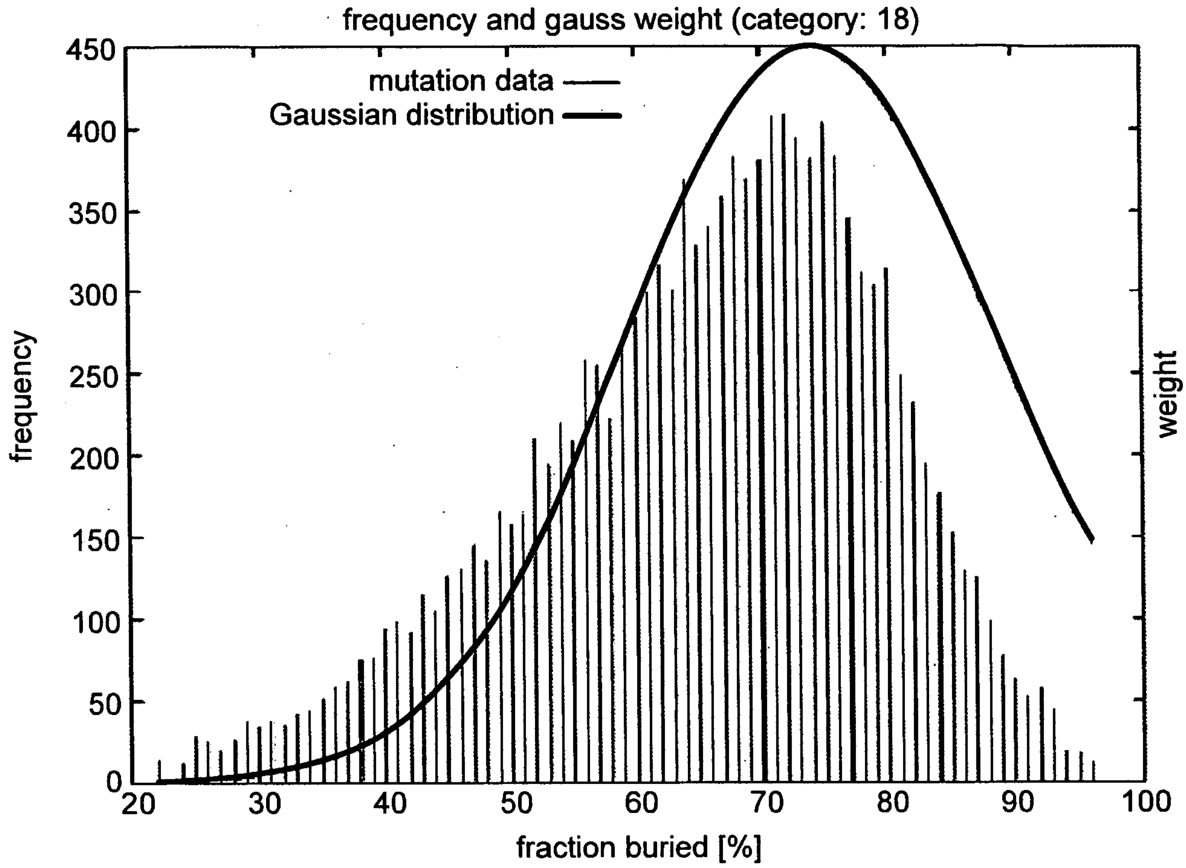
frequency and gauss weight (category: 18)
mutation data
Gaussian distribution
frequency
weight
fraction buried [%]
450
400
350
300
250
200
150
100
50
0
20
30
40
50
60
70
80
90
100

FIG.25

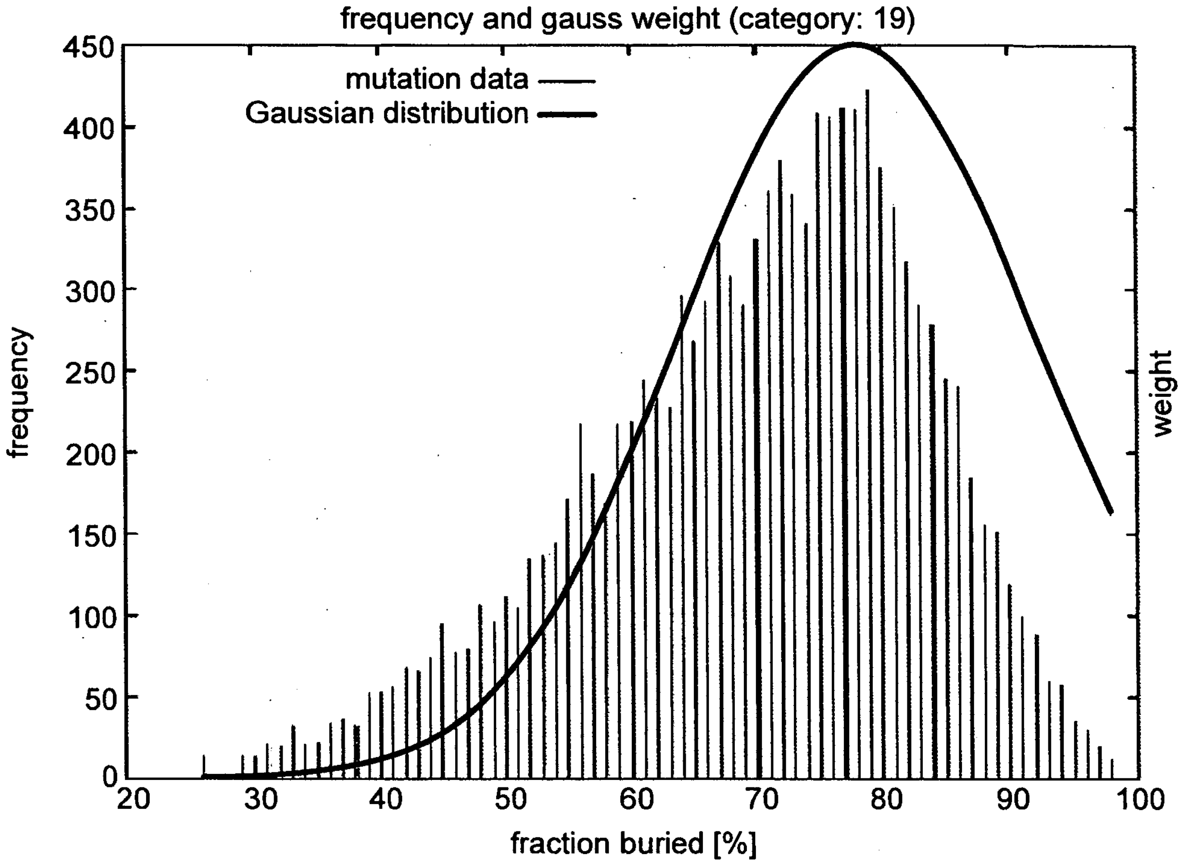
frequency and gauss weight (category: 19)
mutation data
Gaussian distribution
frequency
weight
fraction buried [%]
450
400
350
300
250
200
150
100
50
0
20
30
40
50
60
70
80
90
100

FIG.26

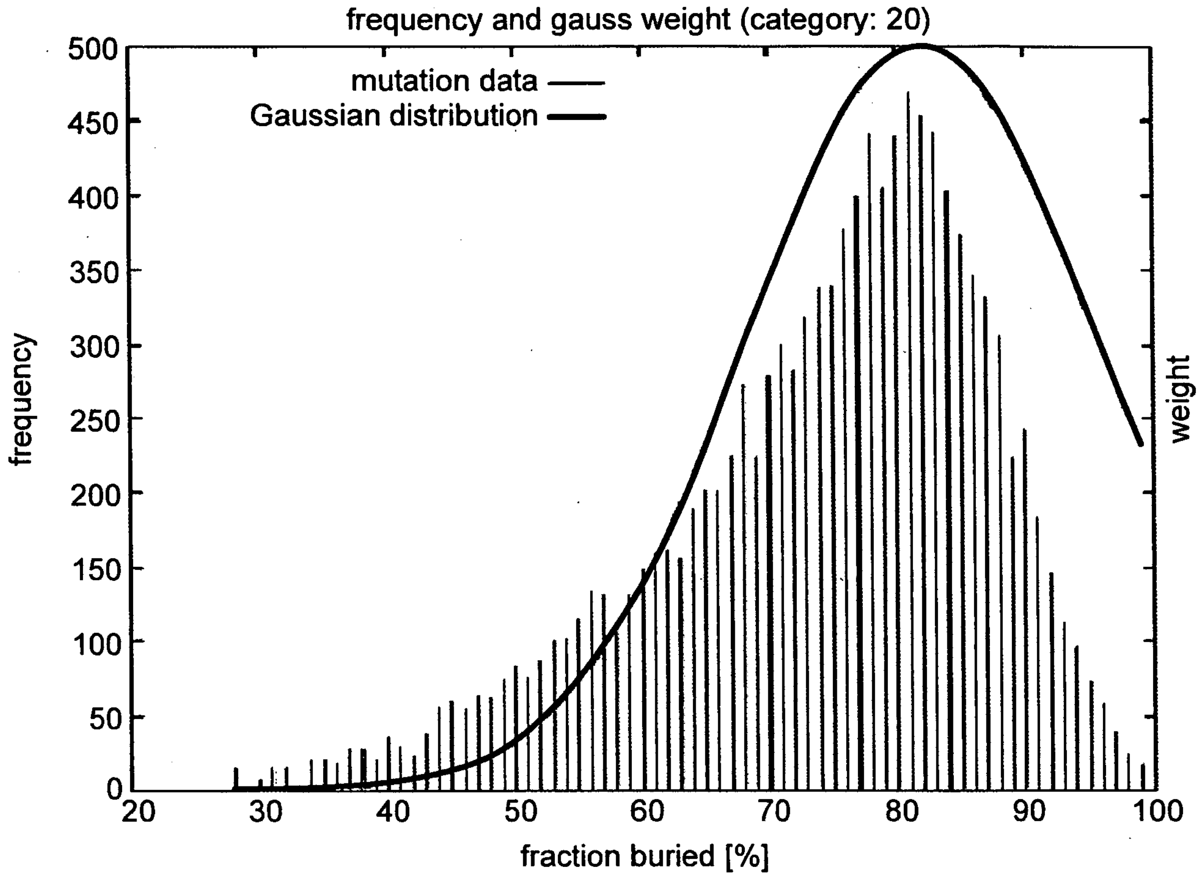
frequency and gauss weight (category: 20)
mutation data
Gaussian distribution
frequency
weight
fraction buried [%]
0
50
100
150
200
250
300
350
400
450
500
20
30
40
50
60
70
80
90
100

FIG.27

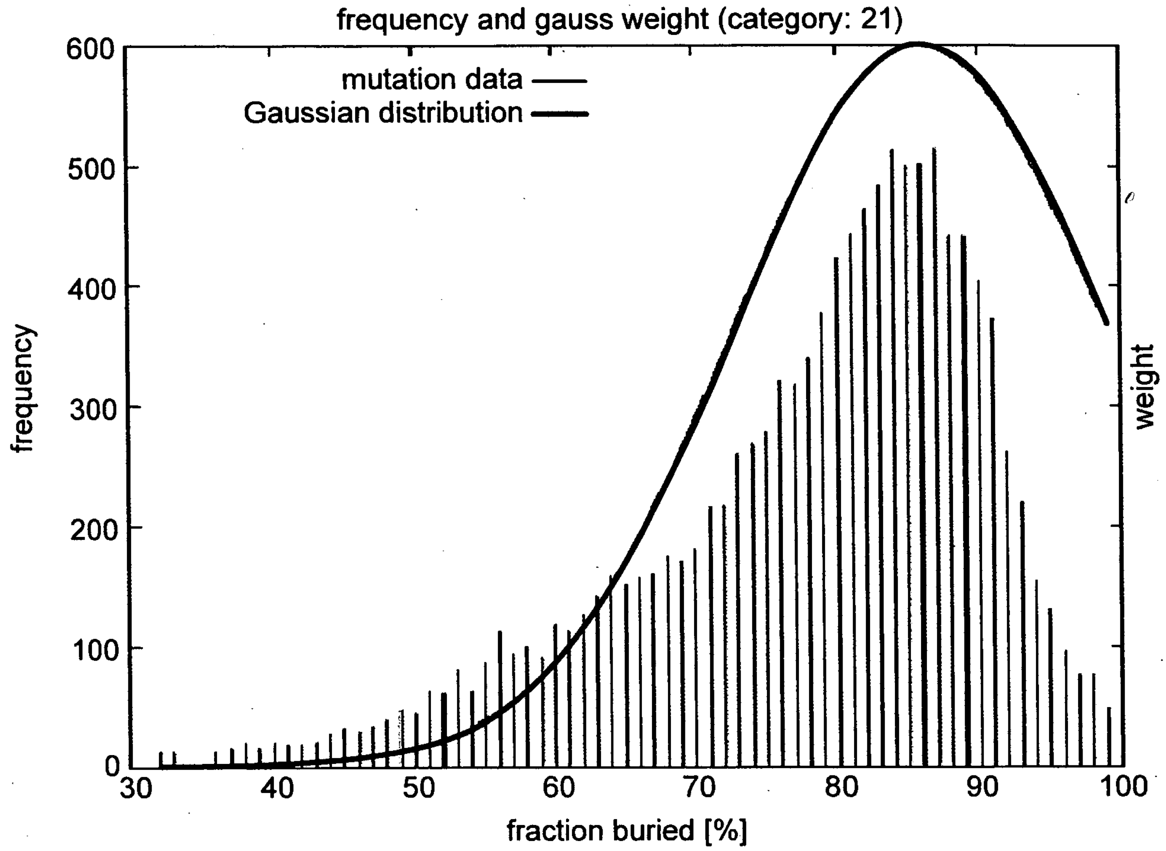
frequency and gauss weight (category: 21)
mutation data
Gaussian distribution
frequency
weight
fraction buried [%]
600
500
400
300
200
100
0
30
40
50
60
70
80
90
100

FIG.28

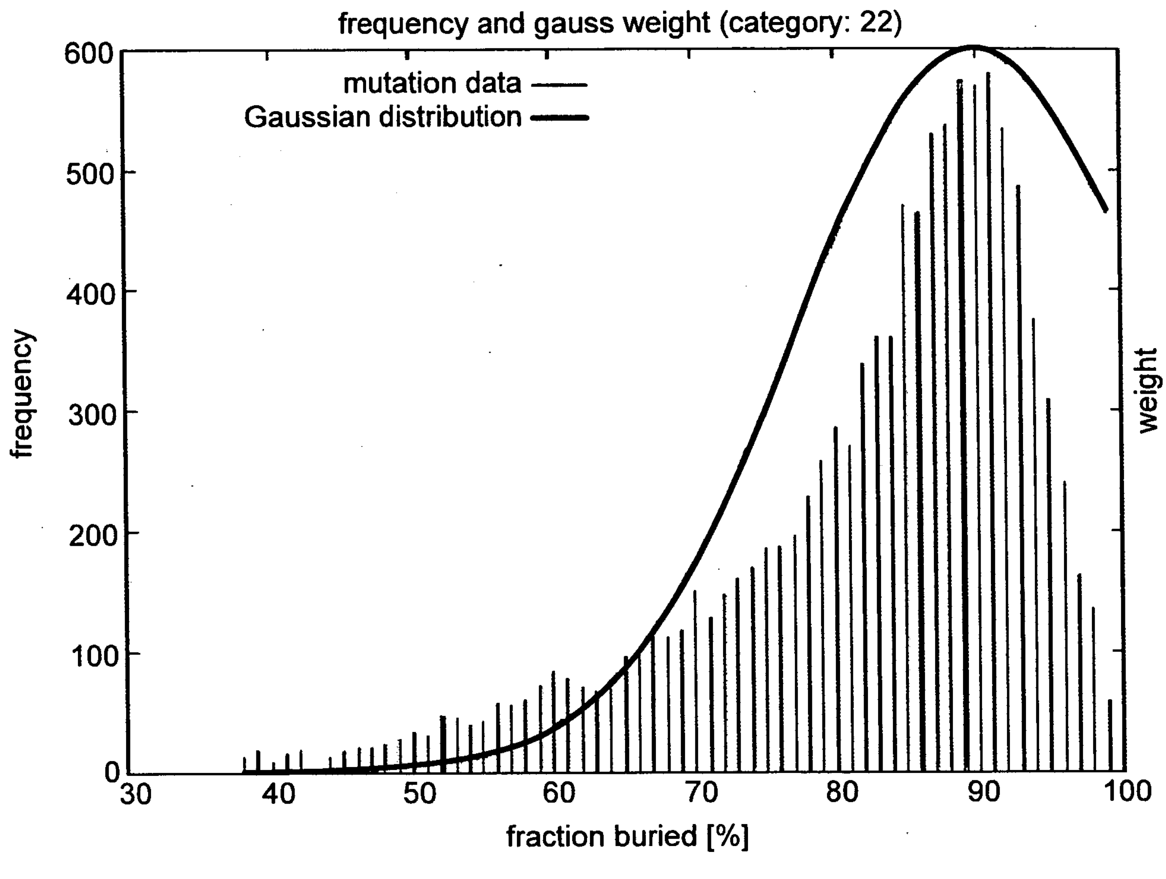
frequency and gauss weight (category: 22)
mutation data
Gaussian distribution
frequency
weight
fraction buried [%]
0
100
200
300
400
500
600
30
40
50
60
70
80
90
100

FIG.29

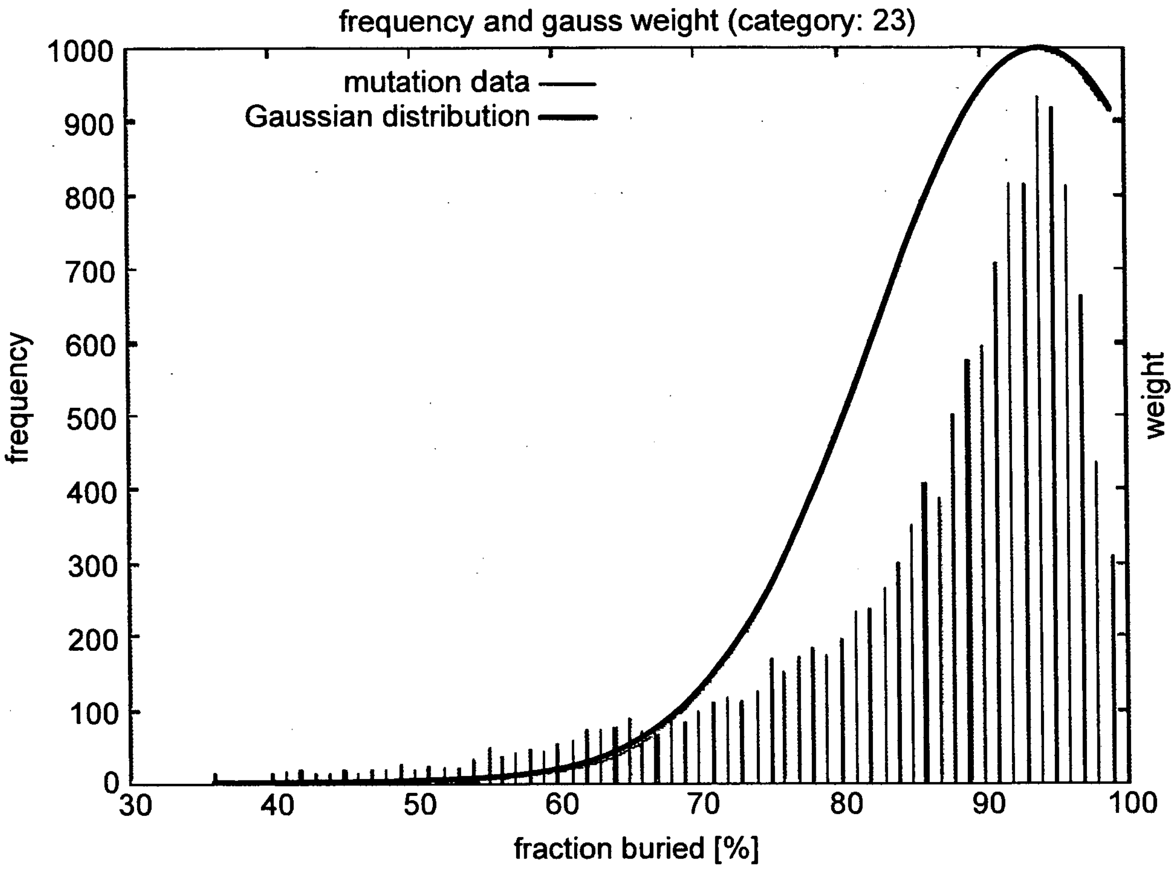
frequency and gauss weight (category: 23)
mutation data
Gaussian distribution
frequency
weight
fraction buried [%]
1000
900
800
700
600
500
400
300
200
100
0
30
40
50
60
70
80
90
100

FIG.30

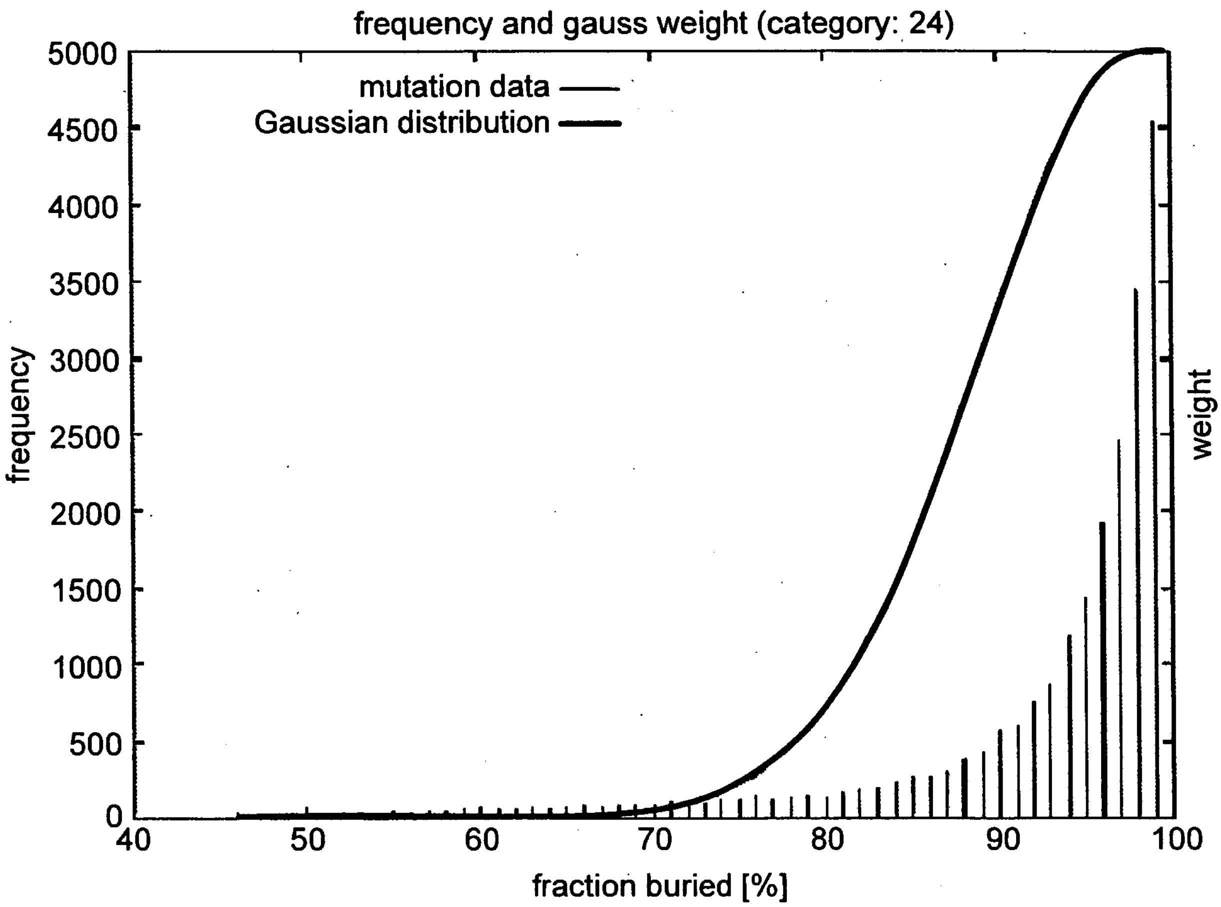
frequency and gauss weight (category: 24)
mutation data
Gaussian distribution
frequency
weight
fraction buried [%]
5000
4500
4000
3500
3000
2500
2000
1500
1000
500
0
40
50
60
70
80
90
100

FIG.31

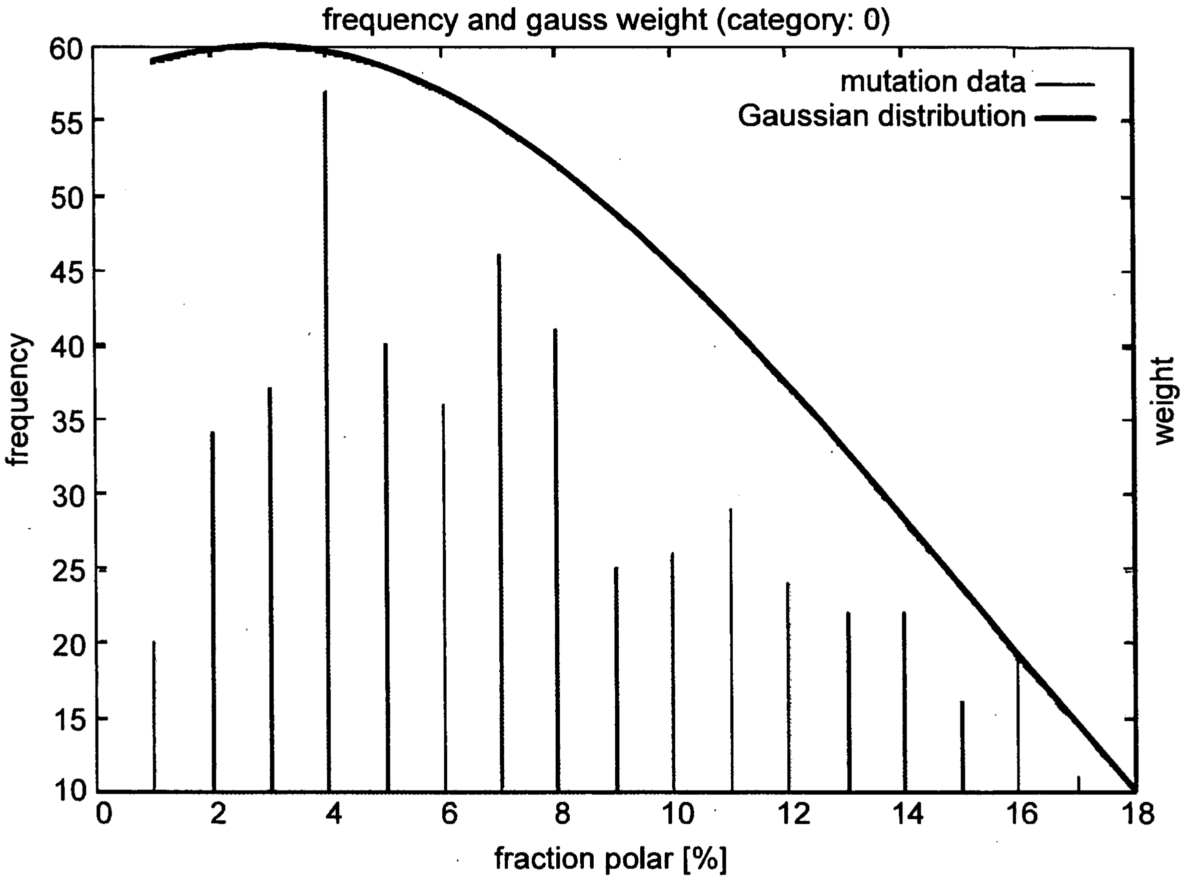
frequency and gauss weight (category: 0)
mutation data
Gaussian distribution
frequency
weight
fraction polar [%]
60
55
50
45
40
35
30
25
20
15
10
0
2
4
6
8
10
12
14
16
18

FIG.32

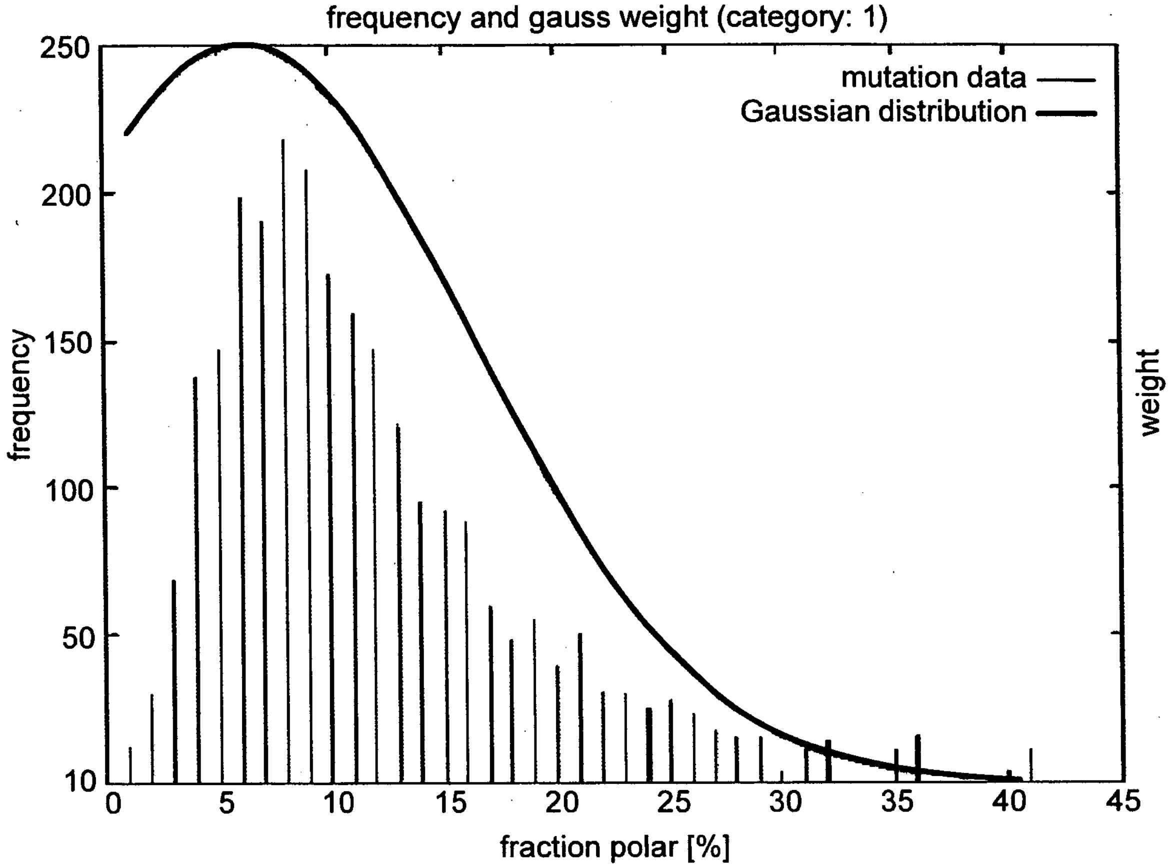
frequency and gauss weight (category: 1)
mutation data
Gaussian distribution
frequency
weight
fraction polar [%]
250
200
150
100
50
10
0
5
10
15
20
25
30
35
40
45

FIG.33

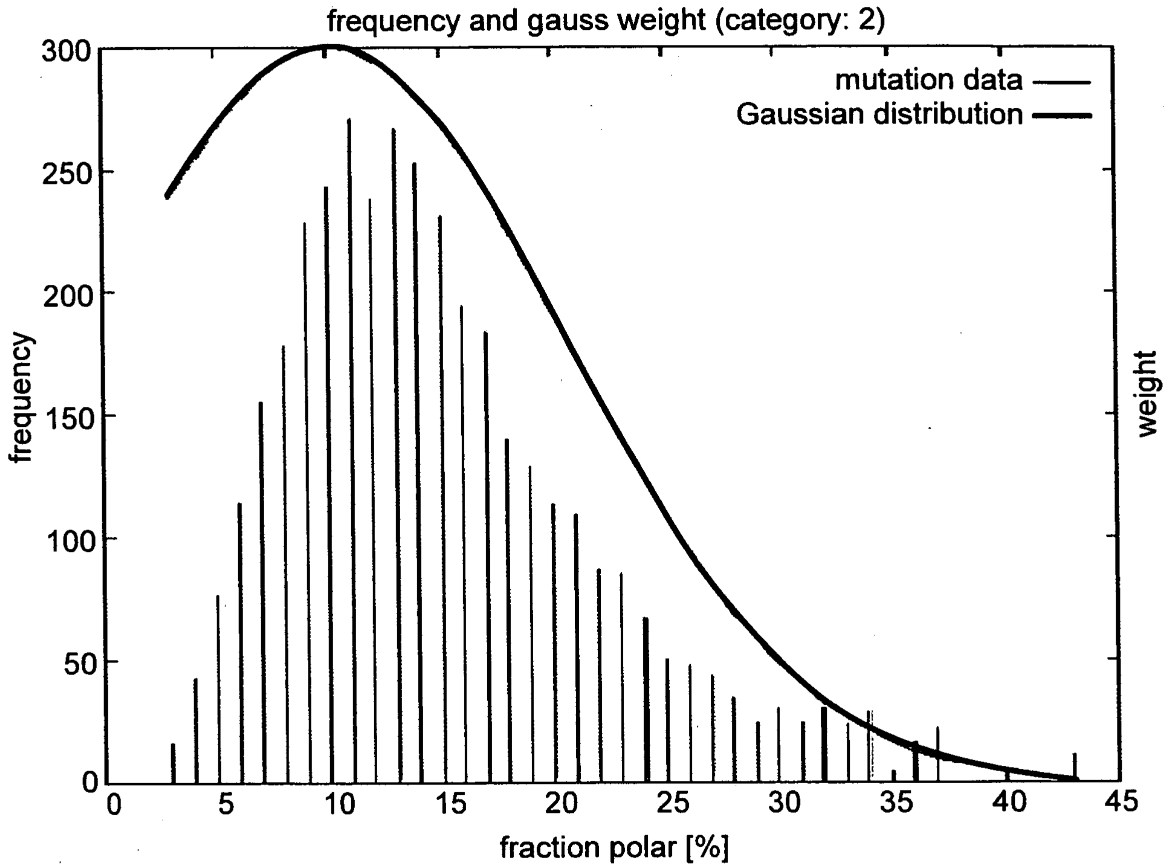
frequency and gauss weight (category: 2)
mutation data
Gaussian distribution
frequency
weight
fraction polar [%]
300
250
200
150
100
50
0
0
5
10
15
20
25
30
35
40
45

FIG.34

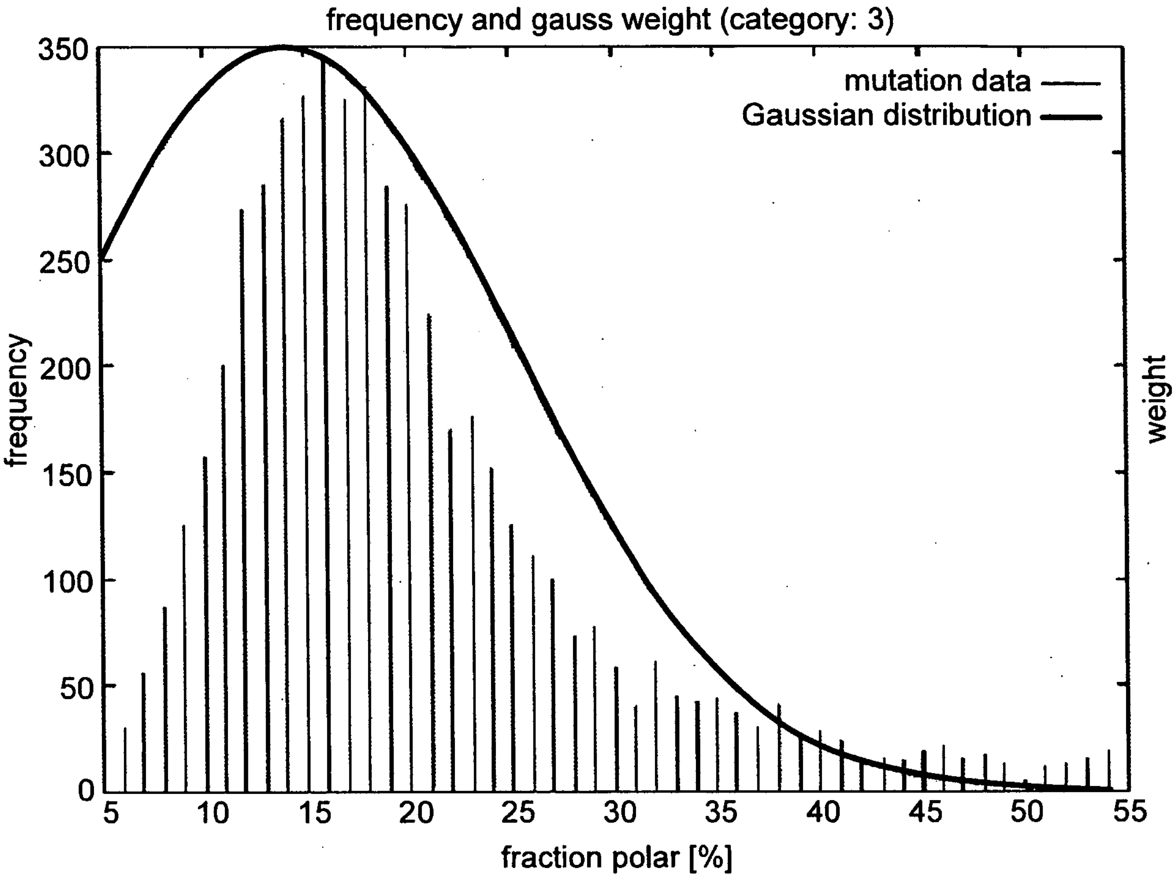
frequency and gauss weight (category: 3)
mutation data
Gaussian distribution
frequency
weight
fraction polar [%]
0
50
100
150
200
250
300
350
5
10
15
20
25
30
35
40
45
50
55

FIG.35

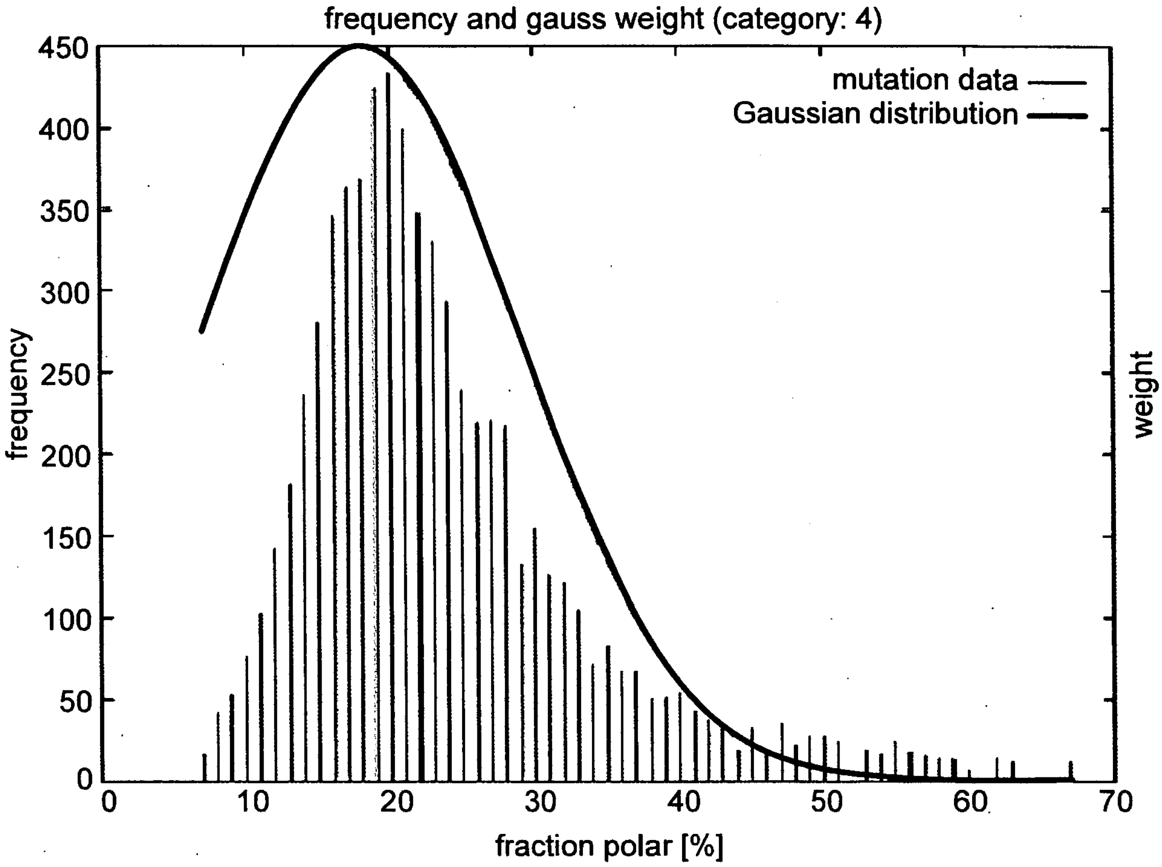
frequency and gauss weight (category: 4)
mutation data
Gaussian distribution
frequency
weight
fraction polar [%]
0
50
100
150
200
250
300
350
400
450
0
10
20
30
40
50
60
70

FIG.36

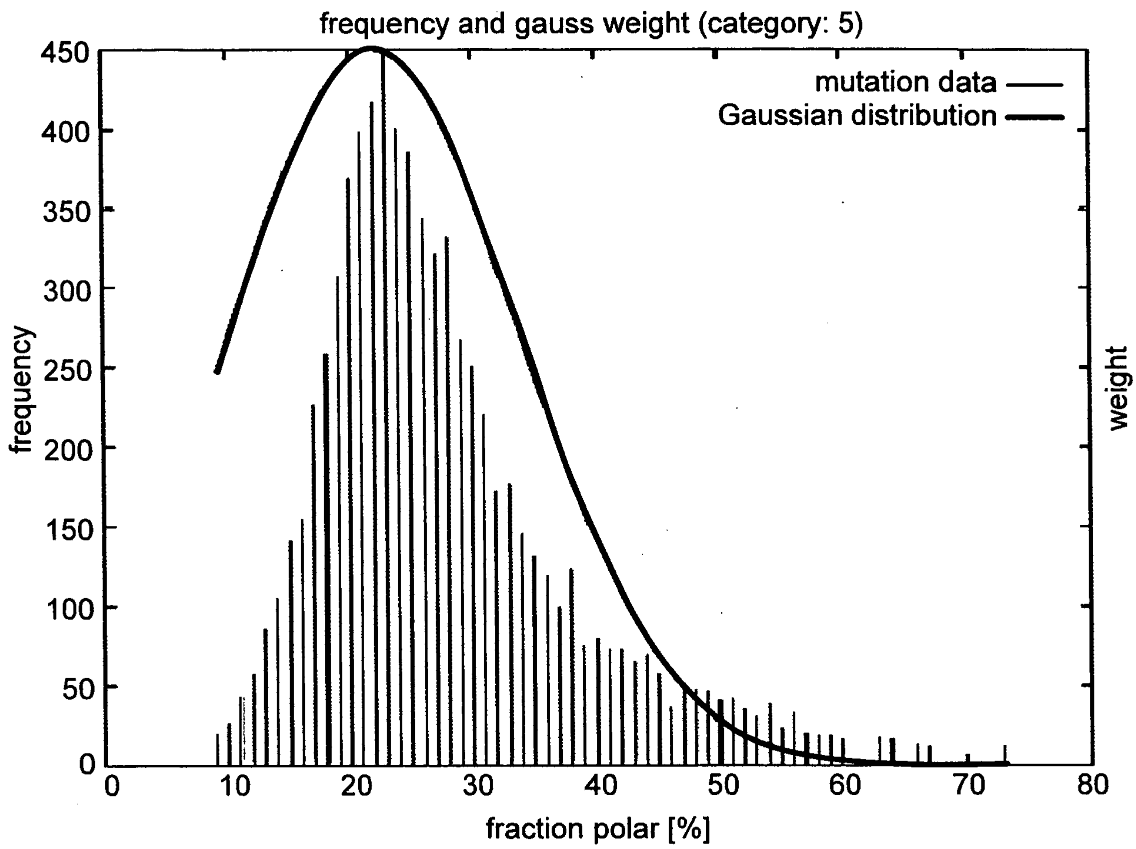
frequency and gauss weight (category: 5)
mutation data
Gaussian distribution
frequency
weight
fraction polar [%]
0
50
100
150
200
250
300
350
400
450
0
10
20
30
40
50
60
70
80

# FIG.37

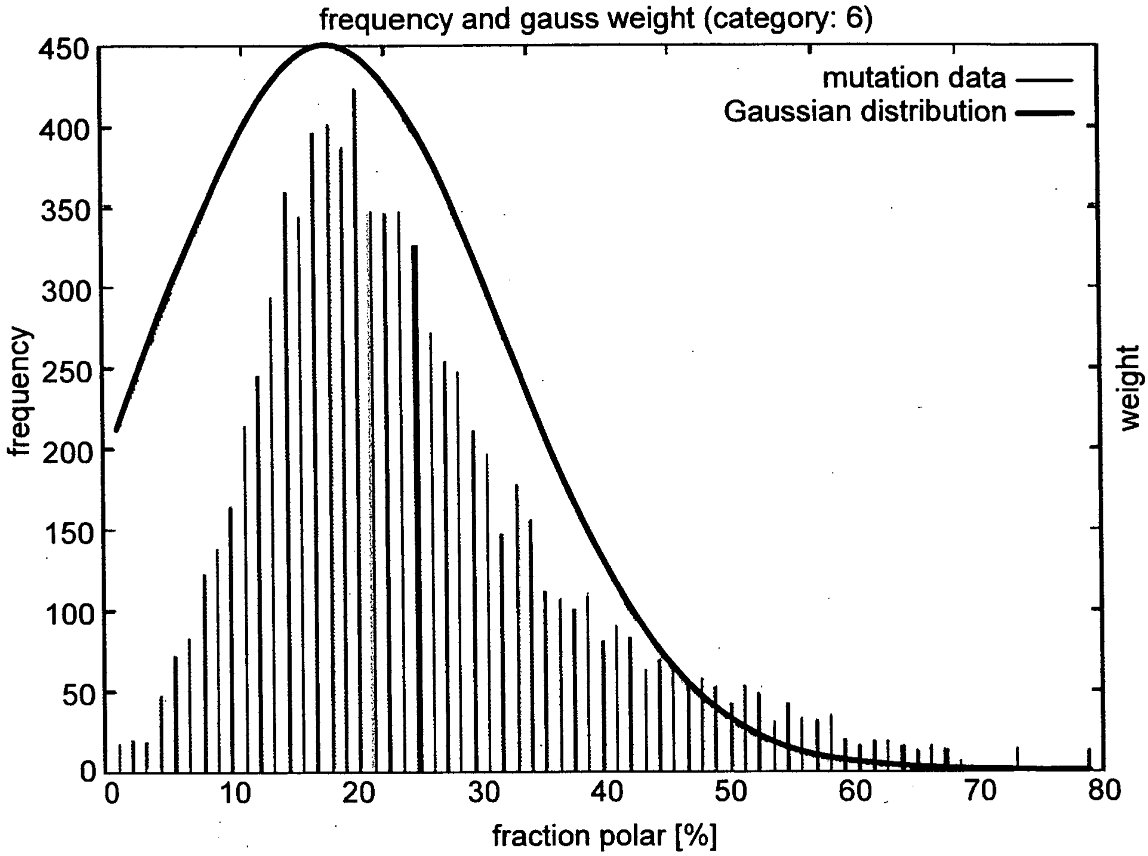
frequency and gauss weight (category: 6)
mutation data
Gaussian distribution
frequency
weight
fraction polar [%]
0
50
100
150
200
250
300
350
400
450
0
10
20
30
40
50
60
70
80

FIG.38

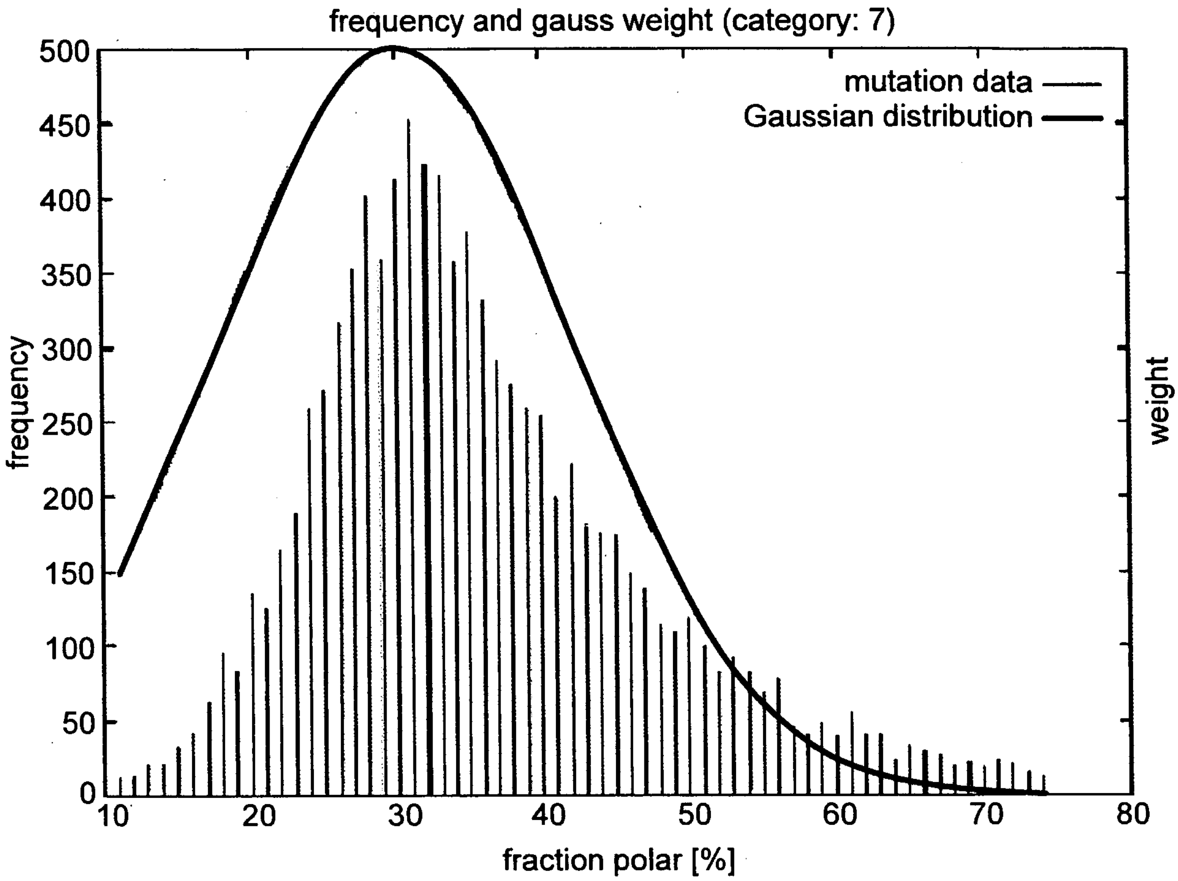
frequency and gauss weight (category: 7)
mutation data
Gaussian distribution
frequency
weight
fraction polar [%]
500
450
400
350
300
250
200
150
100
50
0
10
20
30
40
50
60
70
80

FIG.39

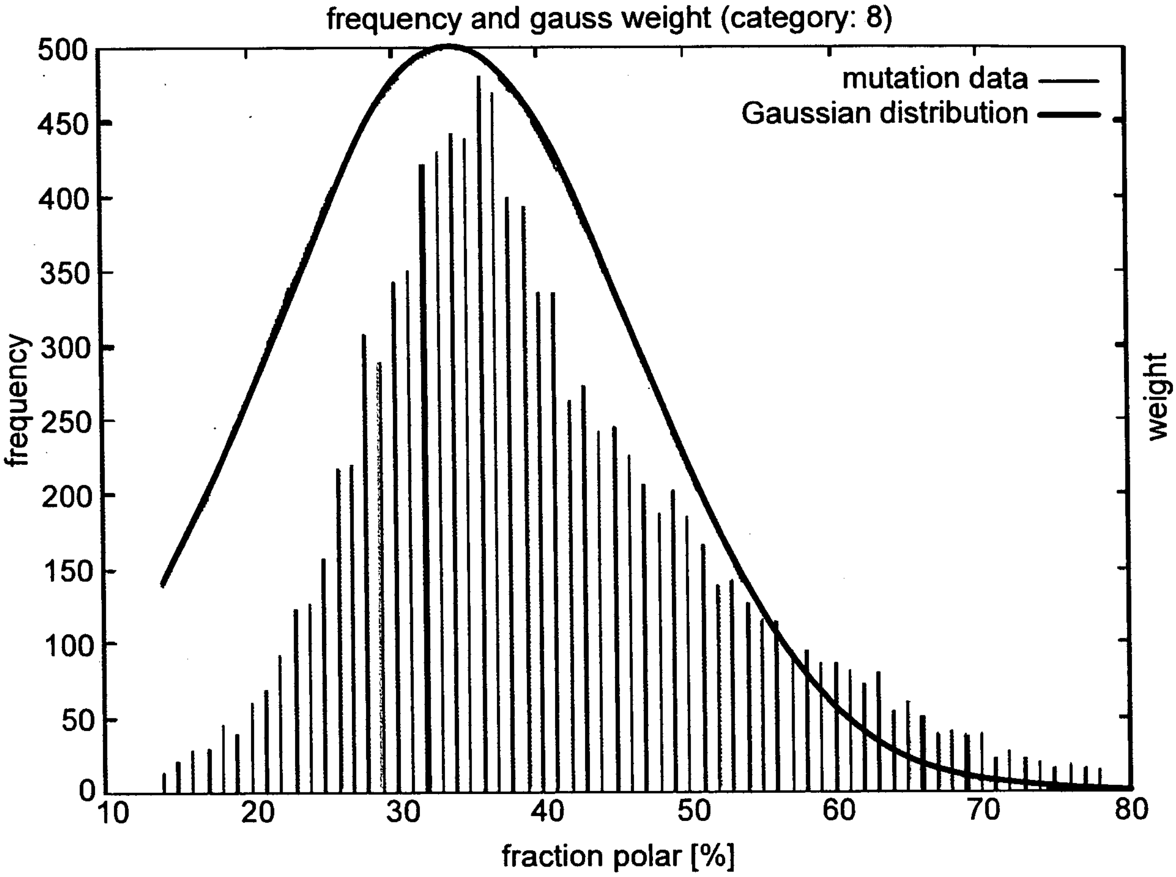
frequency and gauss weight (category: 8)
500
450
400
350
300
250
200
150
100
50
0
frequency
10
20
30
40
50
60
70
80
fraction polar [%]
weight
mutation data
Gaussian distribution

FIG.40

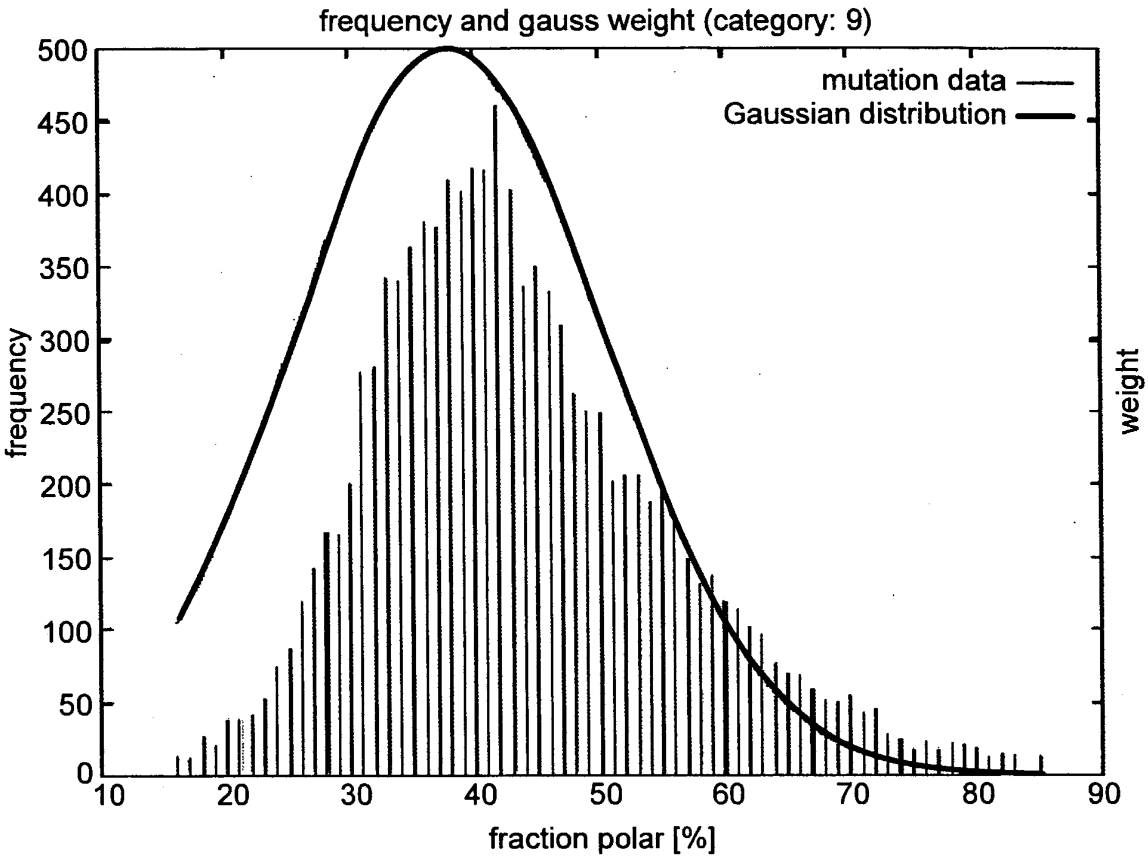
frequency and gauss weight (category: 9)
mutation data
Gaussian distribution
frequency
weight
fraction polar [%]
500
450
400
350
300
250
200
150
100
50
0
10
20
30
40
50
60
70
80
90

FIG.41

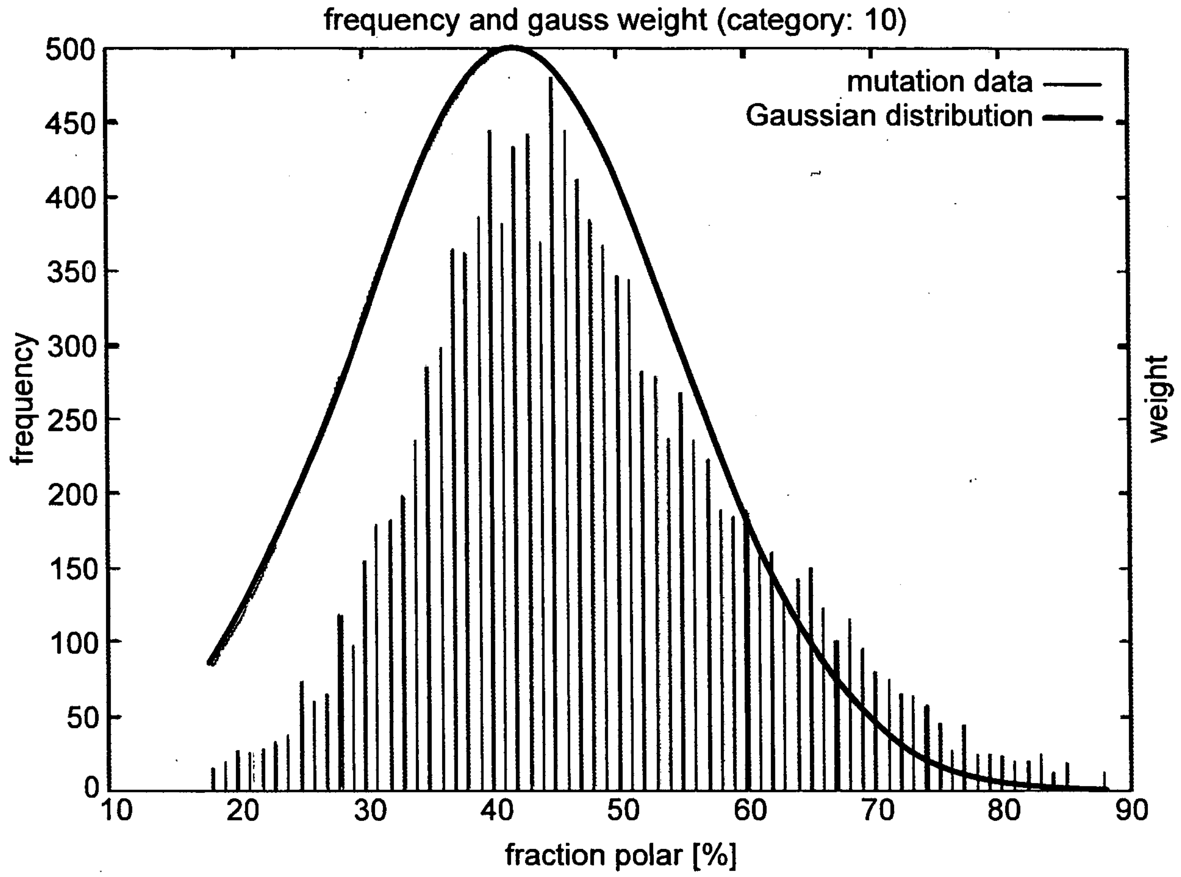
frequency and gauss weight (category: 10)
mutation data
Gaussian distribution
frequency
weight
fraction polar [%]
500
450
400
350
300
250
200
150
100
50
0
10
20
30
40
50
60
70
80
90

FIG.42

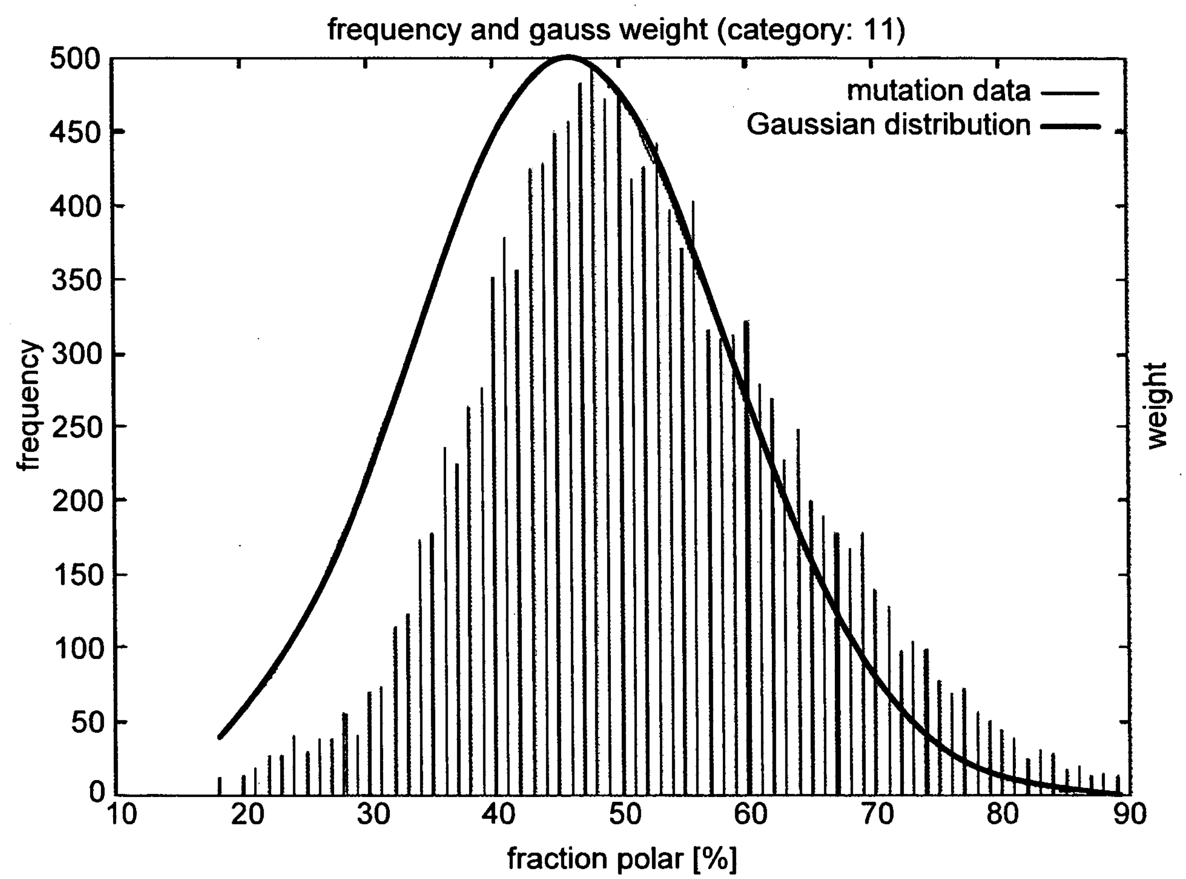
frequency and gauss weight (category: 11)
mutation data
Gaussian distribution
frequency
weight
fraction polar [%]
500
450
400
350
300
250
200
150
100
50
0
10
20
30
40
50
60
70
80
90

FIG.43

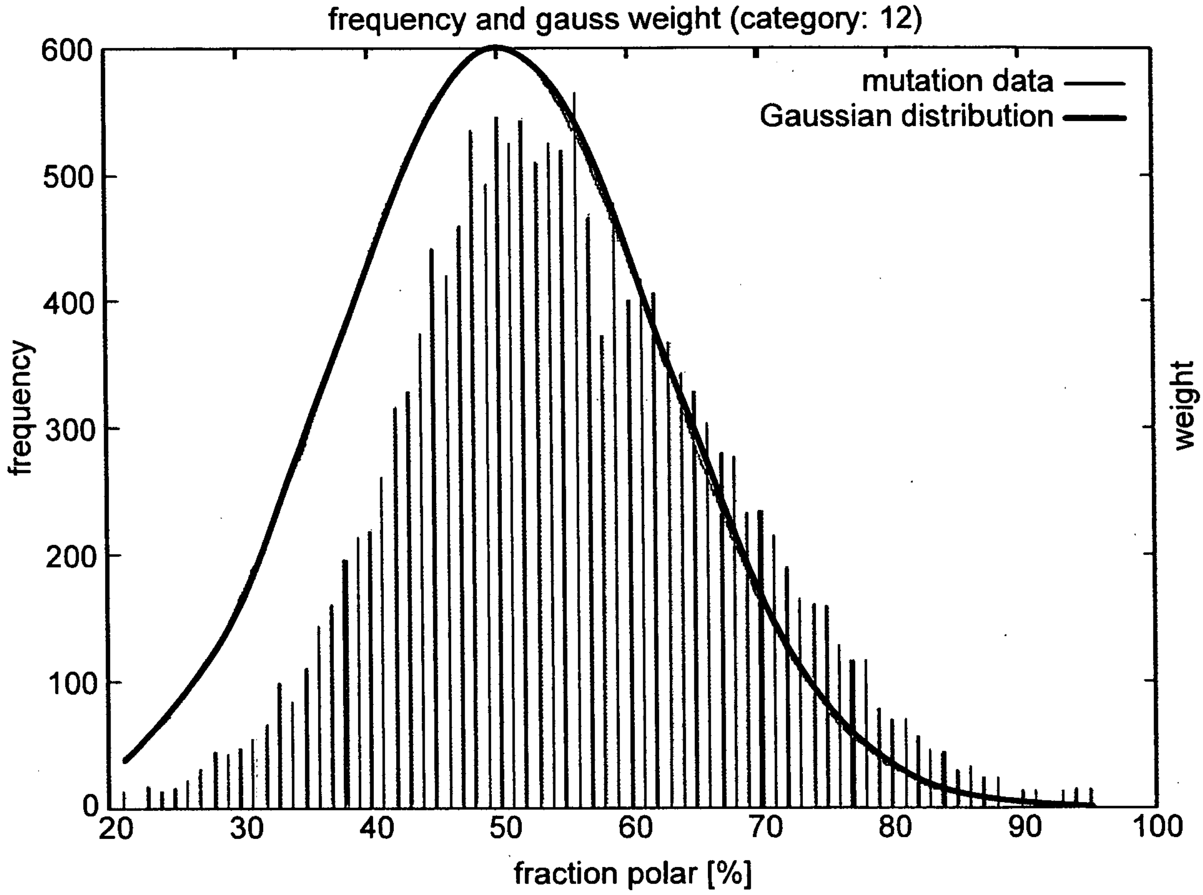
frequency and gauss weight (category: 12)
mutation data
Gaussian distribution
frequency
weight
fraction polar [%]
0
100
200
300
400
500
600
20
30
40
50
60
70
80
90
100

FIG.44

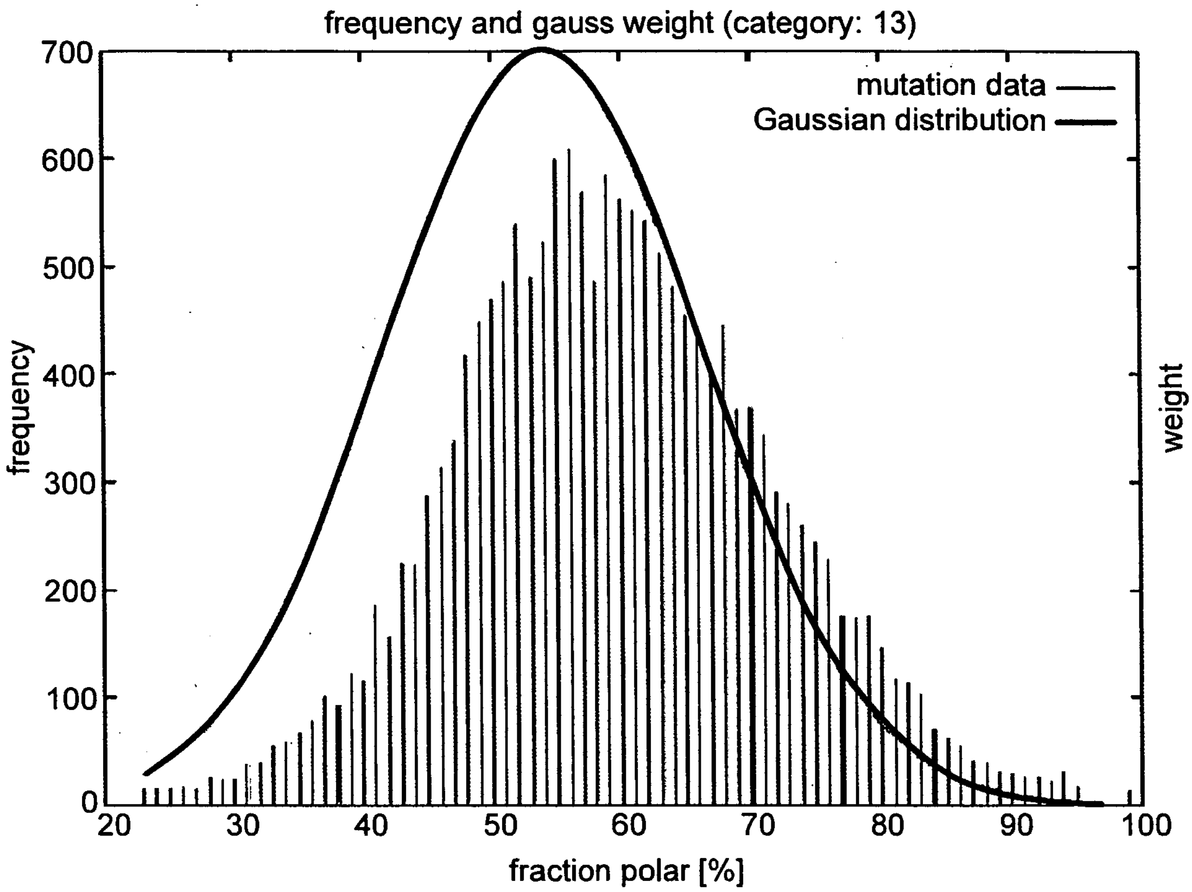
frequency and gauss weight (category: 13)
mutation data
Gaussian distribution
frequency
weight
fraction polar [%]
0
100
200
300
400
500
600
700
20
30
40
50
60
70
80
90
100

FIG.45

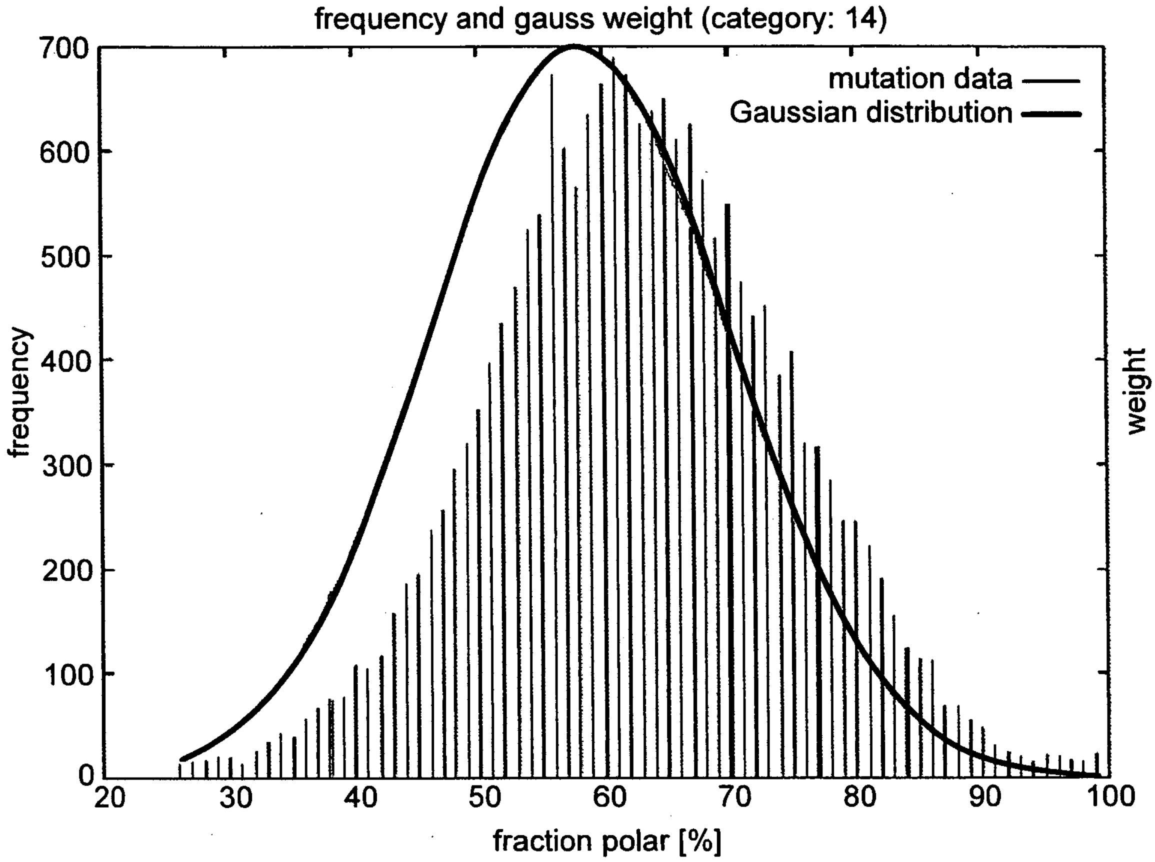
frequency and gauss weight (category: 14)
700
600
500
400
300
200
100
0
20
30
40
50
60
70
80
90
100
frequency
weight
fraction polar [%]
mutation data
Gaussian distribution

FIG.46

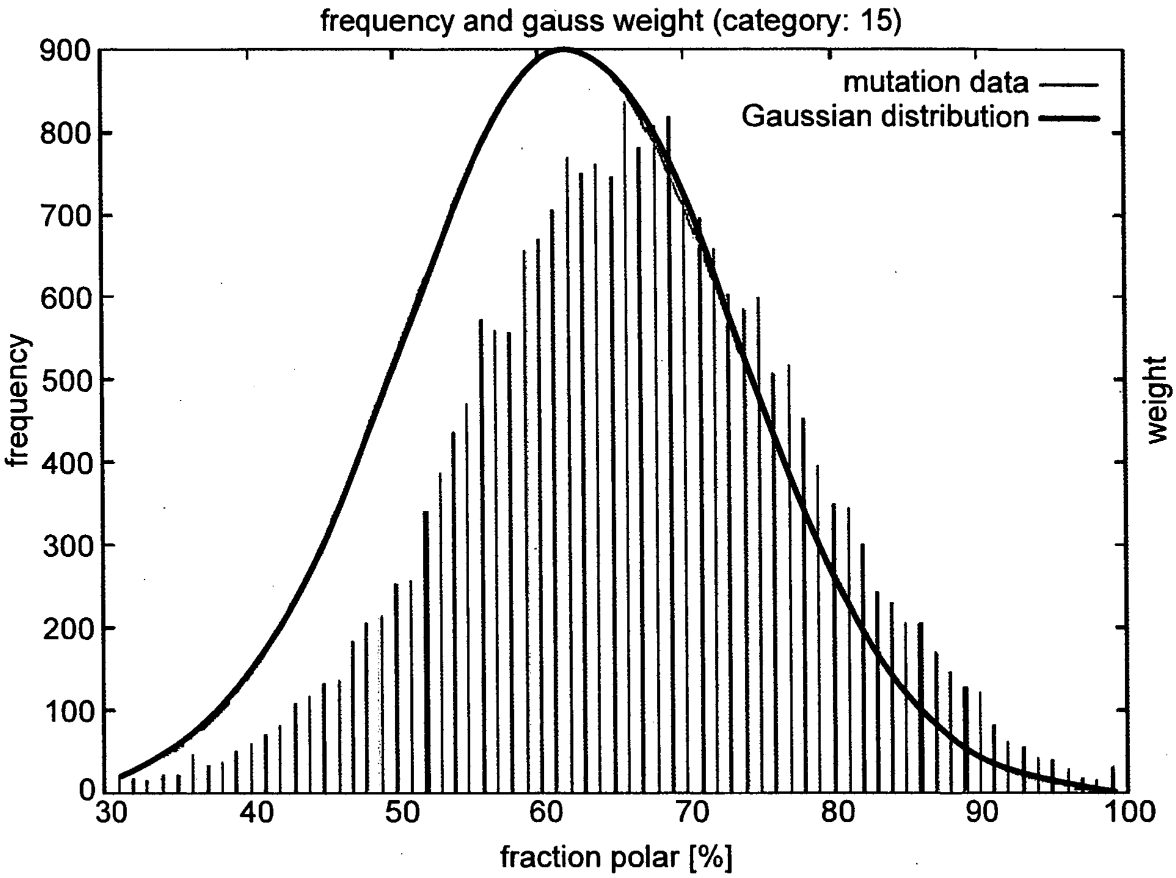
frequency and gauss weight (category: 15)
mutation data
Gaussian distribution
frequency
weight
fraction polar [%]
0
100
200
300
400
500
600
700
800
900
30
40
50
60
70
80
90
100

FIG.47

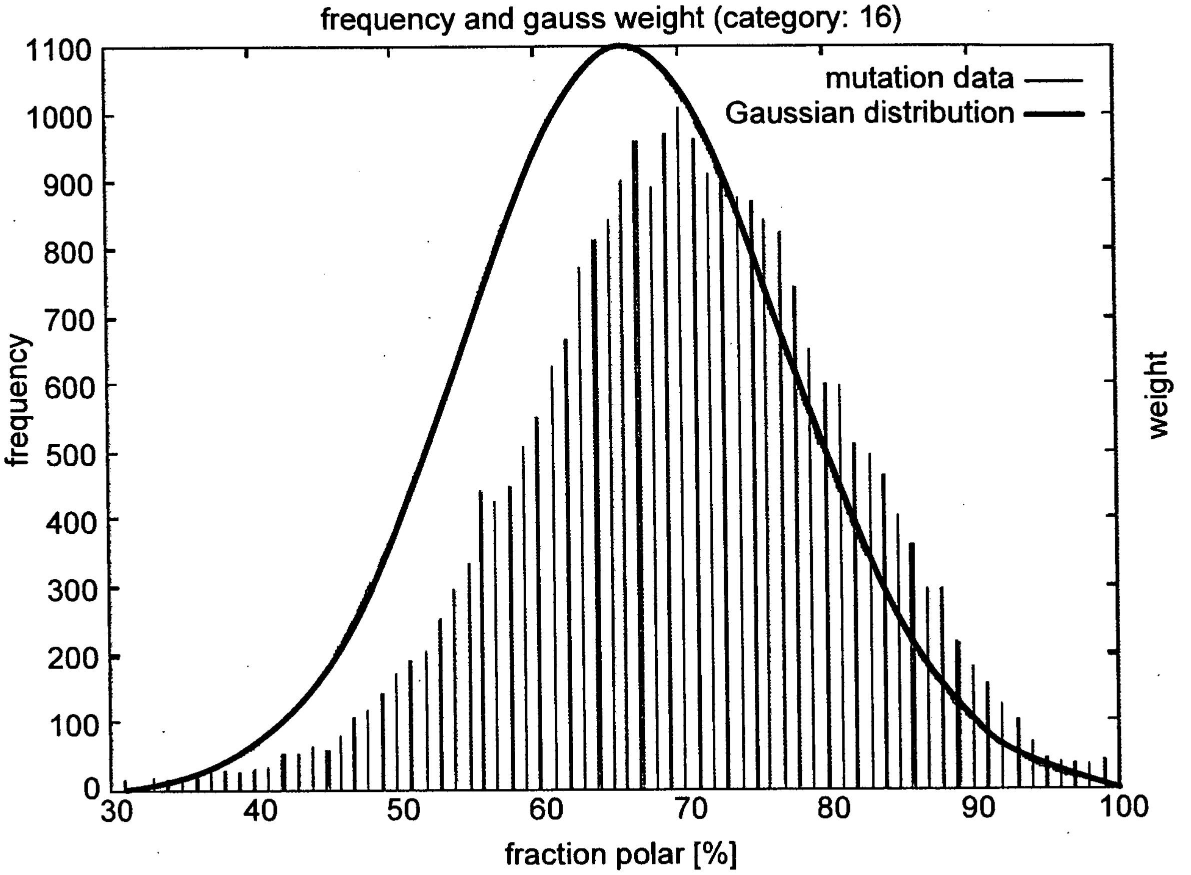
frequency and gauss weight (category: 16)
mutation data
Gaussian distribution
frequency
weight
fraction polar [%]
0
100
200
300
400
500
600
700
800
900
1000
1100
30
40
50
60
70
80
90
100

FIG.48

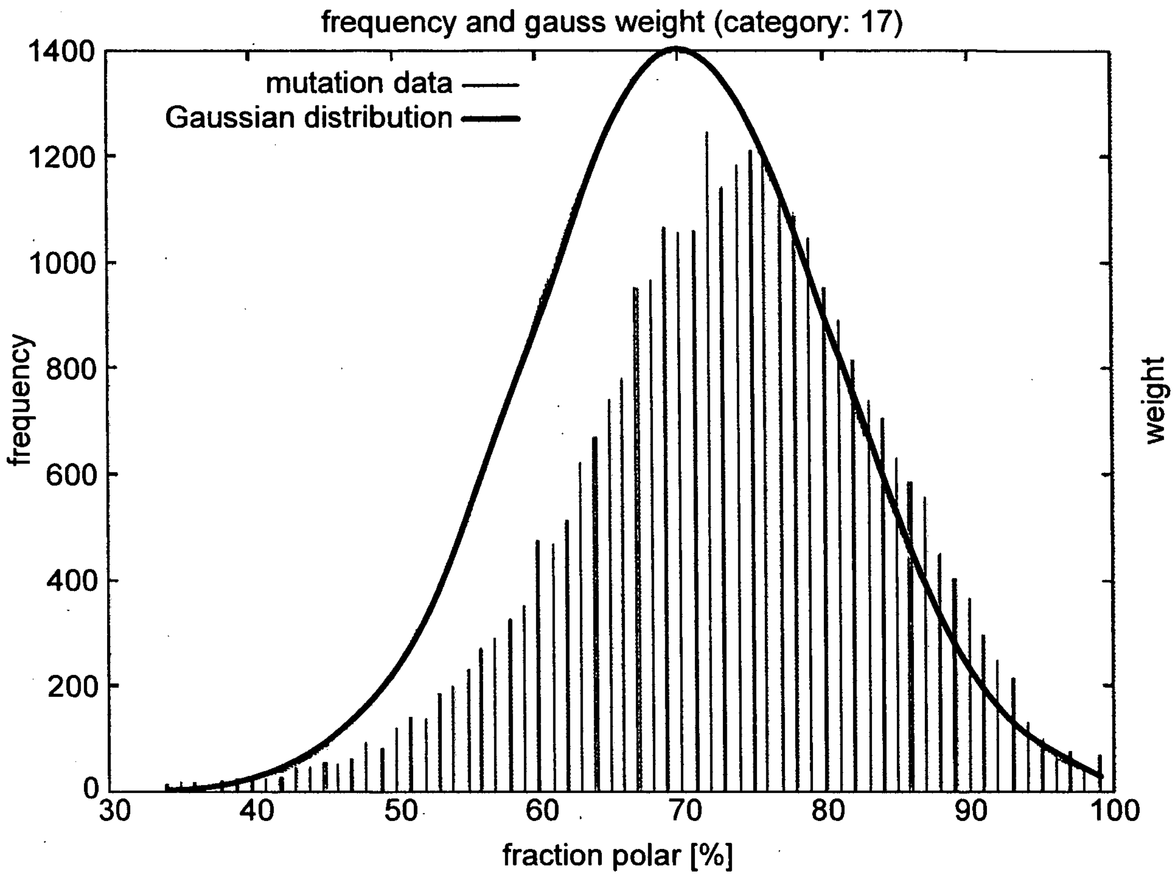
frequency and gauss weight (category: 17)
mutation data
Gaussian distribution
frequency
weight
fraction polar [%]
0
200
400
600
800
1000
1200
1400
30
40
50
60
70
80
90
100

FIG.49

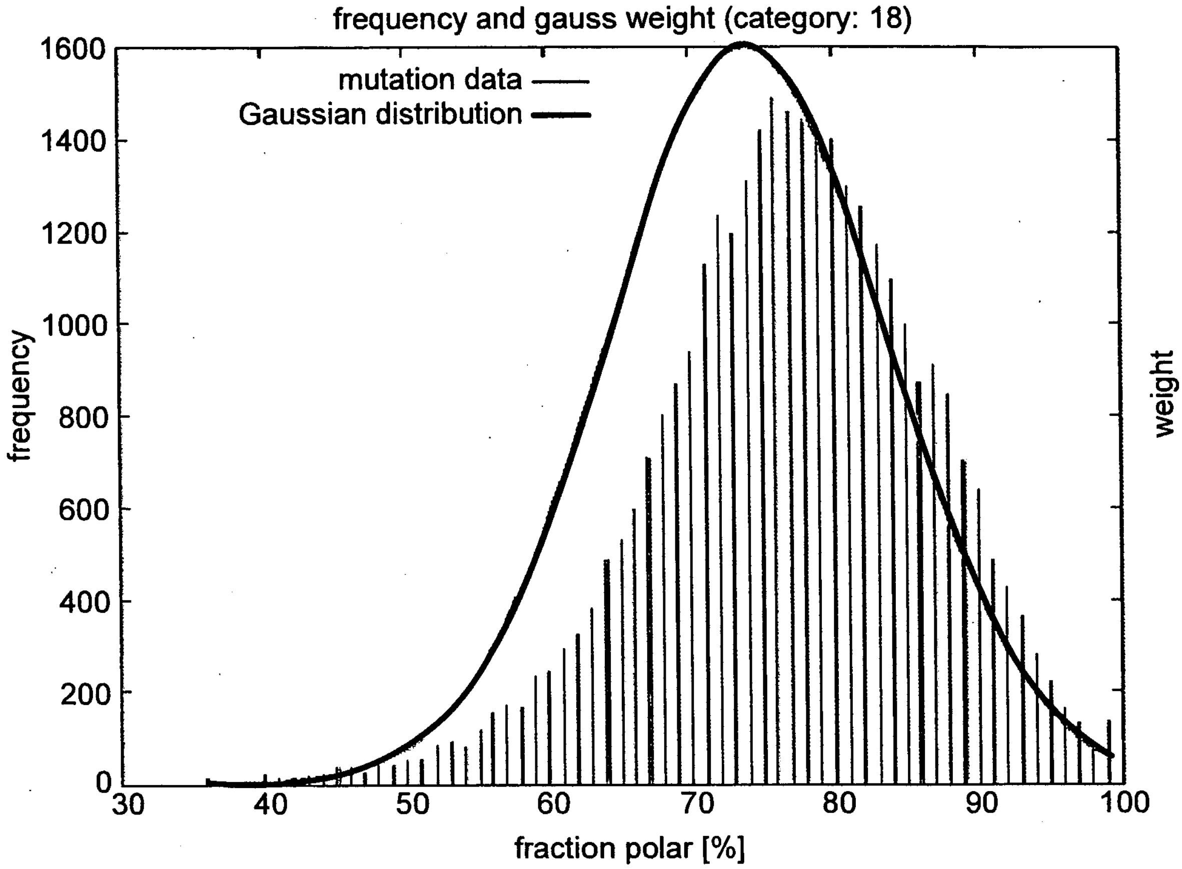
frequency and gauss weight (category: 18)
mutation data
Gaussian distribution
frequency
weight
fraction polar [%]
0
200
400
600
800
1000
1200
1400
1600
30
40
50
60
70
80
90
100

FIG.50

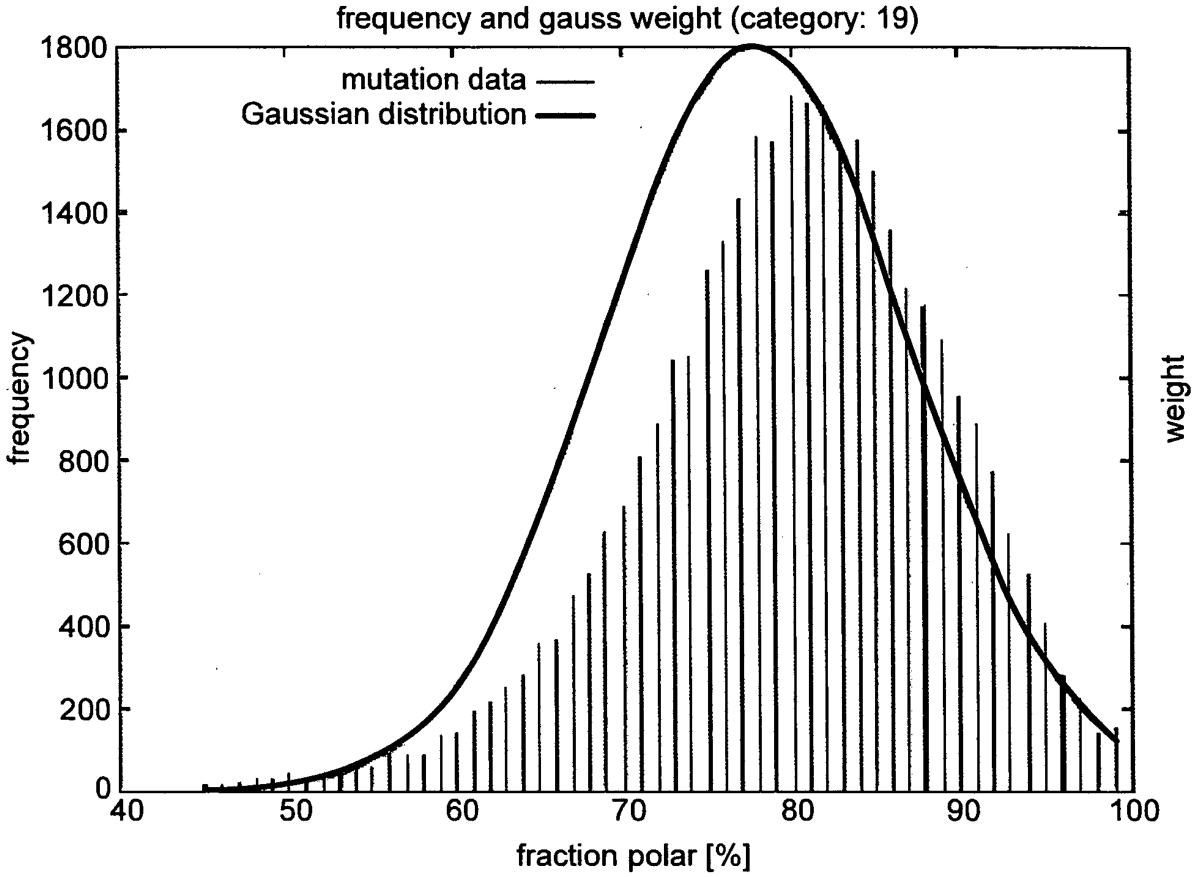
frequency and gauss weight (category: 19)
mutation data
Gaussian distribution
frequency
weight
fraction polar [%]
1800
1600
1400
1200
1000
800
600
400
200
0
40
50
60
70
80
90
100

# FIG.51

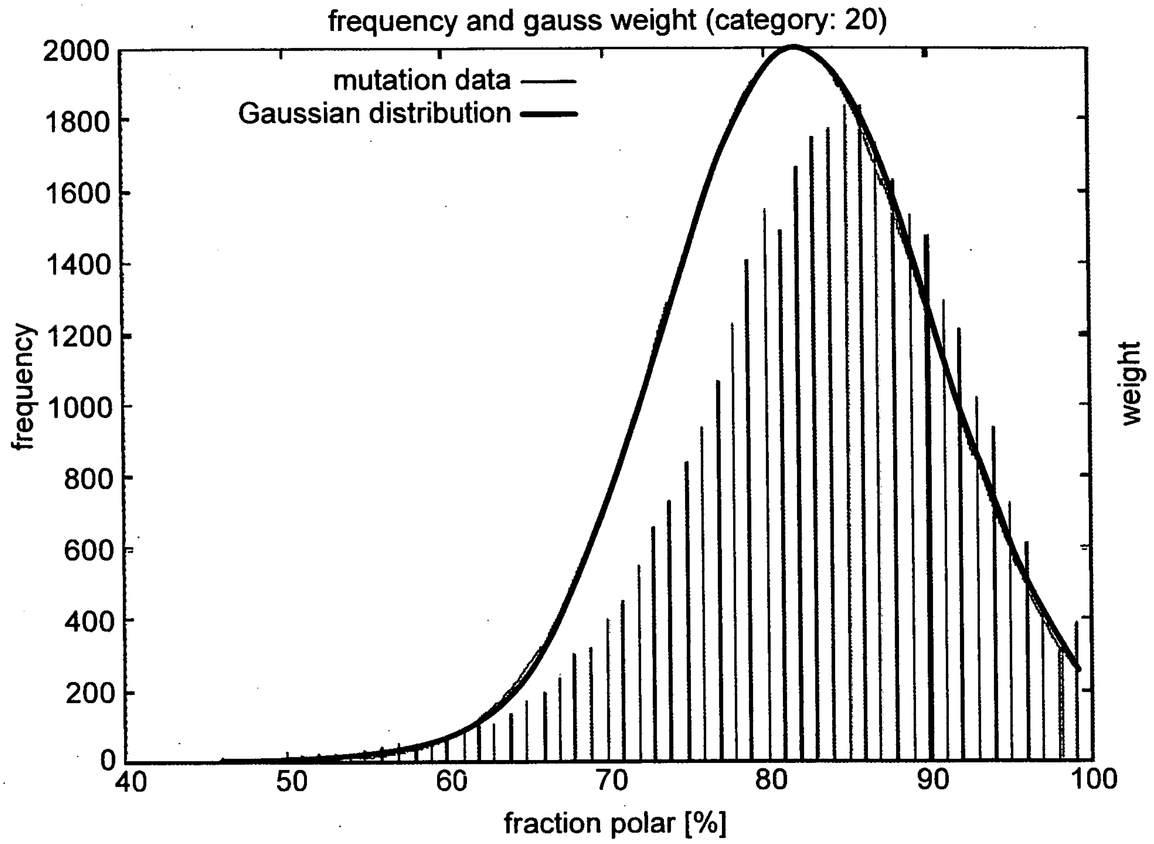
frequency and gauss weight (category: 20)
mutation data
Gaussian distribution
frequency
weight
fraction polar [%]
2000
1800
1600
1400
1200
1000
800
600
400
200
0
40
50
60
70
80
90
100

FIG.52

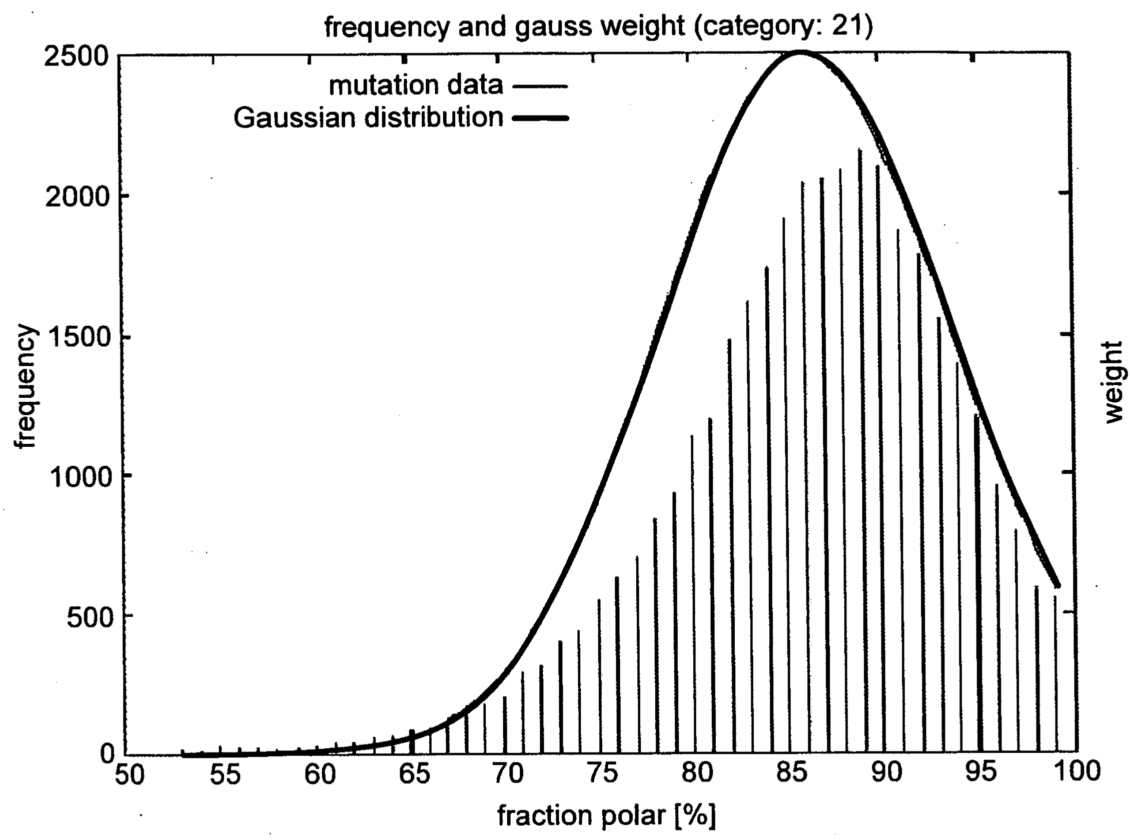
frequency and gauss weight (category: 21)
mutation data
Gaussian distribution
frequency
weight
2500
2000
1500
1000
500
0
50
55
60
65
70
75
80
85
90
95
100
fraction polar [%]

# FIG.53

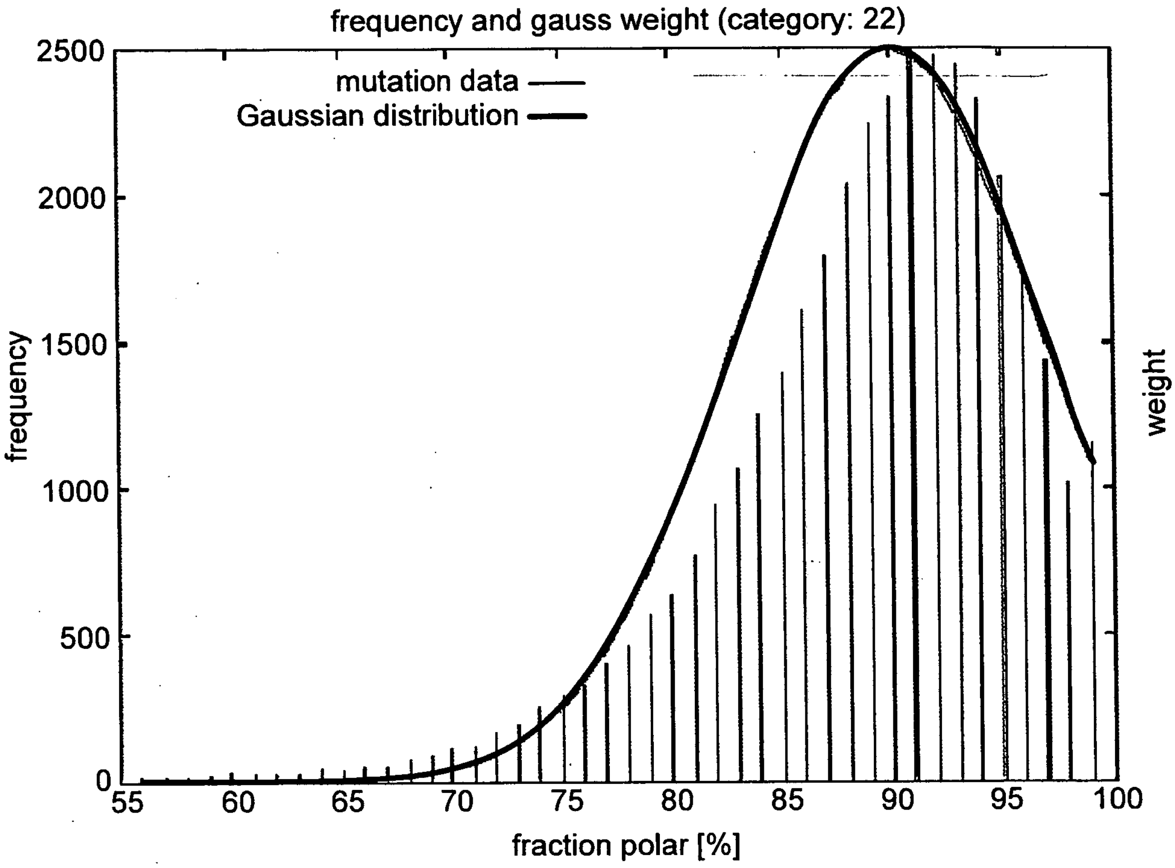
frequency and gauss weight (category: 22)
mutation data
Gaussian distribution
frequency
weight
fraction polar [%]
2500
2000
1500
1000
500
0
55
60
65
70
75
80
85
90
95
100

FIG.54

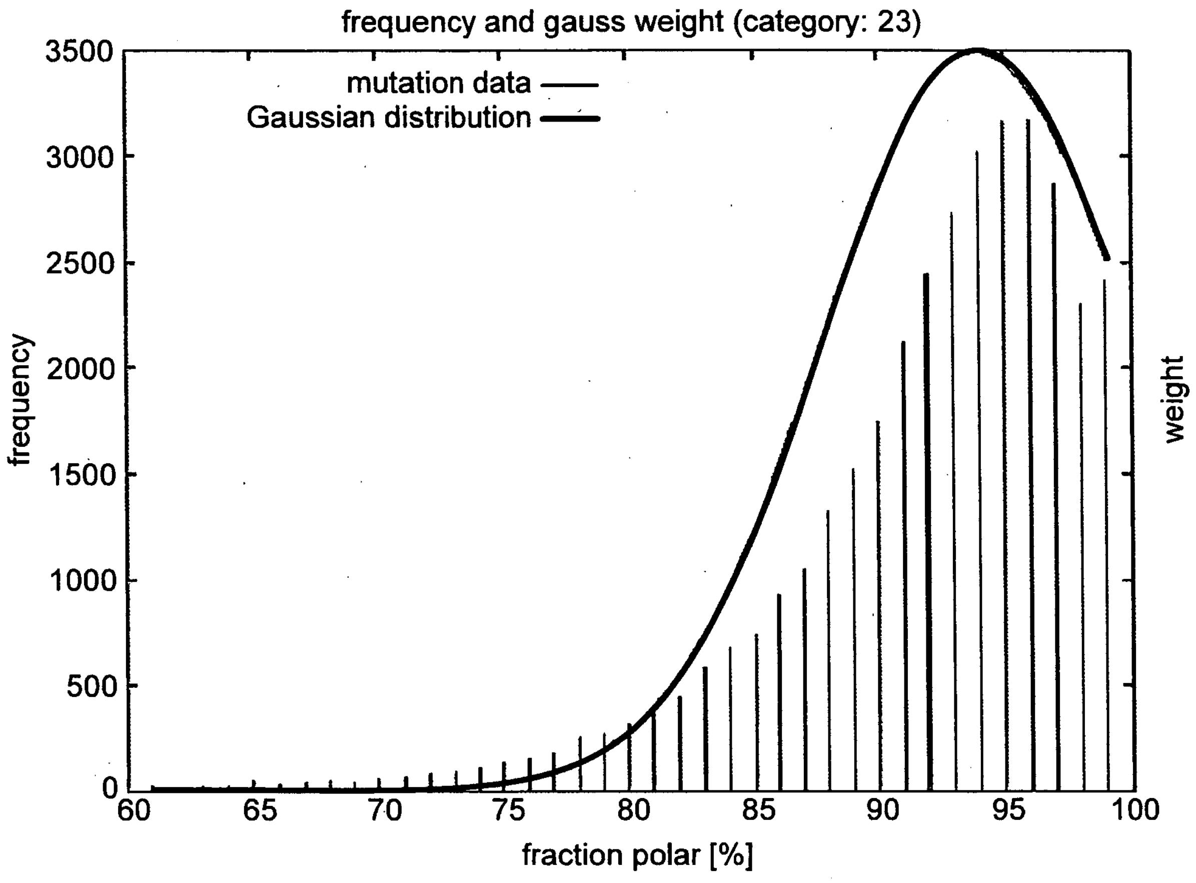
frequency and gauss weight (category: 23)
mutation data
Gaussian distribution
frequency
weight
3500
3000
2500
2000
1500
1000
500
0
60
65
70
75
80
85
90
95
100
fraction polar [%]

# FIG.55

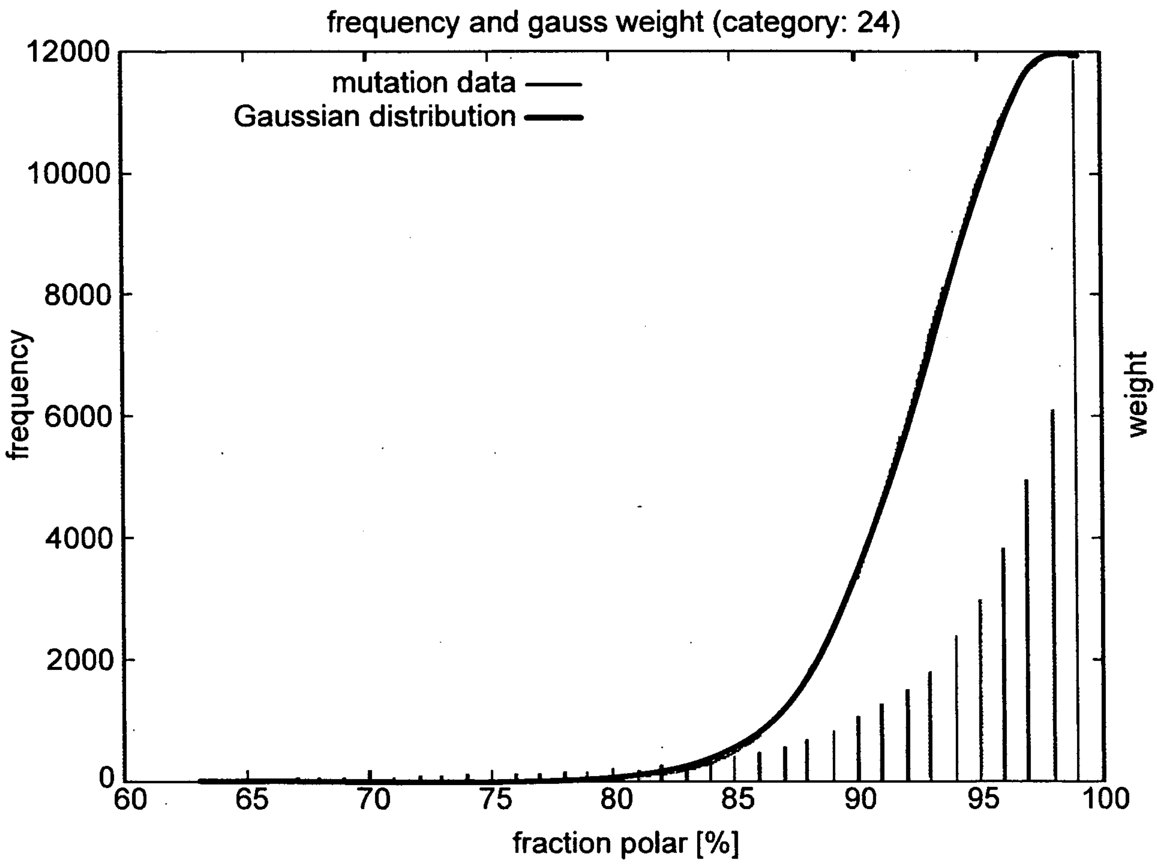
frequency and gauss weight (category: 24)
mutation data
Gaussian distribution
frequency
weight
fraction polar [%]
12000
10000
8000
6000
4000
2000
0
60
65
70
75
80
85
90
95
100

# FIG.56

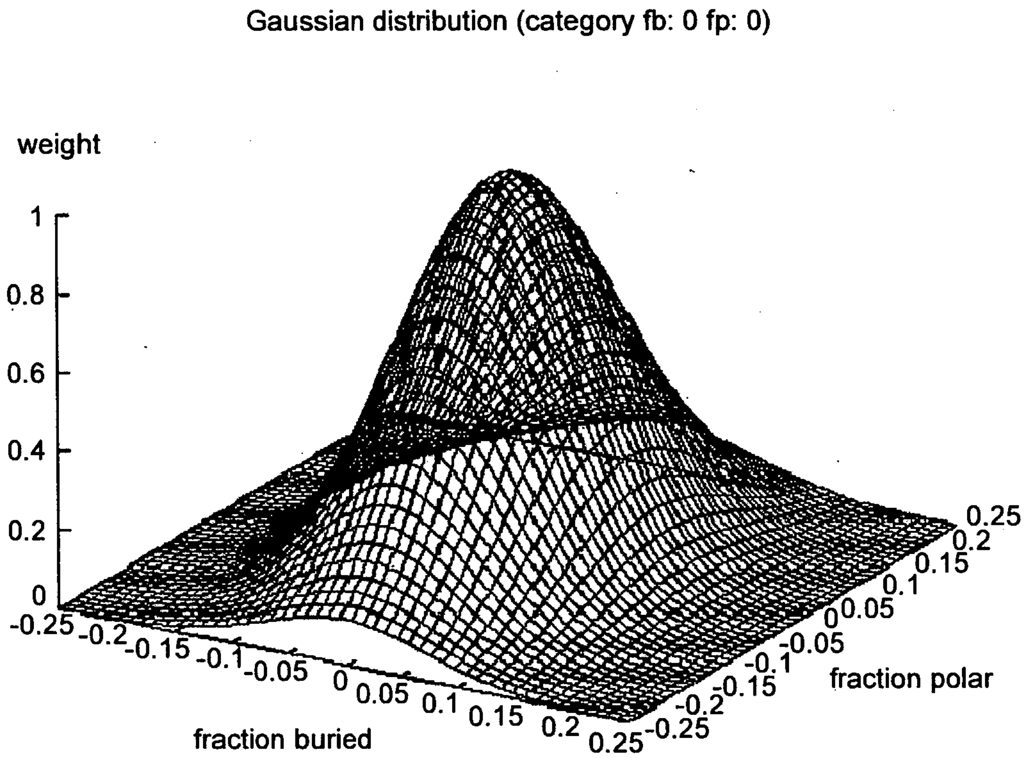
Gaussian distribution (category fb: 0 fp: 0)
weight
1
0.8
0.6
0.4
0.2
0
-0.25 -0.2 -0.15 -0.1 -0.05 0 0.05 0.1 0.15 0.2 0.25
fraction buried
0.25 0.2 0.15 0.1 0.05 0 -0.05 -0.1 -0.15 -0.2 -0.25
fraction polar

# FIG.57

Gaussian distribution (category fb: 12 fp: 12)

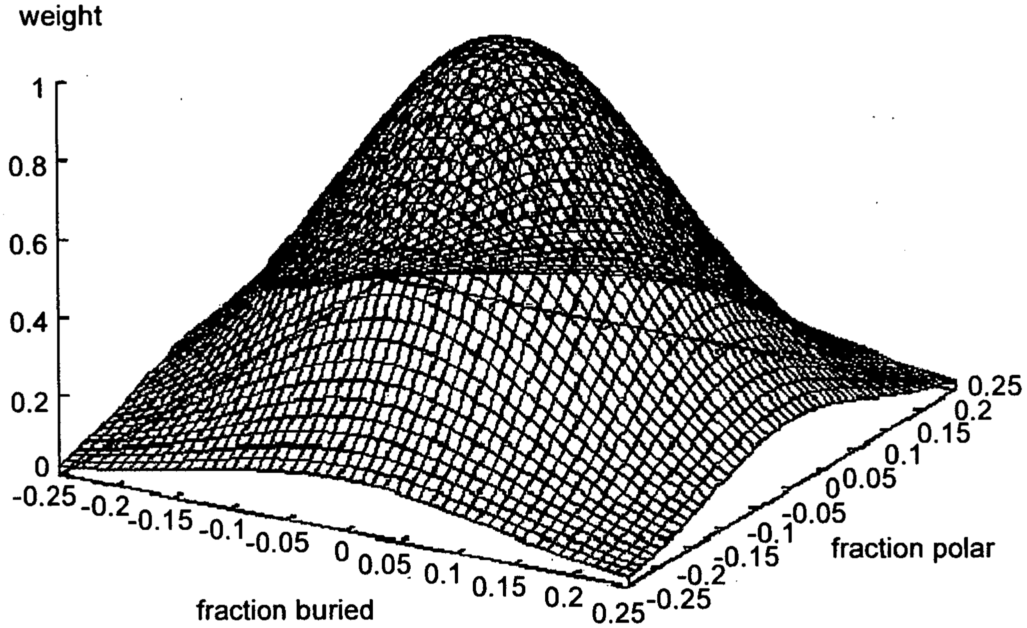

FIG.58

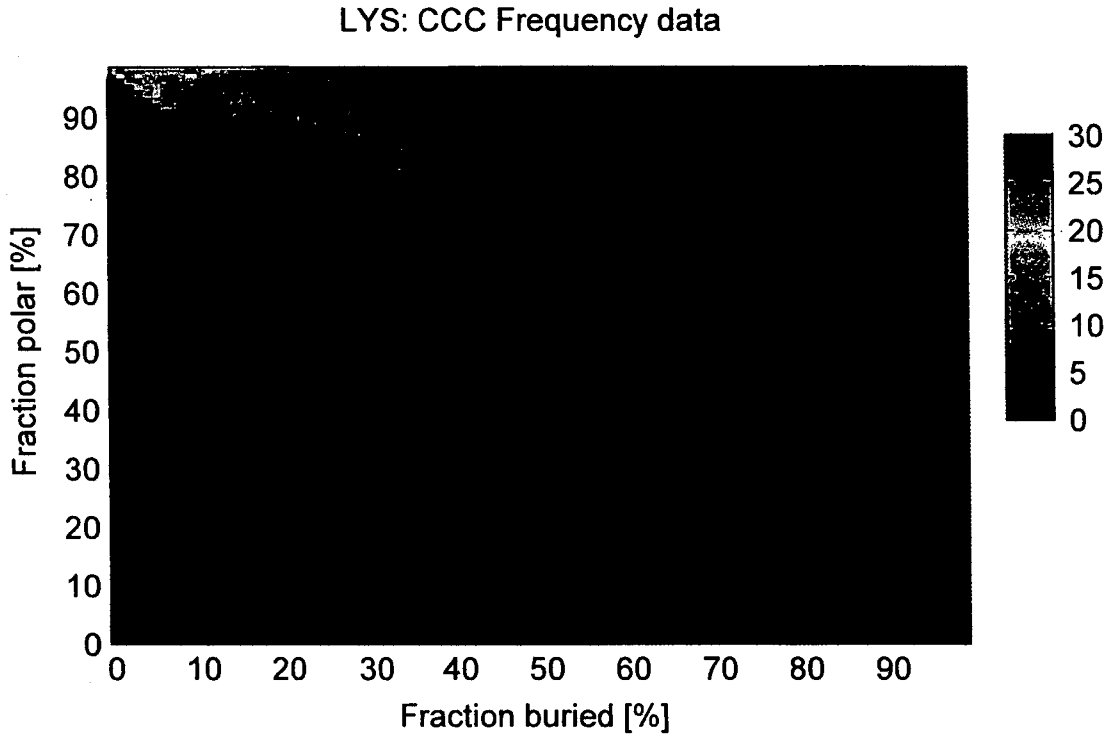
LYS: CCC Frequency data
Fraction polar [%]
0
10
20
30
40
50
60
70
80
90
Fraction buried [%]
0
10
20
30
40
50
60
70
80
90
30
25
20
15
10
5
0

FIG.59

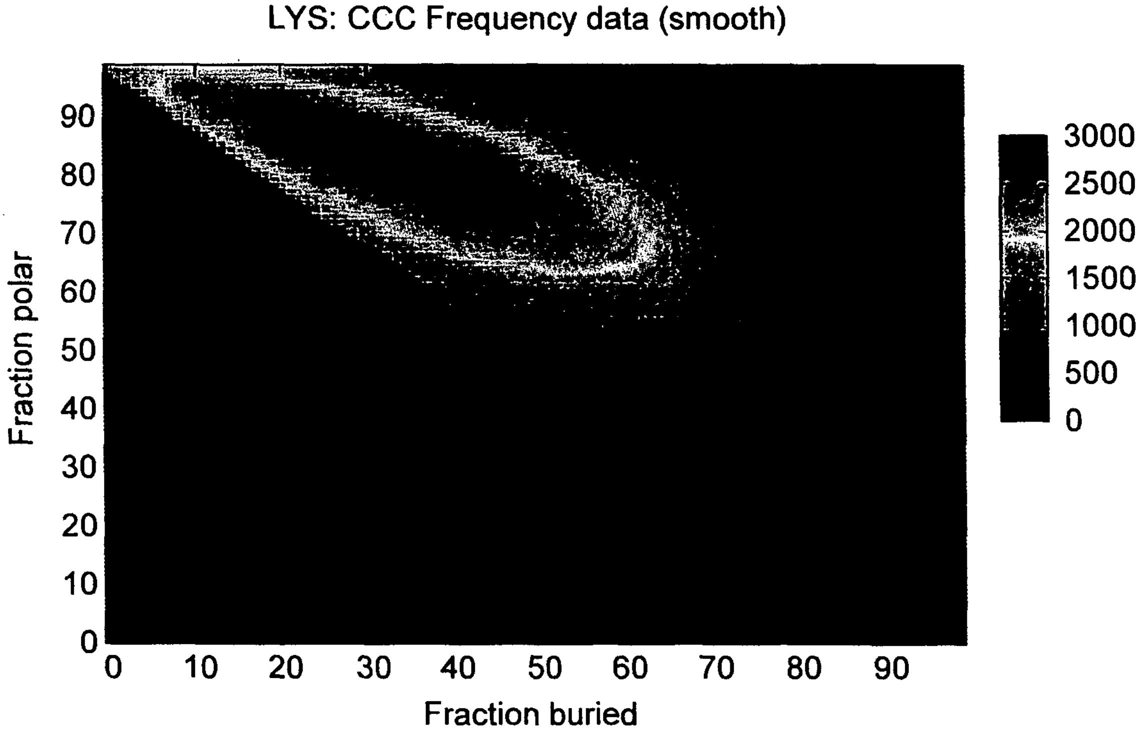
LYS: CCC Frequency data (smooth)
Fraction polar
0
10
20
30
40
50
60
70
80
90
Fraction buried
0
10
20
30
40
50
60
70
80
90
3000
2500
2000
1500
1000
500
0

# FIG.60

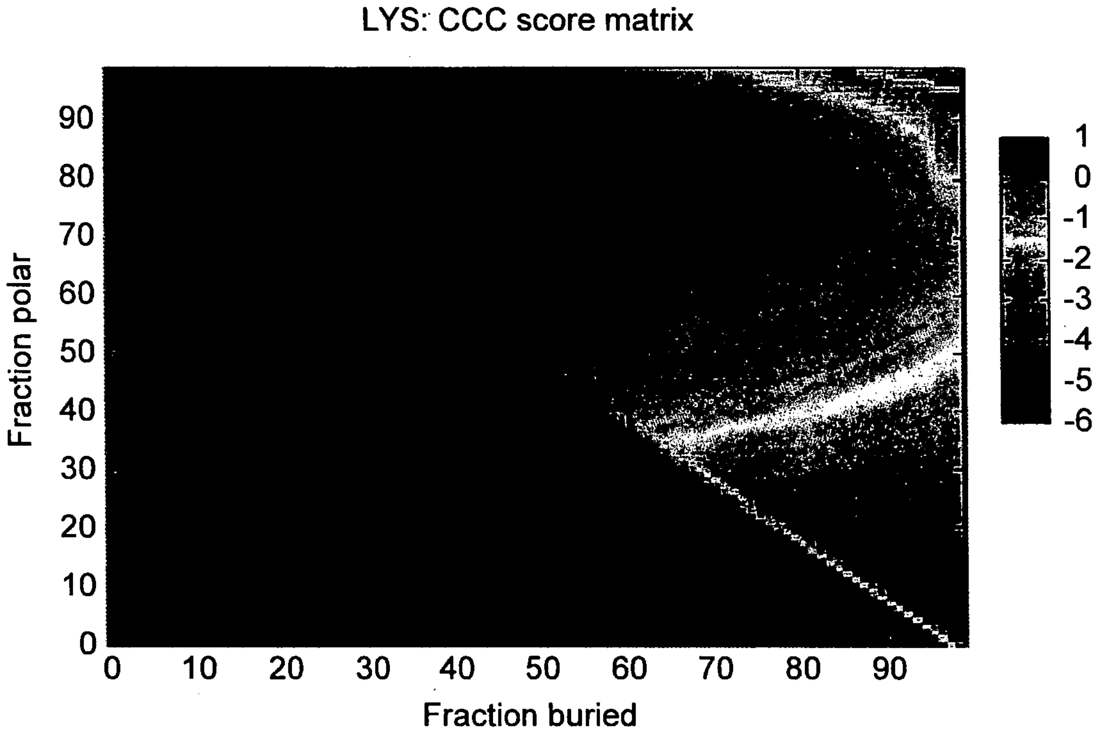
LYS: CCC score matrix
Fraction polar
Fraction buried
0
10
20
30
40
50
60
70
80
90
1
0
-1
-2
-3
-4
-5
-6

FIG.61

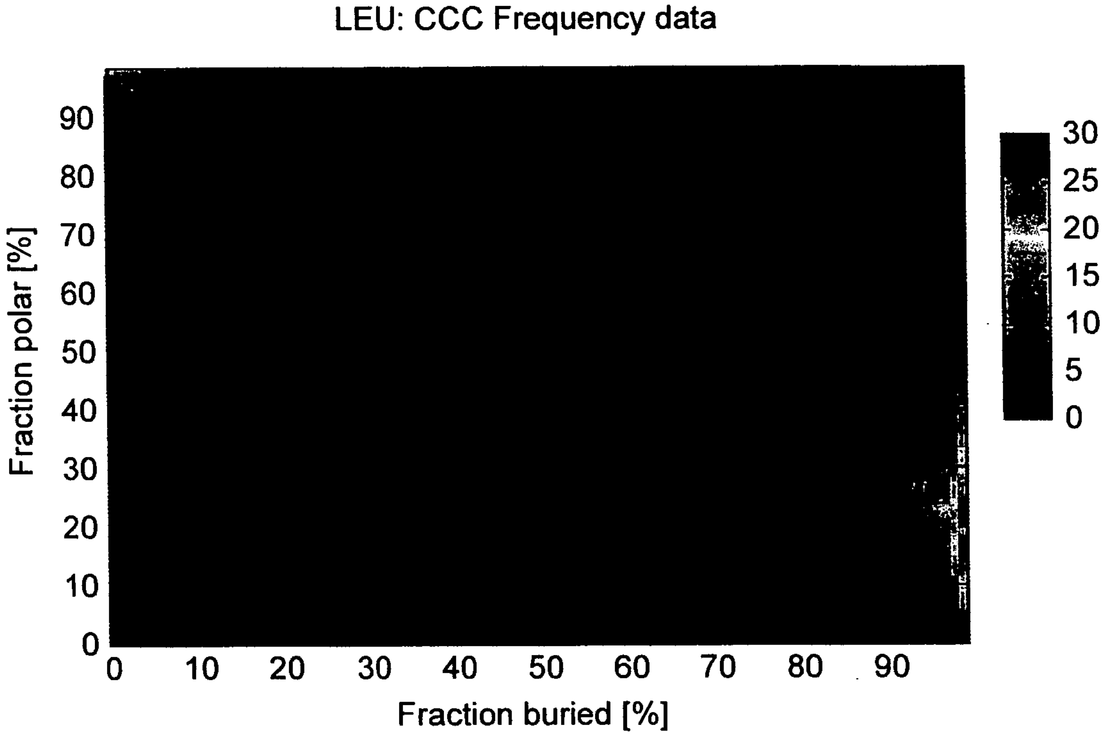
LEU: CCC Frequency data
90
80
70
60
50
40
30
20
10
0
Fraction polar [%]
0
10
20
30
40
50
60
70
80
90
Fraction buried [%]
30
25
20
15
10
5
0

FIG.62

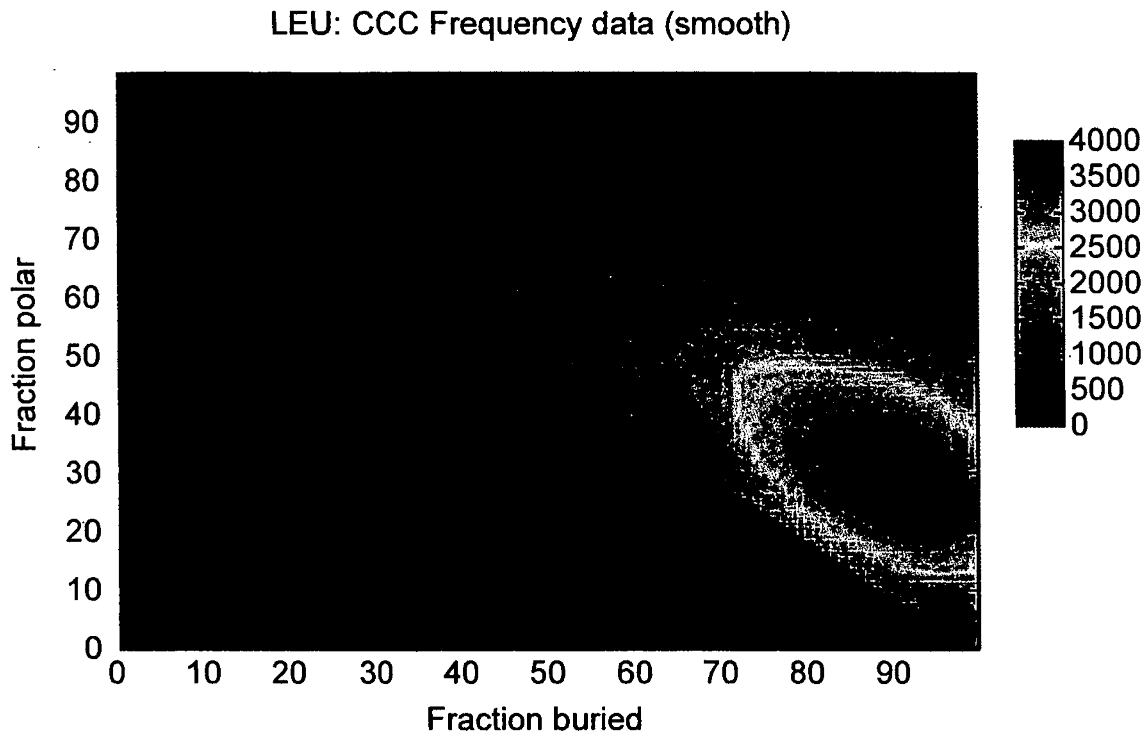
LEU: CCC Frequency data (smooth)
Fraction polar
0
10
20
30
40
50
60
70
80
90
Fraction buried
0
10
20
30
40
50
60
70
80
90
4000
3500
3000
2500
2000
1500
1000
500
0

FIG.63

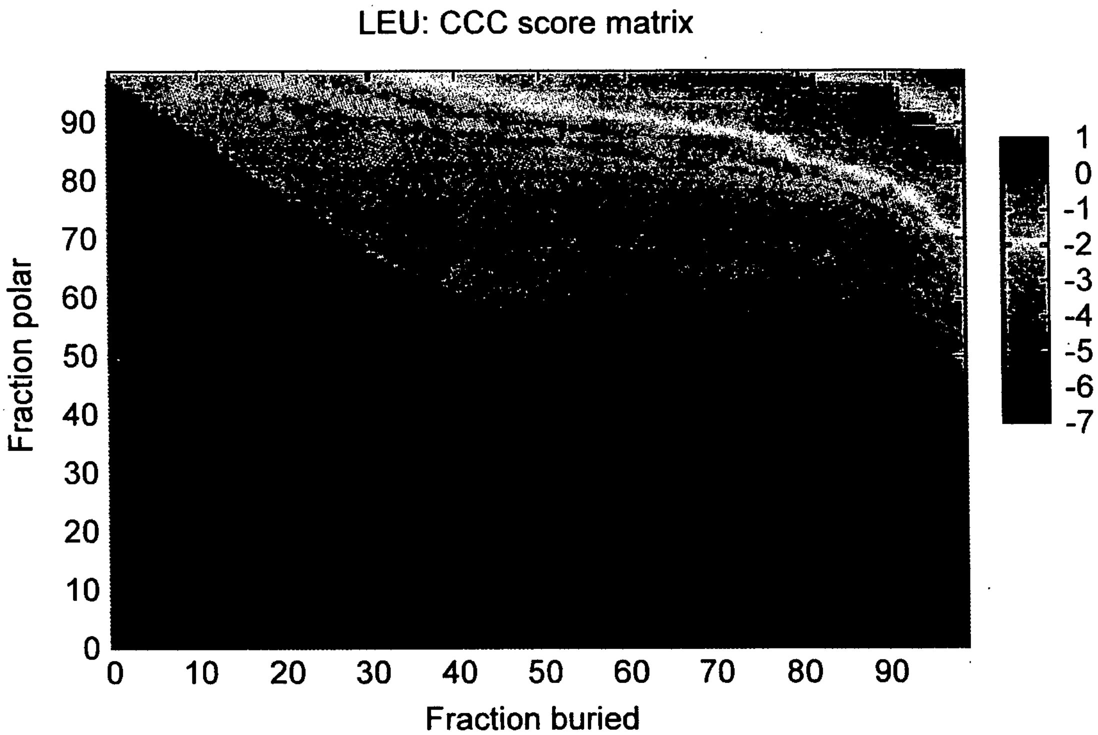
LEU: CCC score matrix
Fraction polar
0
10
20
30
40
50
60
70
80
90
Fraction buried
0
10
20
30
40
50
60
70
80
90
1
0
-1
-2
-3
-4
-5
-6
-7

FIG.64

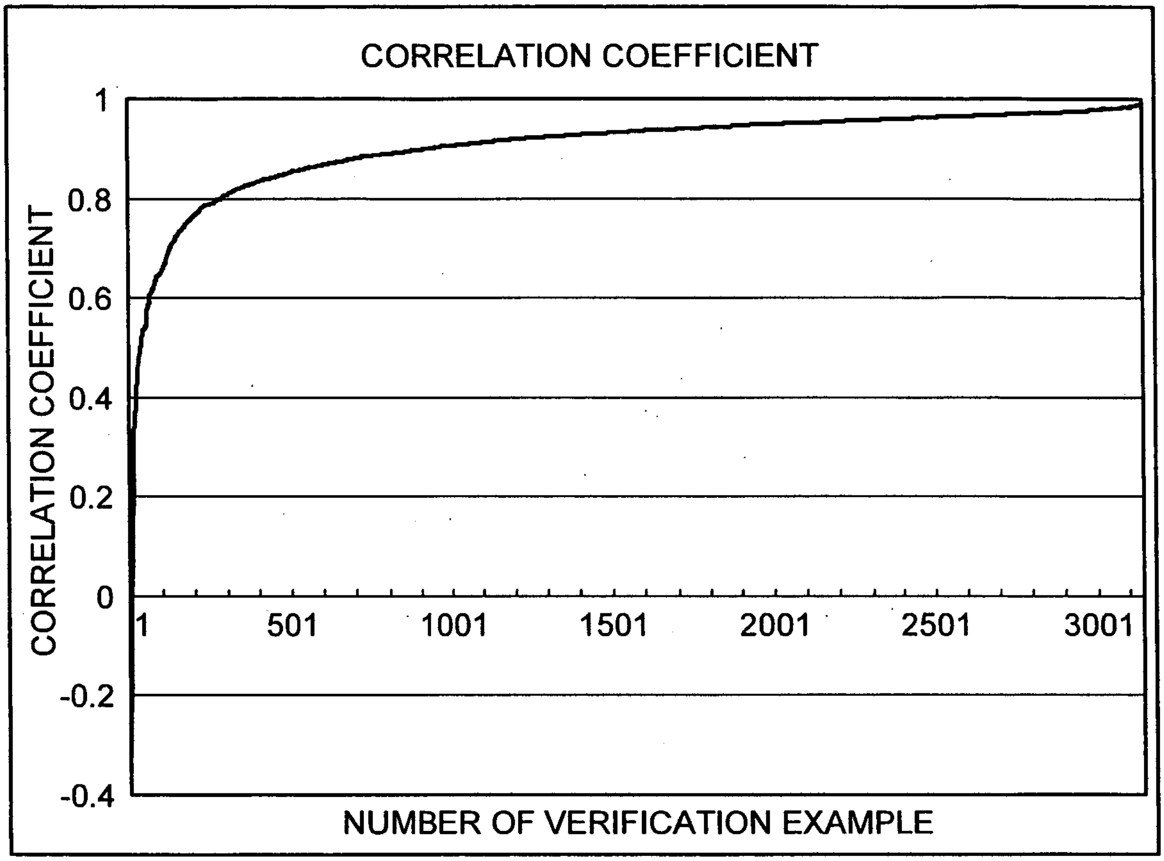
CORRELATION COEFFICIENT
CORRELATION COEFFICIENT
1
0.8
0.6
0.4
0.2
0
-0.2
-0.4
1
501
1001
1501
2001
2501
3001
NUMBER OF VERIFICATION EXAMPLE

# FIG.65A

CAFASP5 Rankings

CAFASP5 Rankings

| # | | | | | | | | | | | | | | | | | | | |
|---|---|---|---|---|---|---|---|---|---|---|---|---|---|---|---|---|---|---|---|
| | Average | 45.516 | | | 15 | 80 | 36 | 97 | 90 | 66 | 45 | 65 | 77 | 6 | 65 | 71 | 7 | 24 |
| | Max | 51.2 | | | 35 | 83 | 38 | 98 | 92 | 75 | 47 | 83 | 82 | 9 | 68 | 76 | 17 | 41 |
| 1. | Zhang-Server | 47.243 | 1 | 0 | 22 | 81 | 37 | 98 | 92 | 67 | 45 | 64 | 74 | 5 | 65 | 74 | 0 | 34 |
| 2 | MQAPCons | 45.103 | 2 | 4 | 33 | 74 | 32 | 91 | 90 | 59 | 41 | 58 | 74 | 5 | 64 | 70 | 15 | 33 |
| 3 | Verify | 44.963 | 3 | 4 | 33 | 83 | 31 | 97 | 91 | 61 | 45 | 64 | 73 | 9 | 64 | 46 | 15 | 37 |
| 4 | Taruna | 43.262 | 4 | 8 | 0 | 79 | 36 | 97 | 88 | 63 | 44 | 61 | 75 | 0 | 58 | 70 | 15 | 0 |
| 5 | Pmodeller6 | 42.836 | 5 | 9 | 0 | 75 | 32 | 91 | 77 | 61 | 43 | 53 | 75 | 0 | 58 | 66 | 15 | 37 |
| 6 | Pcons6 | 42.791 | 6 | 9 | 0 | 77 | 37 | 96 | 91 | 62 | 41 | 58 | 74 | 6 | 65 | 65 | 0 | 34 |
| 7 | CIRCLE | 42.459 | 7 | 10 | 20 | 78 | 33 | 97 | 90 | 63 | 44 | 56 | 75 | 6 | 59 | 70 | 0 | 30 |
| 8 | HHpred2 | 42.379 | 8 | 10 | 0 | 73 | 37 | 96 | 60 | 62 | 45 | 44 | 79 | 0 | 61 | 76 | 12 | 0 |
| 9 | ROBETTA | 42.318 | 9 | 10 | 33 | 80 | 31 | 96 | 91 | 61 | 45 | 64 | 73 | 9 | 64 | 46 | 15 | 34 |
| 10 | HHpred3 | 41.868 | 10 | 11 | 22 | 73 | 36 | 96 | 60 | 62 | 44 | 44 | 79 | 0 | 63 | 76 | 0 | 0 |
| 11 | HHpred1 | 41.814 | 11 | 11 | 0 | 79 | 38 | 97 | 91 | 61 | 46 | 61 | 73 | 0 | 62 | 69 | 0 | 0 |
| 12 | BayesHH | 41.592 | 12 | 11 | 23 | 74 | 37 | 96 | 61 | 62 | 44 | 44 | 77 | 0 | 63 | 72 | 0 | 0 |
| 13 | UNI-EID_expm | 41.393 | 13 | 12 | 0 | 80 | 36 | 96 | 67 | 63 | 44 | 60 | 76 | 0 | 68 | 69 | 0 | 0 |
| 14 | FAMSD | 40.894 | 14 | 13 | 0 | 78 | 32 | 96 | 91 | 63 | 47 | 57 | 82 | 6 | 61 | 70 | 0 | 0 |
| 15 | beautshot | 40.684 | 15 | 13 | 0 | 78 | 37 | 97 | 88 | 64 | 44 | 61 | 75 | 0 | 62 | 64 | 0 | 0 |
| 16 | UNI-EID_bnmx | 40.608 | 16 | 14 | 0 | 82 | 37 | 96 | 91 | 49 | 43 | 60 | 75 | 0 | 66 | 69 | 0 | 0 |
| 17 | FAMS | 40.512 | 17 | 14 | 0 | 76 | 33 | 90 | 90 | 66 | 46 | 56 | 62 | 6 | 54 | 60 | 0 | 0 |
| 18 | RAPTOR-ACE | 40.454 | 18 | 14 | 0 | 79 | 31 | 88 | 90 | 60 | 42 | 60 | 73 | 0 | 65 | 70 | 0 | 35 |
| 19 | MetaTasser | 40.374 | 19 | 14 | 0 | 78 | 24 | 88 | 54 | 37 | 38 | 42 | 75 | 0 | 68 | 44 | 0 | 0 |
| 20 | shub | 39.91 | 20 | 15 | 0 | 82 | 36 | 96 | 89 | 59 | 47 | 66 | 72 | 0 | 62 | 61 | 0 | 0 |

FIG.65B

| # | | | | | | | | | | | | | | | | | | |
|---|---|---|---|---|---|---|---|---|---|---|---|---|---|---|---|---|---|---|
| 21 | FUNCTION | 39.849 | 21 | 15 | 0 | 78 | 33 | 87 | 90 | 64 | 46 | 58 | 82 | 4 | 62 | 65 | 0 | 30 |
| 22 | SP3 | 39.761 | 22 | 15 | 0 | 80 | 33 | 91 | 66 | 63 | 41 | 56 | 75 | 5 | 65 | 70 | 0 | 35 |
| 23 | RAPTOR | 39.611 | 23 | 16 | 0 | 78 | 35 | 97 | 89 | 60 | 45 | 54 | 72 | 0 | 64 | 66 | 0 | 0 |
| 24 | UNI-EID_sfst | 39.448 | 24 | 16 | 0 | 79 | 36 | 96 | 91 | 49 | 41 | 60 | 75 | 0 | 64 | 69 | 0 | 0 |
| 25 | SP4 | 39.423 | 25 | 16 | 0 | 79 | 36 | 91 | 66 | 63 | 44 | 58 | 75 | 0 | 64 | 70 | 0 | 0 |
| 26 | beautshotbase | 39.386 | 26 | 16 | 0 | 77 | 35 | 96 | 90 | 61 | 43 | 57 | 75 | 0 | 65 | 43 | 0 | 0 |
| 27 | RAPTORESS | 39.288 | 27 | 16 | 0 | 78 | 37 | 97 | 87 | 59 | 40 | 54 | 71 | 0 | 63 | 66 | 0 | 0 |
| 28 | SAM_T06_server | 38.947 | 28 | 17 | 0 | 78 | 30 | 97 | 70 | 57 | 38 | 55 | 65 | 5 | 62 | 70 | 0 | 0 |

FIG.66A

CAFASP5 MaxSubDom Ra..

CAFASP5 MaxSubDom Rankings

| # | | | | | | | | | | | | | | | | | | | |
|---|---|---|---|---|---|---|---|---|---|---|---|---|---|---|---|---|---|---|---|
| | Average | 46.626 | | | 15 | 80 | 41 | 97 | 90 | 66 | 45 | 77 | 77 | 6 | 65 | 71 | 7 | 24 |
| | Max | 52.577 | | | 35 | 83 | 50 | 98 | 92 | 75 | 47 | 83 | 82 | 9 | 68 | 76 | 17 | 41 |
| 1 | Zhang-Server | 48.447 | 1 | 0 | 22 | 81 | 37 | 98 | 92 | 67 | 45 | 75 | 74 | 5 | 65 | 74 | 0 | 34 |
| 2 | MQAPCons | 46.213 | 2 | 4 | 33 | 74 | 43 | 91 | 90 | 59 | 41 | 77 | 74 | 5 | 64 | 70 | 15 | 33 |
| 3 | Verify | 45.942 | 3 | 5 | 33 | 83 | 41 | 97 | 91 | 61 | 45 | 64 | 73 | 9 | 64 | 46 | 15 | 37 |
| 4 | Taruna | 44.189 | 4 | 8 | 0 | 79 | 36 | 97 | 88 | 63 | 44 | 76 | 75 | 0 | 58 | 70 | 15 | 0 |
| 5 | HHpred2 | 43.981 | 5 | 9 | 0 | 73 | 37 | 96 | 72 | 62 | 45 | 74 | 79 | 0 | 61 | 76 | 12 | 0 |
| 6 | Pmodeller6 | 43.949 | 6 | 9 | 0 | 75 | 32 | 91 | 77 | 61 | 43 | 75 | 75 | 0 | 58 | 66 | 15 | 37 |
| 7 | CIRCLE | 43.603 | 7 | 9 | 20 | 78 | 42 | 97 | 90 | 63 | 44 | 79 | 75 | 6 | 59 | 70 | 0 | 30 |
| 8 | Pcons6 | 43.497 | 8 | 10 | 0 | 77 | 37 | 96 | 91 | 62 | 41 | 71 | 74 | 6 | 65 | 65 | 0 | 34 |
| 9 | HHpred3 | 43.479 | 9 | 10 | 22 | 73 | 36 | 96 | 72 | 62 | 44 | 74 | 79 | 0 | 63 | 76 | 0 | 0 |
| 10 | ROBETTA | 43.231 | 10 | 10 | 33 | 80 | 41 | 96 | 91 | 61 | 45 | 64 | 73 | 9 | 64 | 46 | 15 | 34 |
| 11 | BayesHH | 42.888 | 11 | 11 | 23 | 74 | 47 | 96 | 61 | 62 | 44 | 75 | 77 | 0 | 63 | 72 | 0 | 0 |
| 12 | HHpred1 | 42.784 | 12 | 11 | 0 | 79 | 38 | 97 | 91 | 61 | 46 | 72 | 73 | 0 | 62 | 69 | 0 | 0 |
| 13 | MetaTasser | 42.072 | 13 | 13 | 0 | 78 | 34 | 88 | 54 | 51 | 38 | 53 | 75 | 0 | 68 | 44 | 0 | 0 |
| 14 | UNI-EID_expm | 41.955 | 14 | 13 | 0 | 80 | 36 | 96 | 67 | 63 | 44 | 60 | 76 | 0 | 68 | 69 | 0 | 0 |
| 15 | FAMSD | 41.872 | 15 | 13 | 0 | 78 | 42 | 98 | 91 | 63 | 47 | 74 | 82 | 6 | 61 | 70 | 0 | 0 |
| 16 | beautshot | 41.652 | 16 | 14 | 0 | 78 | 37 | 97 | 88 | 64 | 44 | 77 | 75 | 0 | 62 | 64 | 0 | 0 |
| 17 | FAMS | 41.64 | 17 | 14 | 0 | 76 | 33 | 90 | 90 | 66 | 46 | 79 | 79 | 6 | 54 | 60 | 0 | 0 |
| 18 | RAPTOR-ACE | 41.505 | 18 | 14 | 0 | 79 | 43 | 88 | 90 | 60 | 42 | 74 | 73 | 0 | 65 | 70 | 0 | 35 |
| 19 | UNI-EID_bnmx | 41.163 | 19 | 15 | 0 | 82 | 37 | 96 | 91 | 49 | 43 | 60 | 75 | 0 | 66 | 69 | 0 | 0 |
| 20 | FUNCTION | 40.907 | 20 | 15 | 0 | 76 | 33 | 87 | 90 | 64 | 46 | 77 | 82 | 4 | 62 | 65 | 0 | 30 |

# FIG.66B

| # | | | | | | | | | | | | | | | | | | |
|---|---|---|---|---|---|---|---|---|---|---|---|---|---|---|---|---|---|---|
| 21 | SP3 | 40.73 | 21 | 15 | 0 | 90 | 33 | 91 | 66 | 63 | 41 | 79 | 75 | 5 | 65 | 70 | 0 | 35 |
| 22 | shub | 40.688 | 22 | 16 | 0 | 82 | 36 | 96 | 89 | 59 | 47 | 66 | 72 | 0 | 62 | 61 | 0 | 0 |
| 23 | RAPTOR | 40.53 | 23 | 16 | 0 | 78 | 48 | 97 | 89 | 60 | 45 | 72 | 72 | 0 | 64 | 66 | 0 | 0 |
| 24 | beautshotbase | 40.28 | 24 | 16 | 0 | 77 | 35 | 96 | 90 | 61 | 43 | 79 | 75 | 0 | 65 | 43 | 0 | 0 |
| 25 | RAPTORESS | 40.249 | 25 | 16 | 0 | 78 | 50 | 97 | 87 | 59 | 40 | 71 | 71 | 0 | 63 | 66 | 0 | 0 |
| 26 | SP4 | 40.215 | 26 | 16 | 0 | 79 | 36 | 91 | 66 | 63 | 44 | 78 | 75 | 0 | 64 | 70 | 0 | 0 |
| 27 | UNI-EID_sfst | 39.909 | 27 | 17 | 0 | 79 | 36 | 96 | 91 | 49 | 41 | 60 | 75 | 0 | 64 | 69 | 0 | 0 |
| 28 | SAM_T06_server | 39.773 | 28 | 17 | 0 | 78 | 40 | 97 | 70 | 57 | 38 | 67 | 65 | 5 | 62 | 70 | 0 | 0 |

# FIG.67A

Automated assessment..

Cumulative Score of all 86 targets, (ranked by TM-score of the first model)

| Predictors (N) | Rank | TM_1(Zscore) | MS_1(Zscore) | GDT_1(Zscore) | RM_1(cov) | DGyr( NC) | TM_B(Zscore) | MS_B(Zscore) | GDT_B(Zscore) | RM_B(cov) | DGyr( NC) |
|---|---|---|---|---|---|---|---|---|---|---|---|
| Zhang-Server(86) | 1 | 58.15( 77.8) | 48.79( 78.7) | 52.09( 77.5) | 6.8(100) | 1.0( 0) | 60.26( 90.1) | 50.66( 83.9) | 54.09( 88.5) | 6.0(100) | 0.9( 0) |
| Pmodeller6(86) | 2 | 55.05( 49.3) | 44.98( 47.2) | 49.00( 51.7) | 7.3( 95) | 1.5( 1) | 57.79( 63.0) | 48.27( 60.9) | 51.78( 63.4) | 7.1( 98) | 1.4( 0) |
| MetaTasser(86) | 3 | 54.87( 46.8) | 43.31( 32.1) | 47.72( 41.3) | 7.3(100) | 1.2( 0) | 56.38( 47.1) | 45.45( 33.8) | 49.28( 39.8) | 7.0(100) | 1.1( 0) |
| ROBETTA(86) | 4 | 54.70( 49.7) | 44.47( 45.9) | 48.78( 50.0) | 7.6(100) | 0.9( 0) | 57.92( 67.5) | 47.98( 63.1) | 51.57( 63.5) | 7.0(100) | 0.9( 0) |
| CIRCLE(86) | 5 | 54.66( 41.5) | 44.70( 38.2) | 48.62( 41.0) | 8.6( 99) | 1.7( 0) | 56.32( 43.0) | 46.77( 40.2) | 50.47( 49.8) | 7.1( 98) | 0.9( 0) |
| Pcons6(86) | 6 | 54.46( 43.6) | 44.94( 44.5) | 48.40( 42.7) | 7.5( 95) | 1.3( 0) | 56.33( 44.1) | 46.83( 42.7) | 50.36( 44.3) | 7.3( 97) | 1.4( 0) |
| HHpred2(86) | 7 | 54.34( 37.6) | 44.76( 37.7) | 48.67( 40.8) | 18.2( 99) | 6.4( 0) | 54.34( 20.6) | 44.76( 18.4) | 48.67( 29.2) | 18.2( 99) | 6.4( 0) |
| beautshot(86) | 8 | 54.31( 39.9) | 44.21( 35.5) | 47.74( 34.7) | 7.7( 98) | 1.0( 1) | 54.31( 23.6) | 44.21( 15.9) | 47.74( 17.0) | 7.7( 98) | 1.0( 1) |
| FAMSD(86) | 9 | 54.09( 35.8) | 44.11( 34.0) | 48.09( 36.7) | 8.0( 99) | 1.3( 0) | 55.56( 34.4) | 45.88( 31.8) | 49.42( 31.6) | 7.3( 97) | 1.1( 0) |
| UNI-EID_expm(86) | 10 | 54.08( 33.9) | 44.58( 35.5) | 48.01( 31.8) | 7.4( 94) | 1.1( 28) | 54.08( 16.4) | 44.58( 15.7) | 48.01( 19.1) | 7.4( 94) | 1.1( 28) |
| RAPTOR-ACE(86) | 11 | 53.96( 37.1) | 43.75( 32.1) | 47.68( 34.5) | 8.2(100) | 1.1( 0) | 56.18( 44.2) | 46.78( 41.3) | 50.23( 43.2) | 7.9(100) | 1.2( 0) |
| FAMS(86) | 12 | 53.81( 34.7) | 43.78( 28.3) | 47.92( 34.5) | 8.2( 99) | 1.2( 0) | 56.07( 39.6) | 46.59( 37.9) | 50.27( 40.6) | 7.5( 99) | 1.1( 0) |
| HHpred1(86) | 13 | 53.79( 29.4) | 44.27( 30.8) | 47.79( 29.5) | 13.5( 99) | 7.0( 0) | 53.79( 12.7) | 44.27( 11.5) | 47.79( 11.7) | 13.5( 99) | 7.0( 0) |
| BayesHH(86) | 14 | 53.77( 36.3) | 43.90( 32.2) | 47.94( 36.9) | 10.2(100) | 3.3( 0) | 53.77( 17.1) | 43.90( 11.2) | 47.94( 17.2) | 10.2(100) | 3.3( 0) |
| HHpred3(86) | 15 | 53.73( 35.7) | 44.29( 35.6) | 48.07( 37.7) | 10.9( 98) | 3.6( 0) | 53.73( 17.6) | 44.29( 15.4) | 48.07( 19.6) | 10.9( 98) | 3.8( 0) |
| RAPTOR(86) | 16 | 53.42( 34.8) | 42.91( 26.3) | 47.23( 31.7) | 8.0(100) | 0.9( 0) | 56.51( 47.9) | 46.25( 40.0) | 50.19( 44.5) | 8.1(100) | 1.6( 0) |
| SP4(86) | 17 | 53.37( 31.5) | 43.18( 27.2) | 47.20( 30.8) | 8.3( 99) | 2.2( 0) | 55.92( 42.8) | 46.02( 36.4) | 49.90( 42.3) | 8.4(100) | 1.9( 0) |
| SP3(86) | 18 | 53.37( 31.8) | 43.42( 27.9) | 47.40( 32.3) | 9.9( 99) | 2.6( 0) | 55.30( 38.0) | 45.62( 30.8) | 49.29( 34.2) | 9.1(100) | 1.9( 0) |
| shub(86) | 19 | 53.35( 30.0) | 43.39( 27.4) | 46.93( 25.7) | 7.5( 95) | 1.0( 1) | 53.35( 12.6) | 43.39( 7.3) | 46.93( 6.6) | 7.5( 96) | 1.0( 1) |
| RAPTORESS(86) | 20 | 53.11( 32.4) | 42.45( 24.0) | 46.50( 28.6) | 8.0(100) | 0.8( 0) | 56.18( 44.2) | 45.78( 35.7) | 48.81( 33.1) | 7.5(100) | 0.9( 0) |
| FUNCTION(86) | 21 | 52.72( 26.2) | 42.68( 23.3) | 46.66( 23.7) | 8.2( 98) | 1.3( 0) | 54.55( 27.0) | 44.83( 22.6) | 48.63( 26.4) | 8.6( 99) | 1.8( 0) |
| SPARKS2(86) | 22 | 52.40( 22.2) | 42.60( 18.5) | 46.26( 20.0) | 10.2( 98) | 2.8( 0) | 54.89( 31.2) | 45.41( 27.1) | 48.96( 28.6) | 8.5(100) | 1.7( 0) |
| UNI-EID_bnmx(86) | 23 | 52.37( 21.5) | 43.73( 30.5) | 46.95( 26.3) | 7.1( 89) | 1.2( 0) | 54.94( 22.4) | 46.29( 33.5) | 49.02( 28.4) | 6.6( 90) | 1.0( 1) |
| beautshotbase(86) | 24 | 52.29( 23.4) | 42.93( 23.9) | 46.51( 22.6) | 7.0( 92) | 1.1( 0) | 52.29( 4.7) | 42.93( 3.3) | 46.51( 3.1) | 7.0( 92) | 1.1( 0) |
| SAM_T08_server(86) | 25 | 51.91( 26.4) | 41.19( 17.4) | 45.64( 22.6) | 8.2(100) | 1.0( 0) | 54.20( 25.0) | 45.48( 31.4) | 48.71( 29.6) | 6.6( 92) | 0.8( 0) |
| 3Dpro(86) | 26 | 51.88( 25.1) | 42.34( 19.8) | 46.45( 26.5) | 8.9(100) | 1.3( 0) | 53.06( 19.1) | 43.76( 14.7) | 47.63( 20.9) | 8.6(100) | 1.1( 0) |
| FOLDpro(86) | 27 | 51.87( 19.1) | 42.28( 16.0) | 46.25( 19.3) | 9.0(100) | 1.4( 0) | 53.08( 15.0) | 43.76( 10.1) | 47.50( 13.9) | 8.9(100) | 1.3( 0) |
| GeneSilicoMetaServer(84) | 28 | 51.52( 27.5) | 41.98( 26.0) | 45.79( 28.5) | 7.9( 95) | 1.1( 0) | 53.65( 28.3) | 44.38( 26.0) | 47.78( 28.1) | 9.9( 98) | 3.3( 0) |
| PROTINFO(86) | 29 | 51.46( 20.8) | 41.65( 16.7) | 45.83( 21.4) | 7.8( 95) | 0.9( 0) | 54.29( 28.9) | 44.42( 22.4) | 48.30( 26.6) | 7.5( 97) | 1.0( 0) |
| Ma-OPUS-server(86) | 30 | 51.36( 15.4) | 41.15( 8.3) | 45.44( 13.5) | 8.4(100) | 0.9( 0) | 54.84( 31.3) | 44.96( 24.0) | 48.71( 27.5) | 7.7(100) | 0.9( 0) |
| PROTINFO-AB(85) | 31 | 50.95( 21.7) | 40.89( 15.0) | 44.88( 19.1) | 8.6( 99) | 1.2( 0) | 52.30( 17.5) | 42.31( 8.8) | 46.20( 14.5) | 8.0( 99) | 1.0( 0) |
| UNI-EID_sfst(86) | 32 | 50.38( 2.8) | 42.44( 16.6) | 45.33( 7.6) | 6.1( 81) | 0.9( 0) | 52.57( 6.6) | 45.17( 22.9) | 47.68( 12.8) | 5.5( 82) | 0.9( 0) |
| Phyre-2(84) | 33 | 50.27( 15.1) | 40.40( 7.8) | 44.37( 12.8) | 8.8( 98) | 1.7( 0) | 51.40( 9.1) | 41.65( 1.4) | 45.39( 5.5) | 8.7( 98) | 1.4( 0) |
| ROKKY(85) | 34 | 49.67( 18.1) | 40.63( 18.7) | 44.31( 18.1) | 10.3(100) | 2.3( 3) | 52.78( 27.1) | 44.10( 28.5) | 47.39( 27.5) | 9.0(100) | 1.6( 4) |
| Bilab-ENABLE(85) | 35 | 49.58( 6.5) | 39.08( -0.1) | 43.27( 3.4) | 8.8(100) | 2.5( 0) | 52.40( 9.8) | 42.25( 3.5) | 46.11( 6.7) | 8.1(100) | 1.0( 0) |

# FIG.67B

| | | | | | | | | | | | |
|---|---|---|---|---|---|---|---|---|---|---|---|
| mGen-3D(88) | 38 | 49.42( 3.7) | 40.25( 8.8) | 44.29( 9.6) | 7.2( 86) | 1.0( 0) | 49.42( -17.5) | 40.25( -12.9) | 44.29( -11.2) | 7.2( 86) | 1.0( 0) |
| LOOPP(86) | 37 | 48.72( 3.5) | 39.03( 2.3) | 43.03( 2.2) | 8.3( 94) | 0.9( 0) | 52.21( 14.9) | 42.45( 10.9) | 46.40( 15.8) | 7.0( 94) | 0.7( 0) |
| FUGUE(86) | 38 | 48.02( -18.2) | 39.14( -7.8) | 42.90( -8.2) | 8.2( 91) | 1.4( 0) | 51.30( -4.2) | 42.18( -0.7) | 45.71( -2.7) | 7.7( 92) | 1.1( 0) |
| FUGMOD(83) | 39 | 48.01( -1.1) | 38.53( -2.3) | 42.40( -1.0) | 8.5( 96) | 1.3( 0) | 51.54( 13.8) | 41.84( 8.1) | 45.32( 8.9) | 8.0( 98) | 1.0( 0) |
| SAM-T02(86) | 40 | 48.01( -13.4) | 39.86( -0.7) | 42.86( -10.2) | 6.0( 77) | 1.0( 0) | 51.07( -8.5) | 43.38( 8.9) | 45.97( -3.8) | 6.6( 78) | 0.8( 0) |
| nFOLD(88) | 41 | 47.75( -18.5) | 38.82( -14.0) | 42.28( -17.0) | 8.1( 90) | 1.1( 0) | 51.44( -0.5) | 42.42( 3.2) | 45.99( 2.7) | 7.5( 92) | 1.0( 0) |
| keasar-server(83) | 42 | 47.66( 13.1) | 38.10( 8.7) | 41.42( 5.3) | 9.1( 96) | 1.5( 0) | 51.76( 22.7) | 42.57( 18.8) | 45.75( 18.0) | 7.9( 97) | 1.0( 0) |
| NN_PUT_lab(82) | 43 | 46.98( -1.7) | 37.98( 1.0) | 41.41( -2.3) | 8.4( 84) | 1.3( 0) | 46.98( -23.2) | 37.98( -18.9) | 41.41( -23.6) | 8.4( 84) | 1.3( 0) |
| Phyre-1(88) | 44 | 46.91( -19.6) | 38.99( -9.1) | 41.84( -18.8) | 8.9( 79) | 0.8( 0) | 46.91( -43.8) | 38.99( -31.7) | 41.84( -40.4) | 8.9( 79) | 0.8( 0) |
| Casplta-FOX(86) | 45 | 46.37( -18.9) | 37.52( -13.6) | 41.01( -18.9) | 8.6( 88) | 1.5( 3) | 52.12( 9.4) | 43.15( 11.9) | 46.40( 8.3) | 7.6( 92) | 1.3( 3) |
| FORTE1(88) | 46 | 46.20( -29.5) | 36.82( -24.4) | 40.44( -28.2) | 8.8( 98) | 1.7( 0) | 50.19( -17.2) | 41.18( -12.8) | 44.59( -15.4) | 8.4( 92) | 1.4( 0) |
| FORTE2(86) | 47 | 45.73( -32.6) | 36.57( -25.3) | 40.17( -30.4) | 10.7( 92) | 3.0( 0) | 49.85( -19.4) | 40.78( -13.7) | 44.15( -17.5) | 8.5( 92) | 1.4( 0) |
| 3D-JIGSAW_POPULUS(86) | 48 | 45.33( -25.8) | 35.78( -24.3) | 39.78( -25.6) | 9.2( 82) | 1.8( 0) | 47.17( -33.6) | 37.80( -28.5) | 41.62( -32.2) | 8.6( 93) | 1.6( 0) |
| 3D-JIGSAW_RECON(86) | 49 | 44.34( -35.8) | 36.02( -26.5) | 39.40( -31.5) | 8.1( 86) | 1.1( 0) | 46.78( -38.4) | 36.06( -30.4) | 41.54( -34.2) | 7.6( 89) | 1.2( 0) |
| karypis.srv(85) | 50 | 44.00( -30.5) | 34.69( -28.9) | 38.49( -30.4) | 8.1( 88) | 0.8( 0) | 46.53( -25.9) | 37.51( -22.4) | 40.90( -27.0) | 7.5( 87) | 0.7( 0) |
| 3D-JIGSAW(88) | 51 | 43.77( -40.6) | 34.80( -35.0) | 38.68( -37.9) | 8.5( 88) | 1.2( 0) | 48.45( -21.8) | 38.85( -20.8) | 42.87( -18.6) | 7.7( 81) | 1.4( 0) |
| karypis.srv.2(86) | 52 | 42.84( -37.2) | 33.66( -36.4) | 37.65( -36.2) | 10.4( 92) | 1.3( 0) | 45.61( -32.2) | 36.23( -32.7) | 40.03( -32.1) | 10.7( 92) | 2.6( 0) |
| SAM-T99(66) | 53 | 42.55( -9.1) | 35.70( 0.1) | 37.48( -7.2) | 5.3( 84) | 0.8( 0) | 44.43( -5.1) | 38.00( 6.3) | 39.57( -1.7) | 4.7( 84) | 0.7( 0) |
| Huber-Torda-Server(83) | 54 | 42.04( -42.8) | 34.71( -32.0) | 37.50( -38.1) | 8.4( 81) | 1.1( 1) | 46.58( -34.0) | 38.59( -25.4) | 41.68( -28.5) | 8.0( 87) | 1.0( 0) |
| forecast-s(85) | 55 | 41.20( -58.5) | 33.44( -49.1) | 36.54( -56.5) | 8.8( 81) | 1.6( 0) | 44.41( -54.0) | 36.70( -40.7) | 39.78( -47.5) | 8.3( 84) | 1.3( 0) |
| Distill(86) | 56 | 39.74( -69.8) | 27.22( -80.6) | 31.88( -88.7) | 11.5(100) | 1.8( 2) | 41.19( -89.6) | 29.60( -106.9) | 33.18( -109.3) | 11.1(100) | 1.3( 4) |
| panther2(70) | 57 | 29.14( -79.2) | 23.79( -60.1) | 26.01( -71.5) | 7.9( 69) | 1.6( 17) | 29.14( -108.1) | 23.79( -82.8) | 26.01( -99.1) | 7.9( 69) | 1.6( 17) |
| CPHmodels(42) | 58 | 27.56( -23.4) | 23.31( -13.2) | 24.37( -18.1) | 4.2( 80) | 0.5( 0) | 27.56( -36.3) | 23.31( -22.5) | 24.37( -28.9) | 4.2( 80) | 0.5( 0) |
| Frankenstein(45) | 59 | 22.28( -20.8) | 18.81( -25.8) | 19.32( -29.1) | 9.2( 98) | 1.4( 2) | 25.19( -24.9) | 19.44( -28.5) | 21.78( -28.8) | 10.1( 99) | 2.8( 8) |

FIG.68

http://www.pdc.kth.s...

| | Name | N | Top1 | Top1 rank | Top5 | Top5 rank | Top1 | Top1 rank | Top5 | Top5 rank | Top1 | Top1 rank | Top5 | Top5 rank | Top1 | Top1 rank | Top5 | Top5 rank |
|---|---|---|---|---|---|---|---|---|---|---|---|---|---|---|---|---|---|---|
| 1 | BestPcons-Pmod | 84 | 53.43 | 1.09 | 53.58 | 1.19 | 49.36 | 1.12 | 49.55 | 1.20 | 58.40 | 1.07 | 58.47 | 1.18 | 52.52 | 1.09 | 52.71 | 1.18 |
| 2 | Zhang-Server | 86 | 53.04 | 1.27 | 55.03 | 1.14 | 48.87 | 1.29 | 50.73 | 1.18 | 58.07 | 1.27 | 60.18 | 1.12 | 52.17 | 1.25 | 54.19 | 1.12 |
| 3 | **Pcons-QA** | 86 | 52.23 | 1.38 | 53.83 | 1.28 | 47.95 | 1.43 | 49.49 | 1.32 | 57.11 | 1.36 | 58.86 | 1.25 | 51.62 | 1.33 | 53.14 | 1.26 |
| 4 | **SBC** | 86 | 51.05 | 1.67 | 54.21 | 1.24 | 46.70 | 1.78 | 49.96 | 1.29 | 56.06 | 1.60 | 59.18 | 1.24 | 50.39 | 1.63 | 53.50 | 1.20 |
| 5 | **SBC_no_Zhang** | 86 | 50.55 | 1.79 | 53.46 | 1.37 | 46.20 | 1.93 | 49.23 | 1.42 | 55.58 | 1.71 | 58.35 | 1.38 | 49.86 | 1.73 | 52.78 | 1.31 |
| 6 | **SAMUDRALA** | 86 | 50.23 | 1.62 | 53.19 | 1.41 | 45.46 | 1.71 | 48.73 | 1.44 | 55.29 | 1.61 | 58.23 | 1.39 | 49.95 | 1.54 | 52.61 | 1.40 |
| 7 | **SAMUDRALA-AB2** | 86 | 49.73 | 1.75 | 52.12 | 1.50 | 45.09 | 1.86 | 47.63 | 1.50 | 54.87 | 1.68 | 57.16 | 1.51 | 49.24 | 1.71 | 51.57 | 1.49 |
| 8 | Pmodeller6 | 86 | 49.69 | 2.11 | 52.64 | 1.50 | 45.02 | 2.32 | 48.35 | 1.51 | 54.99 | 1.95 | 57.72 | 1.46 | 49.08 | 2.04 | 51.87 | 1.52 |
| 9 | ProQ1 | 86 | 49.60 | 1.98 | 52.07 | 1.57 | 44.94 | 2.15 | 47.85 | 1.58 | 54.88 | 1.89 | 57.04 | 1.54 | 48.98 | 1.91 | 51.33 | 1.60 |
| 10 | **SAMUDRALA-AB** | 86 | 49.47 | 1.84 | 51.89 | 1.56 | 44.73 | 1.98 | 47.31 | 1.58 | 54.70 | 1.74 | 57.01 | 1.55 | 48.97 | 1.79 | 51.34 | 1.56 |
| 11 | Pcons6 | 86 | 49.36 | 2.12 | 51.23 | 1.78 | 45.07 | 2.15 | 46.93 | 1.81 | 54.48 | 2.03 | 56.30 | 1.75 | 48.53 | 2.18 | 50.47 | 1.78 |
| 12 | CIRCLE | 86 | 49.35 | 1.95 | 51.22 | 1.79 | 44.78 | 2.12 | 46.85 | 1.91 | 54.58 | 1.80 | 56.27 | 1.69 | 48.69 | 1.95 | 50.52 | 1.79 |
| 13 | ROBETTA | 86 | 49.34 | 1.99 | 52.48 | 1.53 | 44.55 | 2.20 | 48.02 | 1.62 | 54.62 | 1.89 | 57.81 | 1.45 | 48.86 | 1.87 | 51.61 | 1.53 |
| 14 | HHpred2 | 86 | 49.30 | 1.88 | 49.30 | 2.11 | 44.87 | 1.98 | 44.87 | 2.28 | 54.28 | 1.84 | 54.28 | 2.07 | 48.76 | 1.81 | 48.76 | 1.99 |
| 15 | **SAMUDRALA-QA** | 86 | 48.96 | 2.23 | 52.72 | 1.46 | 44.44 | 2.28 | 48.34 | 1.47 | 54.25 | 2.07 | 57.83 | 1.46 | 48.19 | 2.33 | 52.01 | 1.46 |
| 16 | UNI-EID_expm | 86 | 48.87 | 2.08 | 48.87 | 2.33 | 44.59 | 2.06 | 44.59 | 2.37 | 53.99 | 1.91 | 53.99 | 2.10 | 48.04 | 2.20 | 48.04 | 2.52 |
| 17 | FAMSD | 86 | 48.80 | 2.12 | 50.37 | 2.01 | 44.22 | 2.29 | 46.02 | 2.12 | 54.02 | 1.93 | 55.54 | 1.84 | 48.16 | 2.15 | 49.56 | 2.06 |
| 18 | beautshot | 86 | 48.79 | 2.08 | 48.79 | 2.42 | 44.33 | 2.28 | 44.33 | 2.75 | 54.24 | 1.80 | 54.24 | 2.07 | 47.81 | 2.08 | 47.81 | 2.44 |
| 19 | HHpred3 | 86 | 48.74 | 2.01 | 48.74 | 2.25 | 44.39 | 2.15 | 44.39 | 2.46 | 53.68 | 1.99 | 53.68 | 2.20 | 48.16 | 1.88 | 48.16 | 2.11 |
| 20 | *ProQ* | 86 | 48.73 | 2.19 | 52.84 | 1.49 | 44.05 | 2.46 | 48.35 | 1.57 | 54.08 | 2.01 | 58.02 | 1.42 | 48.06 | 2.09 | 52.14 | 1.48 |
| 21 | MetaTasser | 86 | 48.65 | 1.90 | 50.40 | 1.79 | 43.39 | 2.12 | 45.54 | 1.96 | 54.79 | 1.68 | 56.31 | 1.58 | 47.77 | 1.90 | 49.35 | 1.84 |
| 22 | HHpred1 | 86 | 48.60 | 2.15 | 48.60 | 2.48 | 44.29 | 2.32 | 44.29 | 2.73 | 53.66 | 2.03 | 53.66 | 2.27 | 47.84 | 2.10 | 47.84 | 2.44 |
| 23 | BayesHH | 86 | 48.57 | 1.99 | 48.57 | 2.22 | 43.99 | 2.10 | 43.99 | 2.47 | 53.69 | 1.93 | 53.69 | 2.07 | 48.02 | 1.93 | 48.02 | 2.13 |

FIG.69

http://www.pdc.kth.s...

| | EASY | | (MaxSub+TM-score+GDT_TS)/3 | | | | MaxSub | | | | TM-score | | | | GDT_TS | | | |
|---|---|---|---|---|---|---|---|---|---|---|---|---|---|---|---|---|---|---|
| | Name | N | Top1 | Top1 rank | Top5 | Top5 rank | Top1 | Top1 rank | Top5 | Top5 rank | Top1 | Top1 rank | Top5 | Top5 rank | Top1 | Top1 rank | Top5 | Top5 rank |
| 1 | Zhang-Server | 47 | 36.75 | 1.14 | 37.27 | 1.06 | 34.86 | 1.16 | 35.43 | 1.11 | 39.60 | 1.10 | 40.05 | 1.01 | 35.78 | 1.15 | 36.32 | 1.05 |
| 2 | Pcons-QA | 47 | 36.53 | 1.18 | 37.11 | 1.09 | 34.52 | 1.27 | 35.22 | 1.15 | 39.35 | 1.09 | 39.89 | 1.03 | 35.73 | 1.16 | 36.24 | 1.10 |
| 3 | SAMUDRALA | 47 | 35.89 | 1.24 | 36.71 | 1.16 | 33.70 | 1.30 | 34.67 | 1.20 | 38.84 | 1.19 | 39.57 | 1.10 | 35.11 | 1.22 | 35.88 | 1.17 |
| 4 | SAMUDRALA-AB2 | 47 | 35.77 | 1.33 | 36.55 | 1.14 | 33.57 | 1.41 | 34.53 | 1.17 | 38.81 | 1.24 | 39.42 | 1.11 | 34.93 | 1.33 | 35.70 | 1.14 |
| 5 | SAMUDRALA-AB | 47 | 35.68 | 1.38 | 36.54 | 1.18 | 33.46 | 1.49 | 34.51 | 1.20 | 38.76 | 1.27 | 39.42 | 1.14 | 34.84 | 1.37 | 35.69 | 1.18 |
| 6 | BestPcons-Pmod | 45 | 35.57 | 1.13 | 35.61 | 1.15 | 33.82 | 1.18 | 33.86 | 1.24 | 38.23 | 1.06 | 38.26 | 1.06 | 34.65 | 1.15 | 34.71 | 1.15 |
| 7 | UNI-EID_expm | 47 | 35.35 | 1.45 | 35.35 | 1.56 | 33.13 | 1.58 | 33.13 | 1.73 | 38.47 | 1.24 | 38.47 | 1.29 | 34.45 | 1.53 | 34.45 | 1.66 |
| 8 | SBC | 47 | 35.28 | 1.67 | 37.02 | 1.11 | 33.22 | 1.95 | 35.22 | 1.19 | 38.23 | 1.41 | 39.67 | 1.07 | 34.39 | 1.65 | 36.17 | 1.07 |
| 9 | beautshot | 47 | 35.26 | 1.62 | 35.26 | 1.85 | 33.08 | 1.84 | 33.08 | 2.20 | 38.49 | 1.31 | 38.49 | 1.44 | 34.22 | 1.70 | 34.22 | 1.92 |
| 10 | shub | 47 | 35.21 | 1.61 | 35.21 | 1.80 | 33.02 | 1.85 | 33.02 | 2.00 | 38.44 | 1.31 | 38.44 | 1.40 | 34.17 | 1.68 | 34.17 | 2.00 |
| 11 | SBC_no_Zhang | 47 | 35.21 | 1.72 | 36.73 | 1.16 | 33.16 | 2.01 | 34.94 | 1.24 | 38.18 | 1.43 | 39.44 | 1.12 | 34.30 | 1.71 | 35.82 | 1.12 |
| 12 | CIRCLE | 47 | 35.17 | 1.65 | 35.99 | 1.37 | 32.90 | 1.85 | 33.96 | 1.49 | 38.29 | 1.42 | 38.92 | 1.21 | 34.33 | 1.68 | 35.08 | 1.40 |
| 13 | FAMSD | 47 | 35.12 | 1.66 | 35.69 | 1.48 | 32.85 | 1.89 | 33.59 | 1.68 | 38.26 | 1.42 | 38.72 | 1.24 | 34.23 | 1.68 | 34.76 | 1.51 |
| 14 | FAMS | 47 | 35.00 | 1.76 | 36.13 | 1.29 | 32.70 | 1.95 | 34.14 | 1.37 | 38.10 | 1.49 | 39.02 | 1.19 | 34.19 | 1.85 | 35.24 | 1.32 |
| 15 | FOLDpro | 47 | 34.96 | 1.69 | 35.70 | 1.45 | 32.65 | 1.93 | 33.56 | 1.60 | 38.04 | 1.56 | 38.62 | 1.37 | 34.20 | 1.59 | 34.93 | 1.38 |
| 16 | SP3 | 47 | 34.90 | 1.82 | 35.67 | 1.58 | 32.65 | 2.25 | 33.59 | 1.77 | 38.07 | 1.40 | 38.67 | 1.28 | 34.00 | 1.81 | 34.75 | 1.68 |
| 17 | SAMUDRALA-QA | 47 | 34.90 | 1.76 | 36.60 | 1.17 | 32.71 | 1.89 | 34.63 | 1.19 | 38.05 | 1.52 | 39.46 | 1.12 | 33.94 | 1.88 | 35.70 | 1.19 |
| 18 | ProQ1 | 47 | 34.89 | 1.73 | 36.08 | 1.32 | 32.59 | 2.06 | 34.13 | 1.38 | 38.03 | 1.44 | 38.95 | 1.18 | 34.07 | 1.68 | 35.17 | 1.40 |
| 19 | RAPTOR | 47 | 34.88 | 1.85 | 35.66 | 1.57 | 32.53 | 2.13 | 33.45 | 1.69 | 38.10 | 1.50 | 38.73 | 1.32 | 34.02 | 1.92 | 34.80 | 1.69 |
| 20 | 3Dpro | 47 | 34.85 | 1.85 | 35.38 | 1.63 | 32.58 | 2.15 | 33.18 | 1.74 | 37.92 | 1.54 | 38.34 | 1.50 | 34.05 | 1.86 | 34.61 | 1.64 |
| 21 | HHpred3 | 47 | 34.75 | 1.58 | 34.75 | 1.71 | 32.50 | 1.76 | 32.50 | 1.96 | 37.72 | 1.48 | 37.72 | 1.56 | 34.03 | 1.49 | 34.03 | 1.62 |

FIG.70

http://www.pdc.kth.s..

Click the top links to re-sort the table according to a particular score.

ALL (86) EASY (47) HARD (39)

| | HARD | | (MaxSub+TM-score+GDT_TS)/3 | | | | MaxSub | | | | TM-score | | | | GDT_TS | | | |
|---|---|---|---|---|---|---|---|---|---|---|---|---|---|---|---|---|---|---|
| | Name | N | Top1 | Top1 rank | Top5 | Top5 rank | Top1 | Top1 rank | Top5 | Top5 rank | Top1 | Top1 rank | Top5 | Top5 rank | Top1 | Top1 rank | Top5 | Top5 rank |
| 1 | BestPcons-Pmod | 39 | 17.86 | 1.06 | 17.97 | 1.25 | 15.54 | 1.06 | 15.69 | 1.16 | 20.17 | 1.09 | 20.21 | 1.37 | 17.87 | 1.03 | 18.00 | 1.22 |
| 2 | Zhang-Server | 39 | 16.29 | 1.47 | 17.77 | 1.26 | 14.01 | 1.48 | 15.31 | 1.28 | 18.47 | 1.55 | 20.13 | 1.28 | 16.39 | 1.39 | 17.87 | 1.23 |
| 3 | **SBC** | 39 | 15.77 | 1.71 | 17.19 | 1.45 | 13.48 | 1.62 | 14.74 | 1.42 | 17.83 | 1.91 | 19.51 | 1.54 | 15.99 | 1.61 | 17.33 | 1.41 |
| 4 | **Pcons-QA** | 39 | 15.69 | 1.75 | 16.72 | 1.61 | 13.43 | 1.69 | 14.27 | 1.61 | 17.77 | 1.95 | 18.98 | 1.70 | 15.89 | 1.61 | 16.90 | 1.52 |
| 5 | **SBC_no_Zhang** | 39 | 15.33 | 1.93 | 16.72 | 1.76 | 13.04 | 1.84 | 14.30 | 1.73 | 17.39 | 2.23 | 18.91 | 1.93 | 15.57 | 1.74 | 16.96 | 1.63 |
| 6 | Pmodeller6 | 39 | 15.23 | 2.28 | 16.55 | 1.85 | 12.81 | 2.11 | 14.26 | 1.65 | 17.33 | 2.66 | 18.65 | 2.22 | 15.54 | 2.07 | 16.74 | 1.68 |
| 7 | ROBETTA | 39 | 14.84 | 2.28 | 16.95 | 1.62 | 12.33 | 2.44 | 14.60 | 1.61 | 17.02 | 2.54 | 19.28 | 1.70 | 15.18 | 1.88 | 16.99 | 1.54 |
| 8 | ProQ1 | 39 | 14.71 | 2.52 | 15.99 | 2.07 | 12.35 | 2.26 | 13.72 | 1.91 | 16.85 | 3.02 | 18.08 | 2.40 | 14.92 | 2.28 | 16.15 | 1.92 |
| 9 | Pcons6 | 39 | 14.66 | 2.41 | 15.68 | 2.26 | 12.54 | 2.22 | 13.42 | 2.11 | 16.69 | 2.60 | 17.77 | 2.57 | 14.76 | 2.40 | 15.84 | 2.11 |
| 10 | *ProQ* | 39 | 14.65 | 2.35 | 17.11 | 1.65 | 12.23 | 2.25 | 14.64 | 1.66 | 16.81 | 2.71 | 19.31 | 1.84 | 14.90 | 2.08 | 17.38 | 1.47 |
| 11 | HHpred2 | 39 | 14.56 | 2.61 | 14.56 | 3.36 | 12.40 | 2.44 | 12.40 | 3.07 | 16.57 | 2.85 | 16.57 | 3.91 | 14.71 | 2.55 | 14.71 | 3.08 |
| 12 | MetaTasser | 39 | 14.55 | 2.40 | 15.48 | 2.17 | 11.97 | 2.64 | 12.97 | 2.30 | 16.84 | 2.39 | 17.86 | 2.11 | 14.83 | 2.18 | 15.61 | 2.08 |
| 13 | **ProQ-QA** | 39 | 14.47 | 2.25 | 17.11 | 1.52 | 12.45 | 2.06 | 14.70 | 1.41 | 16.18 | 2.56 | 19.41 | 1.60 | 14.78 | 2.12 | 17.20 | 1.53 |
| 14 | **SAMUDRALA** | 39 | 14.35 | 2.58 | 16.49 | 1.91 | 11.75 | 2.76 | 14.07 | 1.88 | 16.44 | 2.75 | 18.66 | 2.03 | 14.84 | 2.23 | 16.73 | 1.82 |
| 15 | **Pmod6_wo_rob** | 39 | 14.30 | 3.09 | 15.33 | 2.66 | 12.08 | 2.64 | 13.02 | 2.27 | 16.27 | 3.71 | 17.40 | 3.20 | 14.54 | 2.94 | 15.56 | 2.51 |
| 16 | **ProQlocal-QA** | 39 | 14.29 | 2.39 | 16.94 | 1.56 | 12.29 | 2.25 | 14.55 | 1.46 | 15.97 | 2.65 | 19.21 | 1.64 | 14.61 | 2.27 | 17.05 | 1.59 |
| 17 | CIRCLE | 39 | 14.18 | 2.53 | 15.23 | 2.93 | 11.88 | 2.57 | 12.89 | 2.88 | 16.29 | 2.64 | 17.35 | 3.24 | 14.37 | 2.40 | 15.45 | 2.66 |
| 18 | **SAMUDRALA-QA** | 39 | 14.06 | 3.33 | 16.13 | 2.10 | 11.73 | 3.04 | 13.70 | 2.03 | 16.20 | 3.64 | 18.37 | 2.30 | 14.24 | 3.31 | 16.31 | 1.98 |
| 19 | *Pmod-ProQaL1478* | 39 | 14.03 | 3.47 | 15.31 | 3.04 | 11.81 | 3.11 | 13.17 | 2.56 | 15.98 | 4.10 | 17.24 | 3.71 | 14.30 | 3.20 | 15.52 | 2.86 |
| 20 | HHpred3 | 39 | 14.00 | 3.02 | 14.00 | 3.74 | 11.89 | 2.93 | 11.89 | 3.54 | 15.97 | 3.43 | 15.97 | 4.38 | 14.13 | 2.71 | 14.13 | 3.31 |

FIG.71A

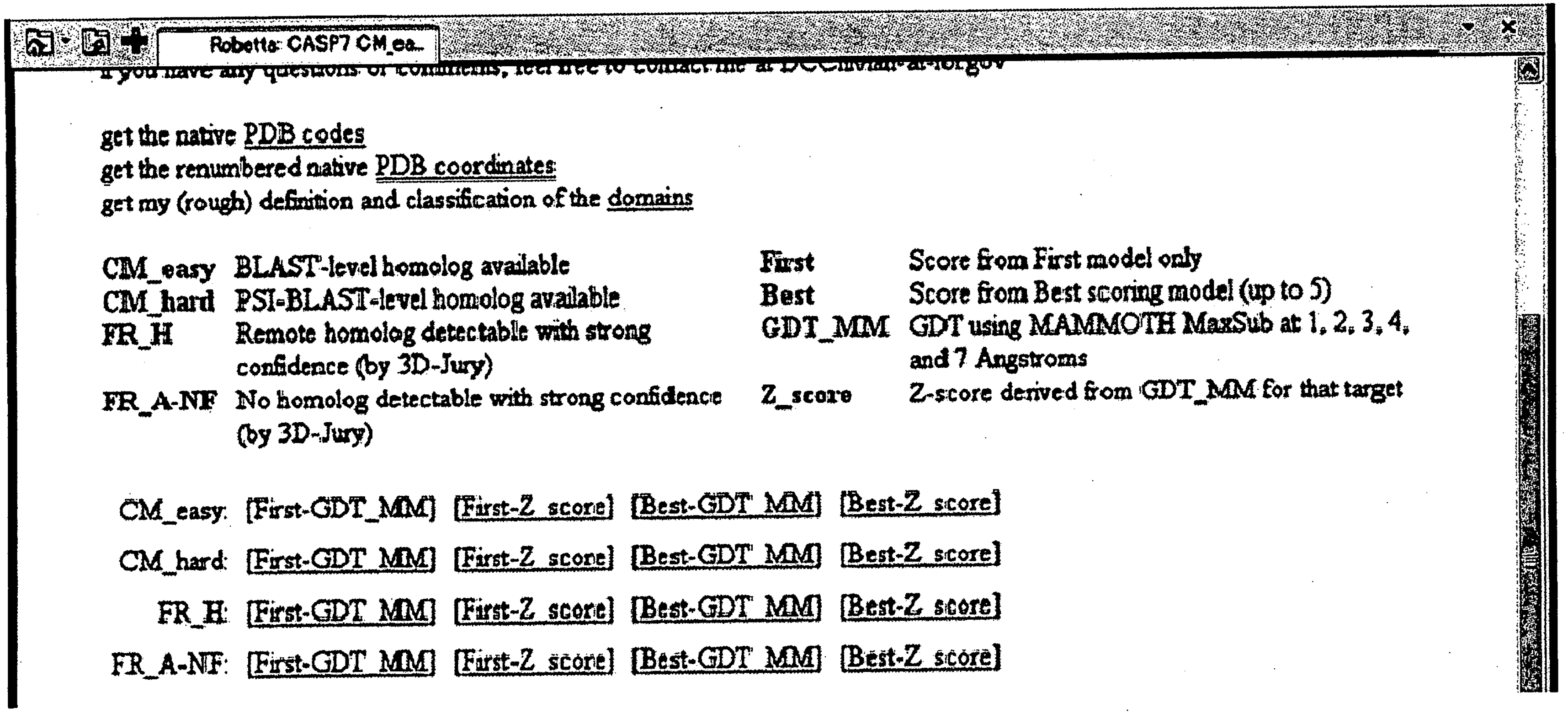
Robetta: CASP7 CM_ea..
get the native PDB codes
get the renumbered native PDB coordinates
get my (rough) definition and classification of the domains
CM_easy BLAST-level homolog available
CM_hard PSI-BLAST-level homolog available
FR_H Remote homolog detectable with strong confidence (by 3D-Jury)
FR_A-NF No homolog detectable with strong confidence (by 3D-Jury)
First Score from First model only
Best Score from Best scoring model (up to 5)
GDT_MM GDT using MAMMOTH MaxSub at 1, 2, 3, 4, and 7 Angstroms
Z_score Z-score derived from GDT_MM for that target
CM_easy: [First-GDT_MM] [First-Z score] [Best-GDT MM] [Best-Z score]
CM_hard: [First-GDT MM] [First-Z score] [Best-GDT MM] [Best-Z score]
FR_H: [First-GDT MM] [First-Z score] [Best-GDT MM] [Best-Z score]
FR_A-NF: [First-GDT MM] [First-Z score] [Best-GDT MM] [Best-Z score]

FIG.71B

| GROUP | SUM ↑ | 288 | 290 | 291_1 | 291_2 | 292_1 | 292_2 | 294_1 | 294_2 | 295_1 | 295_2 | 297 | 302 | 303_1 | 303_2 | 305 |
|---|---|---|---|---|---|---|---|---|---|---|---|---|---|---|---|---|
| Zhang-Server | 4282.75 | 83.30 | 98.15 | 96.16 | 93.08 | 92.54 | 76.65 | 81.26 | 86.70 | 89.49 | 88.73 | 67.11 | 83.33 | 87.03 | 73.95 | 93.3 |
| UNI-EID_expm | 4120.86 | 80.88 | 96.88 | 73.33 | 84.18 | 89.15 | 68.90 | 75.59 | 84.23 | 89.27 | 90.64 | 67.77 | 81.97 | 84.46 | 68.95 | 91.2 |
| FAMSD | 4116.59 | 78.24 | 98.84 | 94.75 | 91.32 | 85.76 | 69.53 | 79.06 | 79.28 | 88.93 | 89.15 | 65.40 | 77.73 | 83.65 | 68.95 | 91.7 |
| FAMS | 4114.07 | 74.95 | 93.06 | 94.14 | 90.66 | 87.80 | 68.17 | 78.58 | 87.32 | 88.70 | 77.66 | 58.96 | 77.88 | 82.97 | 65.26 | 93.3 |
| 3Dpro | 4109.95 | 80.44 | 97.23 | 86.26 | 91.76 | 89.83 | 70.47 | 85.35 | 86.29 | 88.48 | 87.23 | 60.66 | 79.85 | 85.54 | 72.63 | 93.5 |
| FOLDpro | 4101.48 | 80.44 | 97.11 | 84.85 | 91.32 | 90.85 | 70.68 | 83.47 | 86.90 | 88.48 | 87.23 | 62.18 | 80.45 | 82.97 | 72.89 | 93.0 |
| CIRCLE | 4082.57 | 78.68 | 97.92 | 93.54 | 91.98 | 90.17 | 73.09 | 78.58 | 87.32 | 89.61 | 89.79 | 61.14 | 78.94 | 83.65 | 68.95 | 91.5 |
| SP3 | 4042.69 | 80.00 | 92.95 | 56.77 | 81.87 | 89.83 | 73.20 | 79.37 | 87.94 | 89.38 | 88.72 | 66.63 | 83.18 | 82.97 | 64.74 | 91.7 |
| BayesHH | 4036.31 | 71.65 | 98.04 | 66.26 | 84.84 | 89.15 | 70.26 | 79.69 | 71.75 | 89.83 | 89.58 | 65.21 | 78.03 | 84.19 | 66.05 | 93.0 |
| HHpred1 | 4032.78 | 79.12 | 96.88 | 93.74 | 92.64 | 92.88 | 71.41 | 77.95 | 84.02 | 89.49 | 88.72 | 63.79 | 82.88 | 82.84 | 63.95 | 90.1 |
| beautshotbase | 4032.38 | 76.92 | 97.46 | 92.32 | 91.76 | 87.46 | 71.62 | 80.47 | 88.15 | 88.82 | 89.15 | 67.20 | 79.85 | 82.03 | 66.58 | 89.0 |
| HHpred2 | 4032.18 | 71.21 | 96.76 | 63.64 | 84.84 | 86.78 | 71.94 | 79.69 | 71.85 | 89.61 | 90.64 | 64.74 | 79.09 | 83.78 | 70.00 | 92.9 |
| HHpred3 | 4029.87 | 71.21 | 96.76 | 63.64 | 84.84 | 86.78 | 71.94 | 79.69 | 71.85 | 89.61 | 90.64 | 65.12 | 79.09 | 83.78 | 70.00 | 93.5 |
| UNI-EID_bnmx | 4013.21 | 79.78 | 96.88 | 92.93 | 90.77 | 83.05 | 56.02 | 75.59 | 84.02 | 89.27 | 89.58 | 66.54 | 68.03 | 82.97 | 64.47 | 90.1 |
| Pmodeller6 | 4008.16 | 74.07 | 93.06 | 76.36 | 85.49 | 84.75 | 70.37 | 81.10 | 76.29 | 89.16 | 87.23 | 60.09 | 73.48 | 82.97 | 69.47 | 87.6 |
| beautshot | 4003.60 | 78.02 | 97.34 | 89.70 | 91.32 | 85.43 | 73.82 | 73.54 | 84.23 | 85.09 | 86.38 | 65.88 | 81.82 | 84.32 | 69.21 | 91.2 |
| SP4 | 4001.41 | 79.78 | 92.95 | 56.77 | 81.87 | 86.44 | 67.33 | 79.21 | 87.22 | 89.38 | 88.72 | 65.12 | 83.18 | 82.97 | 64.74 | 90.5 |
| SPARKS2 | 4000.09 | 80.88 | 92.37 | 56.77 | 82.09 | 92.54 | 63.25 | 81.10 | 87.01 | 89.38 | 88.72 | 67.39 | 83.18 | 76.08 | 47.10 | 91.7 |
| RAPTOR | 3998.79 | 78.46 | 97.69 | 92.73 | 92.09 | 86.44 | 73.40 | 66.46 | 77.73 | 89.27 | 84.47 | 65.78 | 77.12 | 75.13 | 57.63 | 91.2 |
| ROBETTA | 3989.03 | 80.22 | 97.22 | 94.75 | 90.22 | 88.48 | 70.89 | 73.39 | 76.19 | 89.04 | 84.04 | 67.39 | 71.97 | 76.49 | 55.00 | 90.2 |

# FIG.72A

Robetta: CASP7 CM_ha...

| GROUP | SUM ↑ | 289_1 | 289_2 | 293 | 298_1 | 298_2 | 311 | 316_1 | 322 | 330_1 | 330_2 | 331 | 338_1 | 338_2 | 342 | 357 | 360 | 364 | 36: |
|---|---|---|---|---|---|---|---|---|---|---|---|---|---|---|---|---|---|---|---|
| Zhang-Server | 1722.80 | 49.44 | 26.76 | 51.38 | 70.27 | 82.04 | 59.31 | 51.50 | 71.69 | 69.38 | 73.54 | 59.57 | 72.45 | 64.96 | 51.60 | 54.09 | 53.79 | 69.28 | 56. |
| HHpred3 | 1647.65 | 48.24 | 35.68 | 47.02 | 70.95 | 83.22 | 60.92 | 51.40 | 65.49 | 68.63 | 64.31 | 65.18 | 67.97 | 57.17 | 52.66 | 46.06 | 51.55 | 68.63 | 53. |
| MetaTasser | 1645.34 | 35.45 | 28.92 | 45.16 | 57.97 | 58.50 | 51.03 | 54.90 | 65.49 | 72.88 | 72.92 | 65.76 | 60.28 | 60.71 | 51.60 | 56.67 | 52.41 | 71.24 | 57. |
| HHpred2 | 1643.58 | 50.21 | 32.43 | 49.07 | 70.95 | 83.22 | 60.92 | 51.40 | 65.49 | 68.88 | 60.61 | 65.18 | 68.25 | 52.39 | 53.38 | 46.06 | 51.55 | 68.63 | 52. |
| BayesHH | 1634.07 | 51.16 | 44.86 | 49.16 | 73.11 | 75.80 | 40.23 | 50.20 | 67.32 | 68.63 | 64.31 | 65.18 | 71.19 | 53.98 | 52.31 | 43.79 | 51.21 | 71.63 | 55. |
| RAPTOR-ACE | 1614.97 | 45.24 | 37.84 | 46.22 | 67.97 | 80.00 | 60.69 | 48.30 | 65.07 | 67.88 | 63.69 | 60.72 | 61.68 | 60.89 | 52.19 | 50.76 | 53.28 | 68.50 | 48. |
| Pcons6 | 1592.14 | 48.33 | 26.76 | 46.84 | 63.11 | 73.76 | 58.39 | 41.00 | 65.50 | 68.00 | 63.69 | 57.56 | 64.62 | 61.24 | 50.06 | 45.60 | 53.79 | 67.06 | 51. |
| SP3 | 1591.52 | 44.20 | 32.97 | 45.24 | 67.97 | 80.00 | 57.01 | 39.30 | 64.09 | 68.50 | 63.39 | 60.72 | 67.13 | 55.93 | 51.36 | 53.79 | 40.86 | 66.41 | 54. |
| HHpred1 | 1583.38 | 48.24 | 30.81 | 50.04 | 65.40 | 78.49 | 59.54 | 52.20 | 66.76 | 62.50 | 56.31 | 61.87 | 67.13 | 56.64 | 53.38 | 45.30 | 51.55 | 59.74 | 52. |
| CIRCLE | 1581.90 | 45.58 | 37.03 | 48.62 | 68.24 | 79.57 | 56.09 | 44.70 | 62.54 | 70.00 | 63.38 | 58.27 | 67.27 | 55.22 | 51.13 | 53.48 | 53.62 | 63.92 | 48. |
| SP4 | 1580.27 | 47.98 | 38.65 | 47.46 | 68.51 | 78.71 | 58.16 | 39.00 | 63.94 | 68.62 | 57.85 | 61.73 | 69.65 | 54.34 | 47.58 | 47.73 | 40.86 | 65.75 | 52. |
| Pmodeller6 | 1574.79 | 45.75 | 5.95 | 46.31 | 63.51 | 73.44 | 61.84 | 42.40 | 64.08 | 69.25 | 71.08 | 60.00 | 60.42 | 61.06 | 47.34 | 53.18 | 52.76 | 65.10 | 46. |
| UNI-EID_expm | 1569.49 | 48.93 | 17.30 | 45.60 | 66.75 | 76.99 | 59.54 | 42.80 | 63.94 | 71.13 | 52.00 | 57.70 | 59.44 | 57.52 | 51.72 | 45.91 | 52.41 | 58.69 | 51. |
| FAMSD | 1564.64 | 44.46 | 38.11 | 51.20 | 68.92 | 78.06 | 52.64 | 43.50 | 65.21 | 69.63 | 65.23 | 53.38 | 57.34 | 56.11 | 50.89 | 46.82 | 52.93 | 59.35 | 47. |
| SPARKS2 | 1562.01 | 42.66 | 38.11 | 46.22 | 69.33 | 80.43 | 53.10 | 35.40 | 64.09 | 67.88 | 67.69 | 47.48 | 63.64 | 56.64 | 51.01 | 50.61 | 39.83 | 67.71 | 46. |
| GeneSilicoMetaServer | 1560.36 | 45.66 | 20.00 | 43.65 | 67.30 | 76.56 | 57.24 | 47.10 | 63.80 | 68.50 | 65.23 | 47.77 | 58.32 | 52.04 | 52.19 | 44.54 | 52.41 | 68.50 | 47. |
| RAPTOR | 1558.44 | 48.33 | 49.19 | 49.25 | 68.78 | 69.25 | 60.69 | 43.80 | 62.96 | 61.00 | 45.23 | 60.14 | 65.45 | 56.11 | 49.11 | 50.91 | 53.45 | 65.36 | 47. |
| FAMS | 1553.93 | 45.32 | 38.92 | 50.22 | 65.54 | 64.73 | 52.64 | 47.30 | 67.60 | 68.13 | 68.62 | 53.38 | 67.55 | 54.34 | 51.48 | 43.94 | 52.07 | 62.48 | 49. |
| beautshot | 1553.20 | 50.56 | 12.70 | 48.09 | 71.08 | 63.98 | 59.31 | 50.60 | 64.08 | 69.75 | 50.46 | 61.15 | 58.88 | 57.88 | 50.77 | 51.97 | 50.52 | 69.15 | 51. |
| ROBETTA | 1550.95 | 44.12 | 39.19 | 48.63 | 52.03 | 48.93 | 59.31 | 47.20 | 60.84 | 69.25 | 71.08 | 60.00 | 63.36 | 58.94 | 50.18 | 35.45 | 52.59 | 69.41 | 47. |

FIG.72B

| | | | | | | | | | | | | | | | | | | | |
|---|---|---|---|---|---|---|---|---|---|---|---|---|---|---|---|---|---|---|---|
| RAPTORESS | 1550.07 | 50.39 | 51.62 | 43.73 | 65.81 | 73.55 | 61.84 | 43.10 | 61.13 | 58.63 | 49.23 | 58.42 | 67.27 | 55.93 | 46.87 | 41.21 | 53.62 | 65.10 | 46. |
| shub | 1525.50 | 49.96 | 23.78 | 50.22 | 68.78 | 62.36 | 58.62 | 43.90 | 64.65 | 67.38 | 40.31 | 63.74 | 61.26 | 56.46 | 51.48 | 39.85 | 48.97 | 69.02 | 47. |
| UNI-EID_bnmx | 1520.06 | 48.15 | 21.08 | 45.87 | 66.22 | 75.81 | 57.47 | 39.40 | 63.38 | 67.00 | 49.85 | 57.41 | 62.10 | 52.57 | 51.01 | 45.91 | 52.41 | 57.91 | 49. |
| Phyre-2 | 1501.81 | 41.63 | 33.24 | 35.20 | 70.00 | 55.48 | 59.54 | 41.40 | 63.66 | 61.25 | 66.15 | 61.01 | 51.75 | 37.34 | 50.65 | 47.27 | 53.28 | 66.40 | 44. |
| beautshotbase | 1491.48 | 48.67 | 13.24 | 47.02 | 47.84 | 48.82 | 58.39 | 44.20 | 62.96 | 67.13 | 44.61 | 57.41 | 62.80 | 54.69 | 50.65 | 32.42 | 50.00 | 67.06 | 55. |
| karypis.srv | 1478.53 | 30.47 | 10.27 | 48.71 | 45.95 | 76.24 | 59.54 | 46.90 | 65.35 | 57.25 | 63.69 | 54.53 | 63.50 | 54.87 | 12.54 | 44.39 | 53.97 | 66.67 | 48. |
| PROTINFO | 1476.28 | 11.50 | | 45.78 | 66.22 | 77.20 | 63.91 | 45.80 | 61.41 | 61.63 | 63.38 | 46.91 | 60.42 | 57.35 | 52.78 | 45.45 | 51.90 | 68.76 | 53. |
| FUNCTION | 1474.66 | 45.32 | 38.92 | 50.22 | 60.54 | 76.02 | 59.77 | 43.60 | 66.06 | 69.25 | 67.08 | 48.92 | 54.41 | 48.32 | 51.72 | 27.88 | 52.76 | 58.69 | 41. |
| ROKKY | 1464.01 | 39.66 | 32.43 | 46.93 | 64.32 | 52.90 | 53.79 | 37.60 | 59.44 | 64.25 | 39.39 | 45.90 | 54.26 | 56.64 | 45.44 | 33.33 | 48.62 | 69.02 | 49. |
| 3Dpro | 1448.83 | 37.00 | 4.32 | 35.56 | 64.19 | 78.82 | 61.61 | 46.80 | 63.38 | 69.50 | 64.00 | 46.47 | 54.83 | 55.22 | 49.82 | 30.60 | 45.86 | 71.50 | 48. |
| FOLDpro | 1445.26 | 46.27 | 5.41 | 35.56 | 70.27 | 78.17 | 61.61 | 28.90 | 64.65 | 69.25 | 63.08 | 46.47 | 54.68 | 57.17 | 51.12 | 30.60 | 50.17 | 71.11 | 42. |
| [illegible] | [illegible] | [illegible] | [illegible] | [illegible] | [illegible] | [illegible] | [illegible] | [illegible] | [illegible] | [illegible] | [illegible] | [illegible] | [illegible] | [illegible] | [illegible] | [illegible] | [illegible] | [illegible] | [illegible] |

FIG.73

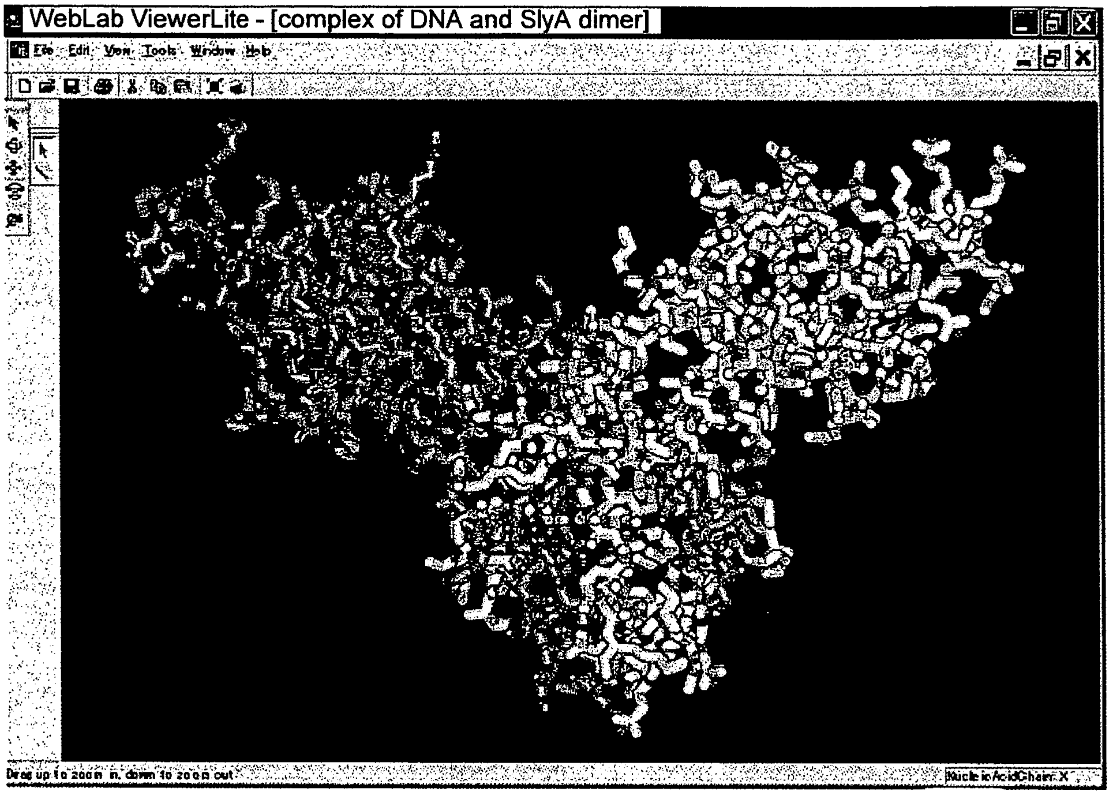
WebLab ViewerLite - [complex of DNA and SlyA dimer]

FIG.74

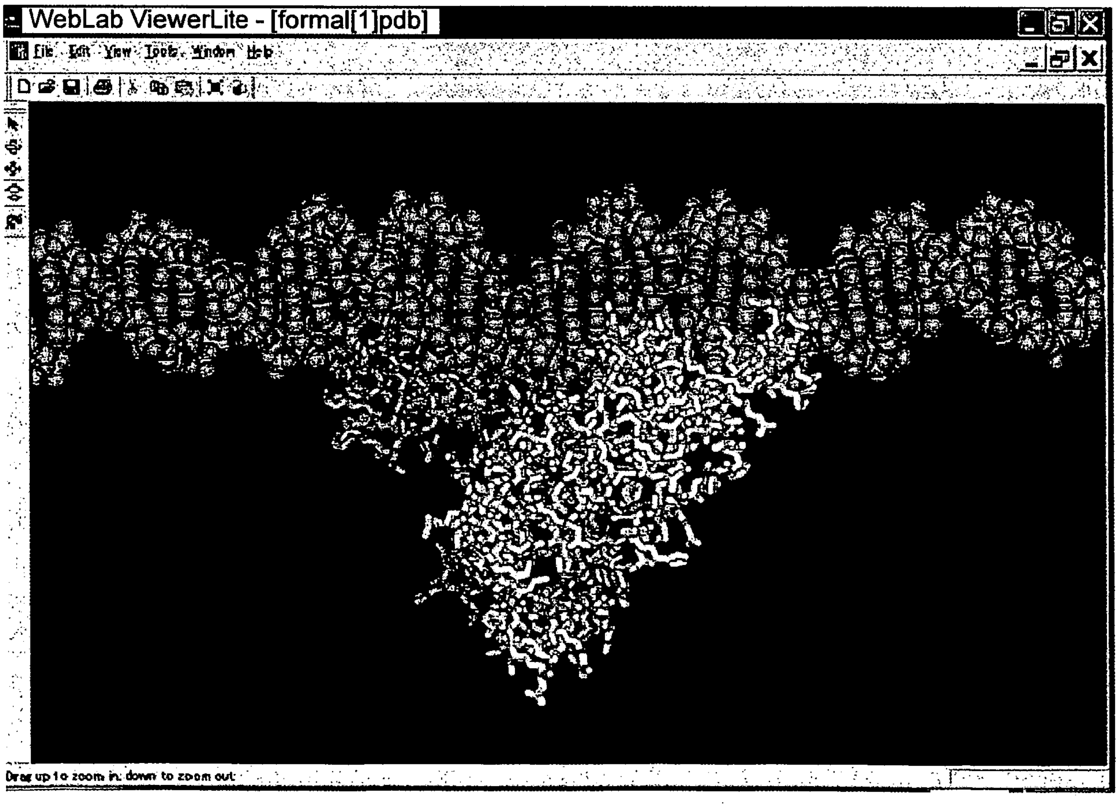
WebLab ViewerLite - [formal[1]pdb]

FIG.75

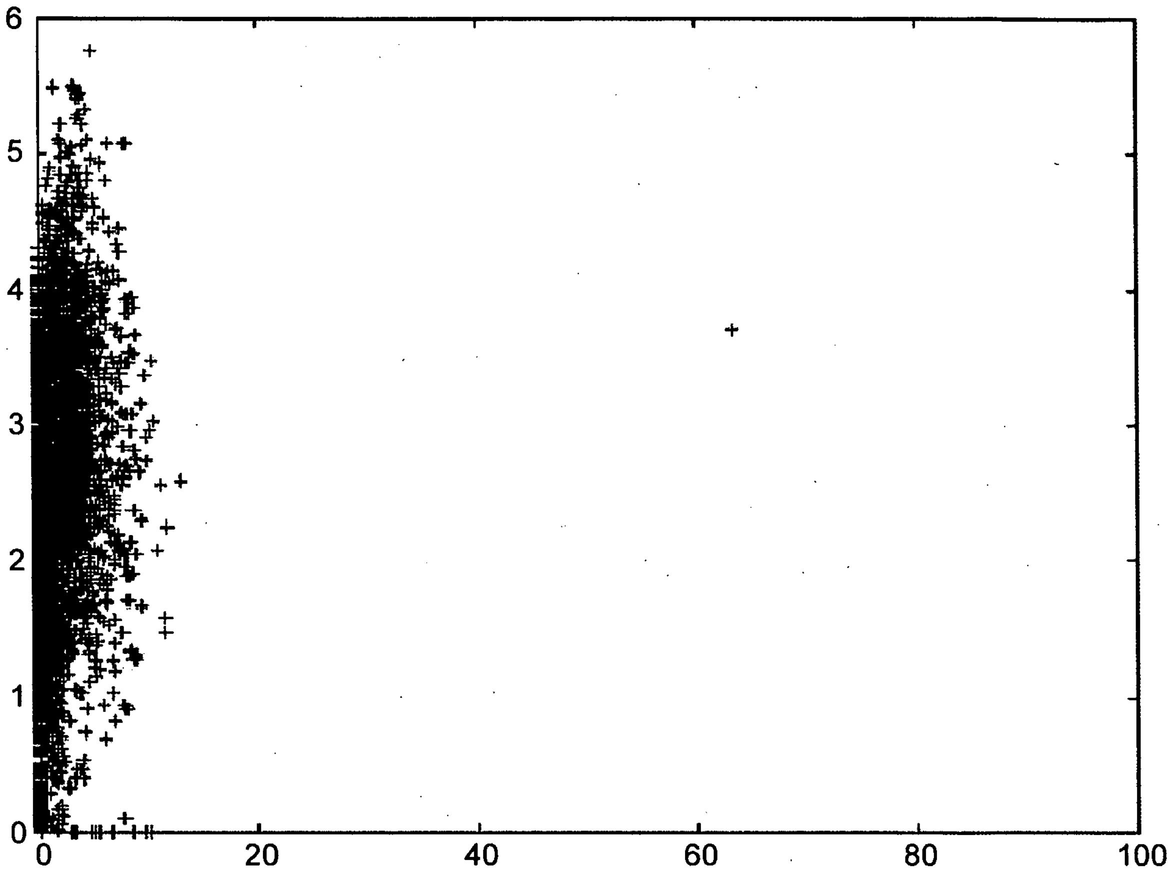

# FIG.76

```
>1MB4_        369    homology    match    mismatch    insertion    deletion
>1PQU_D       371    63.3%       235      107         0            2
(SEQUENCE
NUMBER 1)

1MB4_  : GLYGWRGMVGSVLNQRMVEERDFDLIEPVFFSTSQIGVPAPNF-GKDAGMLHDAFDIESLKQLDAVITCQ
1PQU_D : GFIGWRGMVGSVLNDRMSQENDFENLNPVFFTTSQAGQKAPVFGGKDAGDLKSAFDIEELKKLDIIYTCQ
(SEQUENCE
NUMBER 2) *++***********+**++*+**++++****+***+*++**+*+*****+*++*****+**+**+++***

1MB4_  : GGSYTEKVYPALRQAGWKGYWIDAASTLRMDKEAIITLDPVNLKQILHGIHHGTKTFVGGNCTVSLMLMA
1PQU_D : GGDYTNEVYPKLKATGWDGYWVDAASALRMKDDAIIVLDPVNQHVISEGLKKGIKTFVGGNCTVSLMLMA
         **+**++***+*+++**+***+****+***+++***+*****+++*++*+++*+****************

1MB4_  : LGGLYERGLVEWMSAMTYQAASGAGAQNMRELISQMGVINDAYSSELANPASSILDIDKKVAETMRSGSF
1PQU_D : IGGLFEKDLVEWISYATYQAASGAGAKNMRELLSQMGLLEQAYSSELKDPASSILDIERKVTAKMRADNF
         +***+*++****+*++**********+*****+****++++******++*******++**+++**+++*

1MB4_  : PTDNFGYPLAGSLIPWIDYK-RDNGQSKEEWKAGYEANKILGLQDSPVPIDGTCYRIGAMRCHSQALTIK
1PQU_D : PTDNFGAALGGSLIPWIDKLLPETGQTKEEWKGYAETNKILGLSDNPIPVDGLCYRIGALRCNSQAFTIK
         ******++*+********++++++**+*****+++*+*******+*+*+*+**+******+**+***+***

1MB4_  : LKQNIPLDEIEEMIATHNDWVKYIPNERDITARELTPAKVTGTLSVPVGRLRKMAMGDDFLNA
1PQU_D : LKKDLPLEEIEQIIASHNEWVKYIPNDKEITLRELTPAKVTGTLSVPVGRLRKLAMGPEYLAA
         **+++**+***++**+**+*******+++**+*********************+***+++*+*
```

FIG.77

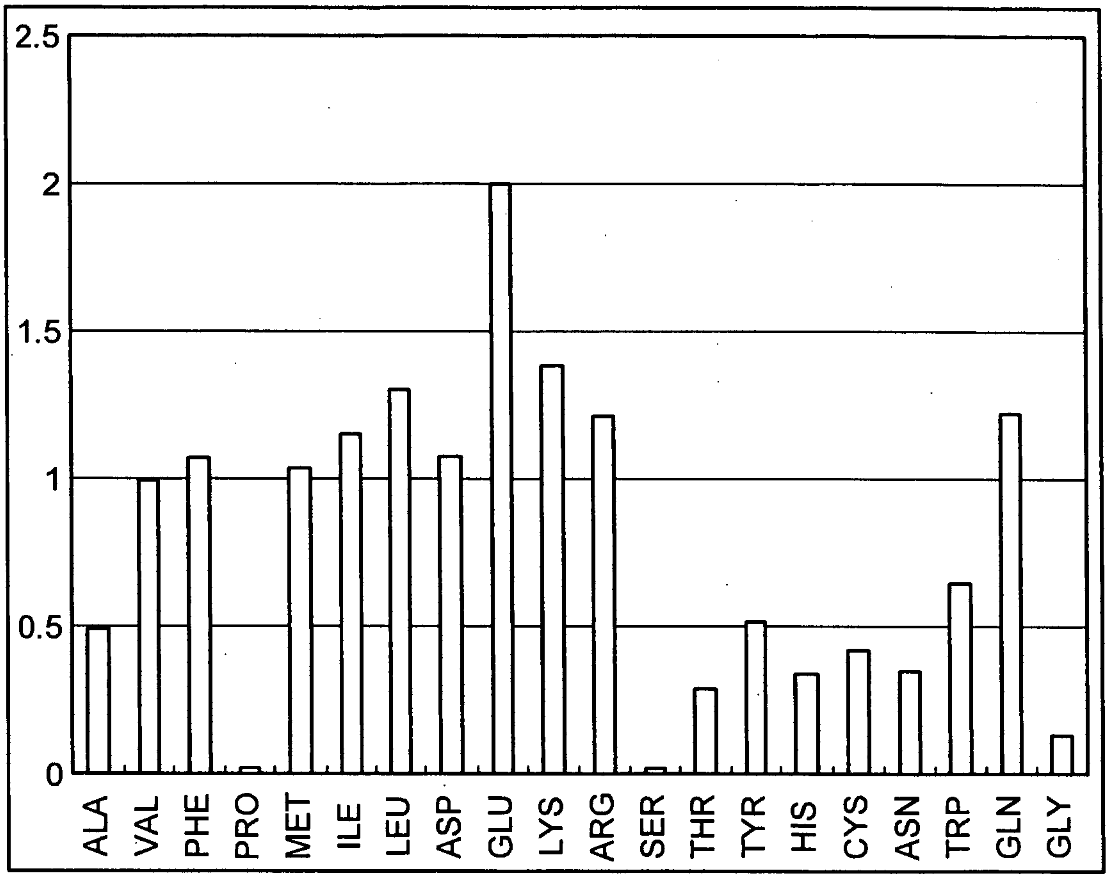
2.5
2
1.5
1
0.5
0
ALA
VAL
PHE
PRO
MET
ILE
LEU
ASP
GLU
LYS
ARG
SER
THR
TYR
HIS
CYS
ASN
TRP
GLN
GLY

**INTERNATIONAL SEARCH REPORT**

International application No.
PCT/JP2007/073005

A. CLASSIFICATION OF SUBJECT MATTER
*G06F19/00*(2006.01)i, *C07K14/00*(2006.01)n

According to International Patent Classification (IPC) or to both national classification and IPC

B. FIELDS SEARCHED

Minimum documentation searched (classification system followed by classification symbols)
G06F19/00, C07K14/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
JMEDPlus(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| A | WO 2004/051546 A1 (In-Silico Sciences, Inc.), 17 June, 2004 (17.06.04), Full text; all drawings (Family: none) | 1-27 |
| A | WO 2005/027009 A1 (ACGT PROGENOMICS AG.), 24 March, 2005 (24.03.05), Full text; all drawings & JP 2007-505372 A & US 2006/253260 A1 & EP 1513092 A1 | 1-27 |
| A | WO 2005/081166 A1 (In-Silico Sciences, Inc.), 01 September, 2005 (01.09.05), Full text; all drawings (Family: none) | 1-27 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 February, 2008 (21.02.08) | 04 March, 2008 (04.03.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2007/073005

C (Continuation). DOCUMENTS CONSIDERED TO BE RELEVANT

| Category* | Citation of document, with indication, where appropriate, of the relevant passages | Relevant to claim No. |
|---|---|---|
| P,A | G.Terashi, M.Takeda-Shitaka, K.Kanou, M.Iwadate, D.Takaya, A.Hosoi, K.Ohta, and H.Umeyama, Template based and freemodeling in CASP7, [online], Heisei 18 Nendo Dai 7 Kai GSIC Symposium (34 Kai Bunshi Seibutsu Joho Kenkyukai), 2006.12.08, [retrieval date: 2008.02.21], Internet <URL: http://famshelp.gsc.riken.jp/famsbase/GSIC_casp7_report_fix.pdf> | 1-27 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Bowie JU ; Luthy R ; Eisenberg D.** A method to identify protein sequences that fold into a known three-dimensional structure. *Science,* 12 July 1991, vol. 253 (5016), 164-70
- **Eisenberg D ; Luthy R ; Bowie JU.** VERIFY3D: assessment of protein models with three-dimensional profiles. *Methods Enzymol.,* 1997, vol. 277, 396-404
- **Ogata, K. ; Umeyama, H.** An automatic homology modeling method consisting of database searches and simulated annealing. *J. Mol. Graphics Mod.,* 2000, vol. 18, 258-272
- **Ogata, K. ; Umeyama, H.** An automatic homology modeling method consisting of data base searches and simulated annealing. *J. Mol. Graphics Mod.,* 2000, vol. 18, 258-272
- *Proteins,* 2005, vol. 7, 27-45
- **Nobuhiko Okada ; Yorie Oi ; Mayuko Takeda-Shitaka ; Kazuhiko Kanou ; Hideaki Umeyama ; Takeshi Haneda ; Tsuyoshi Miki ; Sachiko Hosoya ; Hirofumi Danbara.** Identification of amino acid residues of Salmonella SlyA that are critical for transcriptional regulation. *Microbiology (England),* 2007, vol. 153, 548-560